(19) Europäisches Patentamt
European Patent Office
Office européen des brevets

(11) EP 4 685 142 A1

(12) EUROPEAN PATENT APPLICATION
published in accordance with Art. 153(4) EPC

(43) Date of publication:
28.01.2026 Bulletin 2026/05

(21) Application number: 24774266.1

(22) Date of filing: 22.03.2024

(51) International Patent Classification (IPC):
C07D 409/14 (2006.01) C07D 401/12 (2006.01)
A61P 35/00 (2006.01) A61P 29/00 (2006.01)
A61P 37/06 (2006.01) A61K 31/53 (2006.01)

(52) Cooperative Patent Classification (CPC):
A61K 31/53; A61P 1/00; A61P 9/00; A61P 11/00; A61P 13/12; A61P 17/06; A61P 25/16; A61P 25/28; A61P 29/00; A61P 35/00; A61P 37/00; A61P 37/06; C07D 253/07; C07D 401/12; C07D 409/04; (Cont.)

(86) International application number:
PCT/CN2024/083349

(87) International publication number:
WO 2024/193703 (26.09.2024 Gazette 2024/39)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 23.03.2023 CN 202310291105
26.09.2023 CN 202311247774
28.02.2024 CN 202410221655

(71) Applicant: Chengdu Zenitar Biomedical Technology Co., Ltd.
Chengdu, Sichuan 610000 (CN)

(72) Inventors:
- CHEN, Lijuan
Chengdu, Sichuan 610000 (CN)
- WANG, Taijin
Chengdu, Sichuan 610000 (CN)
- JIA, Tao
Chengdu, Sichuan 610000 (CN)
- LI, Gang
Chengdu, Sichuan 610000 (CN)

(74) Representative: Gulde & Partner
Patent- und Rechtsanwaltskanzlei mbB
Berliner Freiheit 2
10785 Berlin (DE)

# (54) TRIAZINE COMPOUND AND USE THEREOF

(57) The present invention discloses a triazine compound and use thereof, and belongs to the technical field of chemical medicines. The triazine compound shown in formula I, provided by present invention, can be used as an NLRP3 inhibitor, and has high activity and excellent pharmacokinetic properties, thereby providing a new way for treating NLRP3-related diseases.

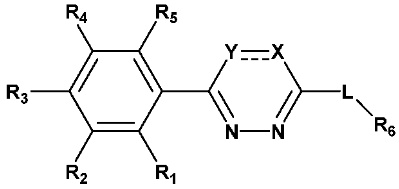

式 I

EP 4 685 142 A1

(52) Cooperative Patent Classification (CPC): (Cont.)
**C07D 409/14**

## Description

## TECHNICAL FIELD

The present invention belongs to the field of chemical medicines, and relates to a triazine compound and use thereof.

## BACKGROUND

Inflammasomes are of protein complexes that can recognize intracellular pathogen associated molecular patterns (PAMPs), or damage associated molecular patterns (DAMPs) and others. The assembly of inflammasomes triggers protein hydrolysis. The dormant procapase-1 is decomposed into active caspase-1, cytokine precursors pro-IL-1β and pro-IL-18 are transformed into mature and biologically active IL-1β and IL-18 respectively, and various biological effects are produced by regulating expressions of inflammation-related genes. As a receptor of innate body immunity, inflammasome activation can defend against pathogen infection and stress-induced damage, but its dysregulated activation can also aggravate inflammatory responses and organ injury. Currently, research on NLRP3 (nucleotide-binding oligomerization domain (NOD)-like receptor family, pyrin domain-containing protein 3) inflammasome is the most popular topic.

The NLRP3 inflammasome is composed of a sensor (NLRP3), an adapter (ASC, also known as PYCARD), and an effector (caspase 1). Typical activation of NLRP3 inflammasome requires dual signals. A first signal activates a TLR4 (Toll like receptor 4) signaling pathway, promoting nuclear translocation of NF-κB, which induces the production of precursors such as IL-1β and IL-18, and triggers post-translational modification of NLRP3. A second signal facilitates the assembly of NLRP3/ASC/pro-caspase-1 complex. Upon activation, the NLRP3/ASC/pro-caspase-1 complex polymerizes with ASC (apoptosis-associated specklike protein containing a CARD), and then ASC interacts with cysteine protease caspase-1 to form the inflammasome complex. Subsequently, the pro-caspase-1 in a precursor form undergoes auto-cleavage and demonstrates an activated form. The activated caspase-1 cleaves pro-inflammatory cytokines IL-1β and IL-18 in the precursor form and converts them into IL-1β and IL-18 in active forms. Cytokines IL-1β and IL-18 are released extracellularly. This process recruits inflammatory cells and amplifies the inflammatory response. Apoptosis-associated specklike protein containing a CARD also can recruit and activate caspase-8, and cleave IL-1β and IL-18 in the precursor form and convert them into IL-1β and IL-18 in active forms, thereby triggering pyroptosis. Atypical activation of NLRP3 inflammasome does not depend on the activation of TLR4 signaling pathway, and is initiated through direct recognition of intracellular LPS by caspase-11, triggering activation of NLRP3 inflammasome and promoting activation and release of Gasdermin D, which mediates cell death.

The aberrant activation of NLRP3 is associated with numerous diseases, primarily including inflammasome-related diseases, immunological diseases, inflammatory diseases, neurological diseases, autoimmune diseases and/or autoinflammatory diseases, cancers, chronic metabolic diseases, and neurodegenerative diseases. For example, cryopyrin-associated periodic syndrome (CAPS), Muckle-Wells syndrome (MWS), familial cold autoinflammatory syndrome (FCAS), neonatal-onset multisystem inflammatory disease (NOMID), familial Mediterranean fever (FMF), non-alcoholic steatohepatitis, alcoholic liver disease, graft-versus-host disease, multiple sclerosis (MS), rheumatoid arthritis, type I/II diabetes mellitus and related complications (for example, nephropathy and retinopathy), psoriasis, Alzheimer's disease, atherosclerosis, gout, chronic kidney disease, sepsis, liver fibrosis, idiopathic pulmonary fibrosis, epilepsy, neuropathic pain, depressive disorders, Parkinson's disease, asthma, acute myocardial infarction, systemic lupus erythematosus, rheumatoid arthritis, Crohn's disease, ulcerative colitis, inflammatory bowel disease, rheumatoid arthritis, ankylosing spondylitis, bronchial asthma, acute respiratory distress syndrome, chronic obstructive pulmonary disease, or ischemic stroke. NLRP3 in the upstream of cytokines can ultimately block inflammation at its source. Therefore, the development of new NLRP3 inflammasome inhibitors has high research value.

## Summary

An objective of the present invention is to invent a triazine compound and use thereof, or stereoisomers, solvates, metabolites, deuterated variants, prodrugs, pharmaceutically acceptable salts or eutectics thereof, including pharmaceutical compositions thereof, for treating NLRP3-related diseases.

In a first aspect, the present invention provides a compound shown in formula I or a pharmaceutically acceptable form thereof, where a structure of the formula I is as follows:

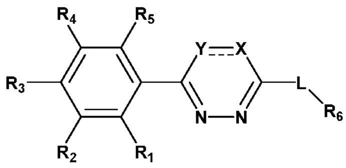

式I

where:

Y- - - -Xrepresents a single bond, Y is selected from $NR_{7a}$, and X is selected from C(=O);

$R_1$ is selected from hydrogen, deuterium, halogen, -OH, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, and 3- to 6-membered cycloalkyl; and in $R_1$, the substituents are selected from deuterium, halogen, -OH, $-NH_2$, or -CN;

$R_2$ and $R_4$ are each independently selected from hydrogen, deuterium, halogen, -OH, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, and 3- to 6-membered cycloalkyl; and in $R_2$ and $R_4$, the substituents are selected from deuterium, halogen, -OH, $-NH_2$, or -CN;

$R_3$ is selected from hydrogen, deuterium, halogen, -OH, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, 3- to 6-membered cycloalkyl, 5- and 6-membered heterocycloalkyl, phenyl, and 5- and 6-membered heteroaryl; in $R_3$, the substituents are selected from deuterium, halogen, -OH, $-NH_2$, -CN, or 3- to 6-membered cycloalkyl; and in $R_3$, 5- and 6-membered heterocycloalkyl and 5- and 6-membered heteroaryl contain 1-3 heteroatoms selected from at least one of N, S, and O;

$R_5$ is selected from hydrogen, deuterium, halogen, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, and 3- to 6-membered cycloalkyl; and in $R_5$, the substituents are selected from deuterium, halogen, -OH, $-NH_2$ or -CN;

or, $R_2$ and $R_3$, $R_3$ and $R_4$, or $R_4$ and $R_5$, together with atoms to which these groups are bound, form a 5- and 6-membered alkane ring, a benzene ring, a 5- and 6-membered alkane heterocyclic ring, or a 5- and 6-membered heteroaromatic ring that is substituted with 0-6 substituents, where the substituents are selected from deuterium, halogen, -OH, $-NH_2$, -CN, an oxo group, $C_{1-6}$ alkyl, $C_{1-6}$ fluoroalkyl, $C_{1-6}$ deuterated alkyl, -O-$C_{1-6}$ alkyl, -O-$C_{1-6}$ fluoroalkyl, -O-$C_{1-6}$ deuterated alkyl, $C_{3-6}$ cycloalkyl, $C_{3-6}$ fluorocycloalkyl, or 3- to 6-membered cycloalkyl formed by two of the substituents bound to a same carbon atom; when $R_2$ and $R_3$, $R_3$ and $R_4$, or $R_4$ and $R_5$, together with the atoms to which these groups are bound, form a ring, the 5- and 6-membered alkane heterocyclic ring and the 5- and 6-membered heteroaromatic ring contain 1-3 heteroatoms selected from at least one of N, S, and O;

when $R_1$ is selected from -OH, $R_2$ and $R_3$, together with the atoms to which these groups are bound, form the benzene ring, a 5-7 membered alkane heterocyclic ring, or the 5- and 6-membered heteroaromatic ring that is substituted with 0-6 substituents, where the substituents are selected from deuterium, halogen, -OH, $-NH_2$, -CN, $C_{1-6}$ alkyl, $C_{1-6}$ fluoroalkyl, $C_{1-6}$ deuterated alkyl, -O-$C_{1-6}$ alkyl, -O-$C_{1-6}$ fluoroalkyl, -O-$C_{1-6}$ deuterated alkyl, $C_{3-6}$ cycloalkyl, $C_{3-6}$ fluorocycloalkyl, or 3- to 6-membered cycloalkyl formed by two of the substituents bound to the same carbon atom; and when $R_2$ and $R_3$, together with the atoms to which these groups are bound, form a ring, the 5-7 membered alkane heterocyclic ring and the 5- and 6-membered heteroaromatic ring contain 1-3 heteroatoms selected from at least one of N and S, or 2 atoms of O;

L is selected from $-(CH_2)_{n1}-$, O, $-(CH_2)_{n1}$-NH-, -NH-$(CH_2)_{n1}$-, and -NH-$CH(CH_2)_{n1}(CH_3)$-, where n1 is an integer selected from 0 to 3;

$R_6$ is selected from 6- to 10-membered aryl, 5- to 10-membered heteroaryl, 3- to 8-membered heterocycloalkyl, 3- to 8-membered cycloalkyl, 6- to 10-membered spirocycloalkyl, 6- to 10-membered heterospirocycloalkyl, 6- to 10-membered bridged cycloalkyl, 6- to 10-membered heterobridged cycloalkyl, and $C_{1-6}$ alkyl that are substituted with 0-6 substituents; in $R_6$, the substituents are selected from $R_{8a}$, halogen, the oxo group, $-OR_{8a}$, $-SR_{8a}$, $-C(=O)R_{8a}$, $-OC(=O)R_{8a}$, $-C(=O)OR_{8a}$, $-C(=O)NR_{8a}R_{8b}$, $-NR_{8a}C(=O)R_{8b}$, $-NR_{8a}R_{8b}$, $-SO_2R_{8a}$, $-SO_2NR_{8a}R_{8b}$, $-NR_{8a}SO_2R_{8b}$, and -CN; and in $R_6$, 5- to 10-membered heteroaryl, 3- to 8-membered heterocycloalkyl, 6- to 10-membered heterospirocycloalkyl, and 6- to 10-membered bridged cycloalkyl contain 1-3 heteroatoms selected from at least one of N, S and O;

$R_{8a}$ and $R_{8b}$ are each independently selected from hydrogen, deuterium, or the following groups substituted with 0-6 substituents: $C_{1-4}$ alkyl, 3- to 6-membered cycloalkyl, 4- to 6-membered heterocycloalkyl, phenyl, 5- and 6-membered heteroaryl, (3- to 6-membered cycloalkyl)-methylene, and (4- to 6-membered heterocycloalkyl)-methylene; in $R_{8a}$ and

$R_{88}$, the substituents are selected from deuterium, halogen, -N($R_{10a}R_{10b}$), -OH, -CN, $C_{1-4}$ alkyl, $C_{1-4}$ alkoxy, $C_{1-4}$ deuterated alkyl, 3- to 6-membered cycloalkyl, 4- to 6-membered heterocycloalkyl, (3- to 6-membered cycloalkyl)-methylene, or (4- to 6-membered heterocycloalkyl)-methylene; and in $R_{8a}$ and $R_{8b}$, 4- to 6-membered heterocycloalkyl, 5- and 6-membered heteroaryl, and (4- to 6-membered heterocycloalkyl)-methylene contain 1-3 heteroatoms selected from at least one of N, S and O; and 4- to 6-membered heterocycloalkyl and (4- to 6-membered heterocycloalkyl)-methylene in the substituents contain 1-3 heteroatoms selected from at least one of N, S and O; or, $R_{8a}$ and $R_{8b}$, together with atoms to which these groups are bound, form a 3- to 6-membered alkylheterocyclic ring substituted with 0-6 substituents; when $R_{8a}$ and $R_{8b}$, together with the atoms to which these groups are bound, form a ring, the substituents are selected from deuterium, halogen, -N($R_{11a}R_{11b}$), -OH, -CN, $C_{1-4}$ alkyl, 3- to 6-membered cycloalkyl, and 4- to 6-membered heterocycloalkyl; and when $R_{8a}$ and $R_{8b}$, together with the atoms to which these groups are bound, form the ring, 3- to 6-membered heterocycloalkyl contains 1-3 heteroatoms selected from at least one of N, S and O, and 4- to 6-membered heterocycloalkyl in the substituents contains 1-3 heteroatoms selected from at least one of N, S and O;

$R_{7a}$ is selected from hydrogen, or the following groups substituted with 0-6 substituents: $C_{1-6}$ alkyl, $C_{2-6}$ alkenyl, $C_{2-6}$ alkynyl, 3- to 6-membered cycloalkyl, 4- to 6-membered heterocycloalkyl, phenyl, and 5- and 6-membered heteroaryl; in $R_{7a}$, the substituents are selected from hydrogen, deuterium, halogen, -OH, -$NH_2$, or -CN; and in $R_{7a}$, 4- to 6-membered heterocycloalkyl and 5- and 6-membered heteroaryl contain 1-3 heteroatoms selected from at least one of N, S and O;

$R_{10a}$, $R_{10b}$, $R_{11a}$, and $R_{11b}$ are each independently selected from hydrogen or $C_{1-4}$ alkyl;

the pharmaceutically acceptable form is selected from pharmaceutically acceptable salts, esters, stereoisomers, polymorphs, solvates, nitrogen oxides, isotope markers, metabolites, or prodrugs.

In some embodiments of the present invention, $R_1$ is selected from hydrogen, deuterium, halogen, -OH, -$NH_2$, -CN, or the following groups optionally substituted with 0-6 substituents: $C_{1-4}$ alkyl, -O-$C_{1-4}$ alkyl, -S-$C_{1-4}$ alkyl, $C_{2-4}$ alkenyl, $C_{2-4}$ alkynyl, and 3- to 6-membered cycloalkyl, where the substituents are selected from deuterium, halogen, -OH, -$NH_2$, or -CN.

In some preferred embodiments of the present invention, $R_1$ is selected from hydrogen, deuterium, halogen, F, Cl, -OH, -$NH_2$, and -CN, or the following groups optionally substituted with 0-3 substituents: $C_{1-4}$ alkyl, -O-$C_{1-4}$ alkyl, -S-$C_{1-4}$ alkyl, and 3- to 6-membered cycloalkyl, where the substituents are selected from deuterium, F, Cl, -OH, -$NH_2$, or -CN.

In some more preferred embodiments of the present invention, $R_1$ is selected from hydrogen, deuterium, F, Cl, -OH, -$CH_3$, fluoromethyl, deuterated methyl, methoxy, fluoromethoxy, deuterated methoxy, cyclopropyl, and fluorocyclopropyl.

In some embodiments of the present invention, $R_2$ and $R_4$ are each independently selected from hydrogen, deuterium, halogen, -OH, -$NH_2$, -CN, or the following groups optionally substituted with 0-6 substituents: $C_{1-4}$ alkyl, -O-$C_{1-4}$ alkyl, -S-$C_{1-4}$ alkyl, $C_{2-4}$ alkenyl, $C_{2-4}$ alkynyl, and 3- to 6-membered cycloalkyl, where the substituents are selected from deuterium, halogen, -OH, -$NH_2$, or -CN.

In some preferred embodiments of the present invention, $R_2$ and $R_4$ are each independently selected from hydrogen, deuterium, halogen, F, Cl, -OH, -$NH_2$, -CN, or the following groups optionally substituted with 0-3 substituents: $C_{1-4}$ alkyl, -O-$C_{1-4}$ alkyl, -S-$C_{1-4}$ alkyl, and 3- to 6-membered cycloalkyl, where the substituents are selected from deuterium, F, Cl, -OH, -$NH_2$, or -CN.

In some more preferred embodiments of the present invention, $R_2$ and $R_4$ are each independently selected from hydrogen, deuterium, F, Cl, -OH, -$CH_3$, fluoromethyl, deuterated methyl, methoxy, fluoromethoxy, deuterated methoxy, cyclopropyl, and fluorocyclopropyl.

In some embodiments of the present invention, $R_3$ is selected from hydrogen, deuterium, halogen, -OH, -$NH_2$, -CN, or the following groups optionally substituted with 0-6 substituents: $C_{1-4}$ alkyl, -O-$C_{1-4}$ alkyl, -S-$C_{1-4}$ alkyl, $C_{2-4}$ alkenyl, $C_{2-4}$ alkynyl, 3- to 6-membered cycloalkyl, 5- and 6-membered heterocycloalkyl, phenyl, and 5- and 6-membered heteroaryl; in $R_3$, the substituents are selected from deuterium, halogen, -OH, -$NH_2$, -CN, -$CF_3$, or cyclopropyl; and in $R_3$, 5- and 6-membered heterocycloalkyl and 5- and 6-membered heteroaryl contain 1-3 heteroatoms selected from at least one of N, S, and O.

In some preferred embodiments of the present invention, $R_3$ is selected from hydrogen, deuterium, F, Cl, -CN, or the following groups optionally substituted with 0-3 substituents: $C_{1-4}$ alkyl, -O-$C_{1-4}$ alkyl, -S-$C_{1-4}$ alkyl, $C_{2-4}$ alkenyl, $C_{2-4}$ alkynyl, 3- to 6-membered cycloalkyl, 5- and 6-membered heterocycloalkyl, phenyl, and 5- and 6-membered heteroaryl; in $R_3$, the substituents are selected from deuterium, F, Cl, -OH, -$NH_2$, -$CF_3$, -CN, or cyclopropyl; and in $R_3$, 5- and 6-membered heterocycloalkyl and 5- and 6-membered heteroaryl contain 1-2 heteroatoms selected from at least one of N, S, and O.

In some more preferred embodiments of the present invention, $R_3$ is selected from hydrogen, deuterium, F, Cl, methyl, fluoromethyl, deuterated methyl, methylthio, fluoromethylthio, deuterated methylthio, methoxy, fluoromethoxy, deuterated methoxy, cyclopropyl, fluorocyclopropyl, vinyl, ethynyl, phenyl, fluorophenyl, and deuterated phenyl.

In some embodiments of the present invention, $R_5$ is selected from hydrogen, deuterium, halogen, $-NH_2$, -CN, or the following groups optionally substituted with 0-6 substituents: $C_{1-4}$ alkyl, -O-$C_{1-4}$ alkyl, -S-$C_{1-4}$ alkyl, $C_{2-4}$ alkenyl, $C_{2-4}$ alkynyl, and 3- to 6-membered cycloalkyl, where the substituents are selected from deuterium, halogen, -OH, $-NH_2$, or -CN.

In some preferred embodiments of the present invention, $R_5$ is selected from hydrogen, deuterium, F, Cl, $-NH_2$, -CN, or the following groups optionally substituted with 0-3 substituents: $C_{1-4}$ alkyl, -O-$C_{1-4}$ alkyl, -S-$C_{1-4}$ alkyl, and 3- to 6-membered cycloalkyl, where the substituents are selected from deuterium, F, Cl, -OH, $-NH_2$, or -CN.

In some more preferred embodiments of the present invention, $R_5$ is selected from hydrogen, deuterium, F, Cl, -CH, $-CH_3$, fluoromethyl, deuterated methyl, methoxy, fluoromethoxy, deuterated methoxy, cyclopropyl, and fluorocyclopropyl.

In some embodiments of the present invention, $R_2$ and $R_3$, $R_3$ and $R_4$, or $R_4$ and $R_5$, together with the atoms to which these groups are bound, form the 5- and 6-membered alkane ring, the benzene ring, the 5- and 6-membered alkane heterocyclic ring, or the 5- and 6-membered heteroaromatic ring that is substituted with 0-6 substituents, where the substituents are selected from deuterium, halogen, -OH, $-NH_2$, -CN, the oxo group, $C_{1-4}$ alkyl, $C_{1-4}$ fluoroalkyl, $C_{1-4}$ deuterated alkyl, -O-$C_{1-4}$ alkyl, -O-$C_{1-4}$ fluoroalkyl, -O-$C_{1-4}$ deuterated alkyl, 3- to 6-membered cycloalkyl, 3- to 6-membered fluorocycloalkyl, or 3- and 4-membered cycloalkyl formed by two of the substituents bound to a same carbon atom; when $R_2$ and $R_3$, $R_3$ and $R_4$, or $R_4$ and $R_5$, together with the atoms to which these groups are bound, forms the ring and the 5- and 6-membered alkane heterocyclic ring, the 5- and 6-membered heteroaromatic ring contain 1-2 heteroatoms selected from at least one of N, S, and O;

In some preferred embodiments of the present invention, $R_2$ and $R_3$, or $R_3$ and $R_4$, together with atoms to which these groups are bound, forms

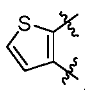, 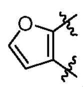, 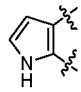, 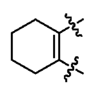,

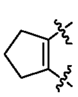, 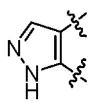, 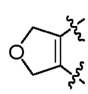, 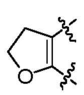, 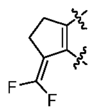, 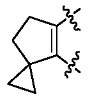, 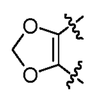, 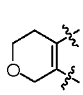, 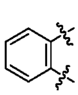, 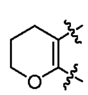, 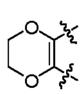,

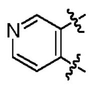 or 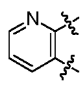

that is substituted with 0-3 substituents, where the substituents are selected from deuterium, F, Br, Cl, -OH, $-NH_2$, -CN, the oxo group, methyl, fluoromethyl, deuterated methyl, methoxy, fluoromethoxy, deuterated methoxy, cyclopropyl, fluorocyclopropyl, or3and 4-membered cycloalkyl formed by two of the substituents bound to the same carbon atom.

In some embodiments of the present invention, when $R_1$ is selected from -OH, $R_2$ and $R_3$, together with the atoms to which these groups are bound, form the benzene ring, the 5- and 6-membered alkane heterocyclic ring, or the 5- and 6-membered heteroaromatic ring that is substituted with 0-6 substituents, where the substituents are selected from deuterium, halogen, -OH, $-NH_2$, -CN, $C_{1-4}$ alkyl, $C_{1-4}$ fluoroalkyl, $C_{1-4}$ deuterated alkyl, -O-$C_{1-4}$ alkyl, -O-$C_{1-4}$ fluoroalkyl, -O-$C_{1-4}$ deuterated alkyl, 3- to 6-membered cycloalkyl, 3- to 6-membered fluorocycloalkyl, or 3- and 4-membered cycloalkyl formed by two of the substituents bound to the same carbon atom; and when $R_2$ and $R_3$, together with the atoms to which these groups are bound, form the ring, the 5- and 6-membered alkane heterocyclic ring contains two atoms of O, and the 5- and 6-membered heteroaromatic ring contains 1-2 heteroatoms selected from at least one of N, S, and O.

In some preferred embodiments of the present invention, when $R_1$ is selected from -OH, $R_2$ and $R_3$, together with the atoms to which these groups are bound, form

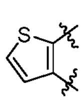, 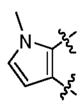,

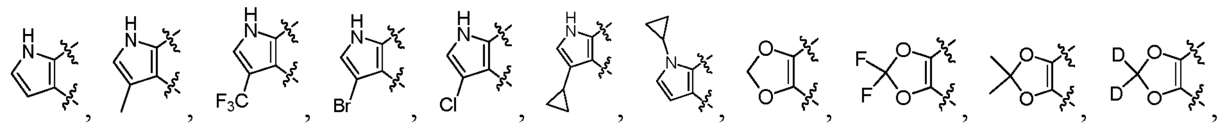

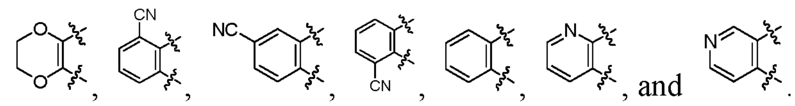

In some embodiments of the present invention, a structural unit

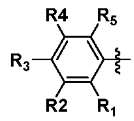

is selected from:

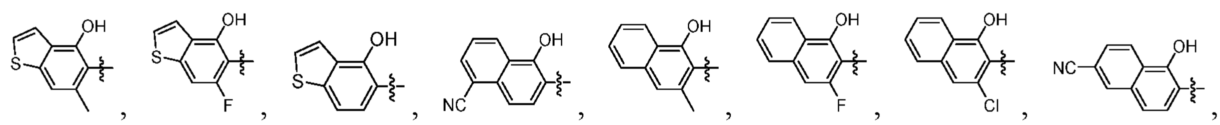

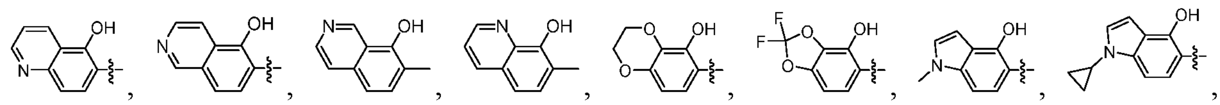

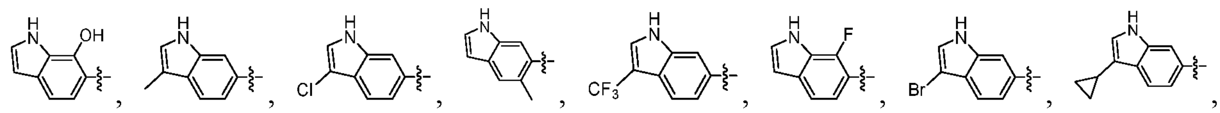

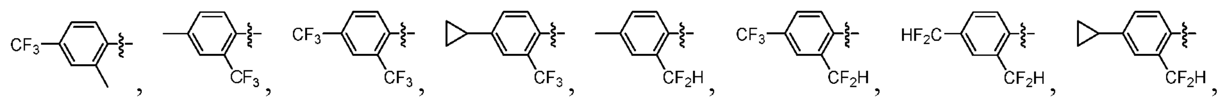

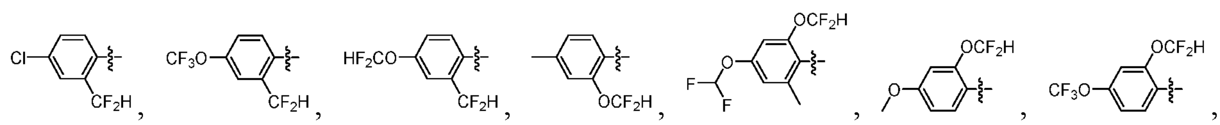

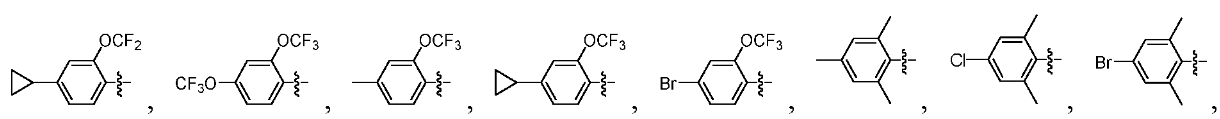

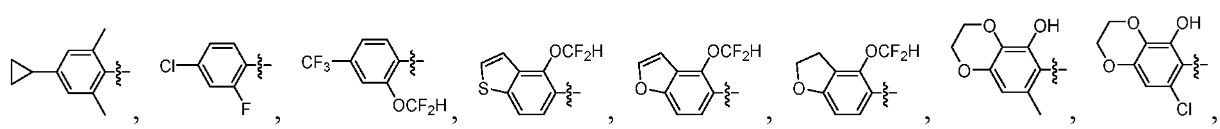

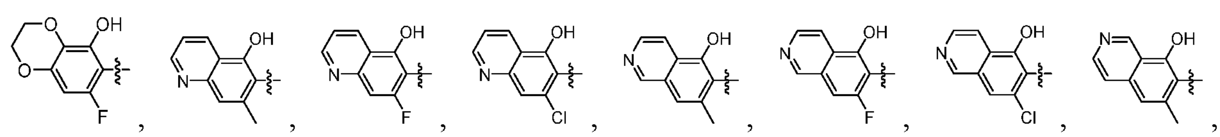

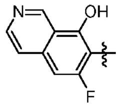, 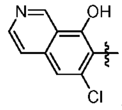, 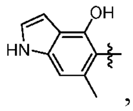, 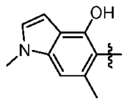, 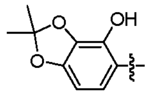, 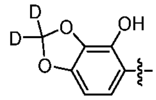, and 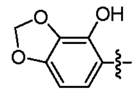.

In some embodiments of the present invention, $R_{7a}$ is selected from hydrogen, or the following groups optionally substituted with 0-6 substituents: $C_{1-4}$ alkyl, $C_{2-6}$ alkenyl, $C_{2-6}$ alkynyl, 3- to 6-membered cycloalkyl, 4- to 6-membered heterocycloalkyl, phenyl, and 5- and 6-membered heteroaryl; and in $R_{7a}$, the substituents are selected from hydrogen, deuterium, halogen, -OH, $-NH_2$, or -CN.

In some preferred embodiments of the present invention, $R_{7a}$ is selected from hydrogen, $C_{1-4}$ alkyl, $C_{1-4}$ fluoroalkyl, $C_{1-4}$ deuterated alkyl, $C_{1-4}$ alkoxy, $C_{1-4}$ fluoroalkoxy, $C_{1-4}$ deuterated alkoxy, carboxyl, $C_{1-4}$ alkoxycarbonyl, $C_{1-4}$ fluoroalkoxycarbonyl, $C_{1-4}$deuterated alkoxycarbonyl, 3- to 6-membered cycloalkyl, 3- to 6-membered fluorocycloalkyl, phenyl, pyridinyl, or

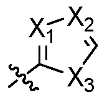;

$X_l$ and $X_2$ are each independently selected from CH and N; and $X_3$ is selected from N-$C_{1-4}$ alkyl, NH, S, and O.

In some more preferred embodiments of the present invention, $R_{7a}$ is selected from hydrogen, methyl, deuterated methyl, fluoromethyl, ethyl, fluoroethyl, isopropyl, fluoroisopropyl, cyclopropyl, fluorocyclopropyl, cyclohexyl, fluorocyclohexyl, phenyl, 2-pyridinyl, and

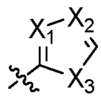;

$X_1$ is selected from CH and N; and $X_3$ is selected from N-methyl, S and O.

In some embodiments of the present invention, a structural unit

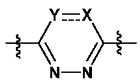

is selected from:

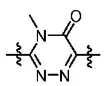, 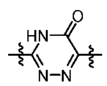, 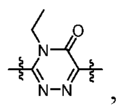, 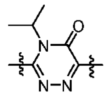, 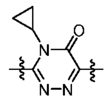, 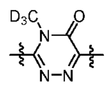, 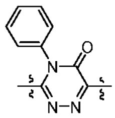, 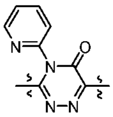, 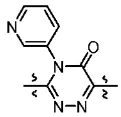,

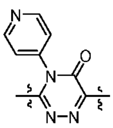, 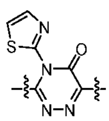, 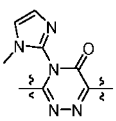, 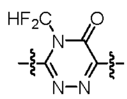, 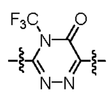, and 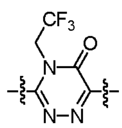.

In some embodiments of the present invention, L is selected from O, -NH-, $-NH-CH_2-$, and $-NH-CH(CH_3)-$.

In some embodiments of the present invention, in $R_6$, the substituents are selected from fluorine, chlorine, the hydroxy group, the cyanogroup, the oxo group, $C_{1-4}$ alkyl, $C_{1-4}$ fluoroalkyl, $C_{1-4}$ deuterated alkyl, $C_{1-4}$ alkoxy, $C_{1-4}$ fluoroalkoxy, $C_{1-4}$ deuterated alkoxy, 3- to 6-membered cycloalkyl, 3- to 6-membered fluorocycloalkyl, the amino group, the dimethylamino group,

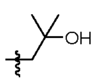, 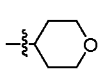, 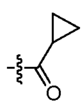, 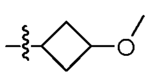, 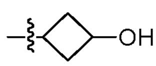, 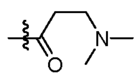,

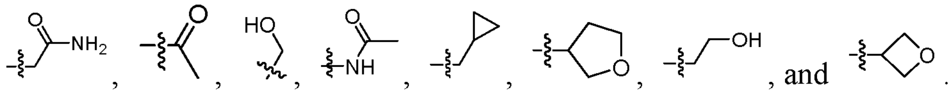
, , , , , , , , and .

In some embodiments of the present invention, $R_6$ is selected from the following structures:

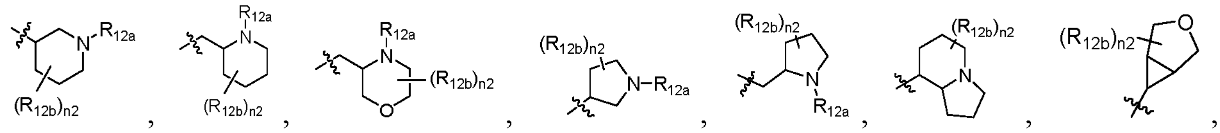
, , , , , , ,

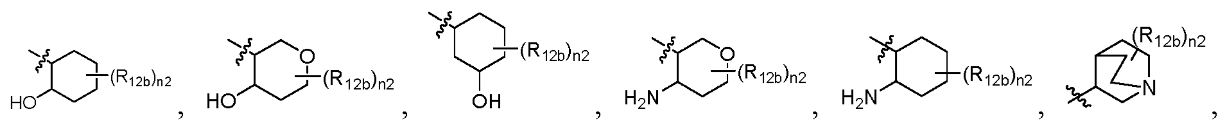
, , , , , ,

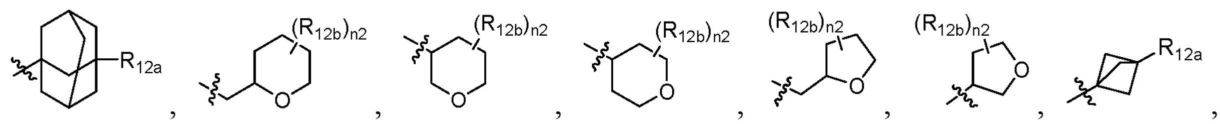
, , , , , , ,

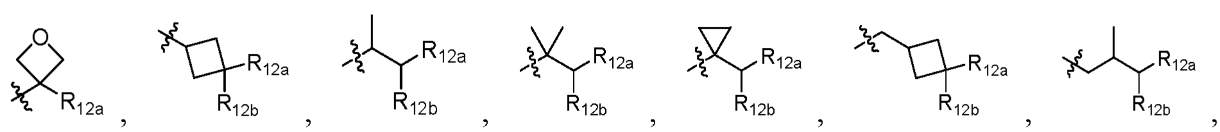
, , , , , , ,

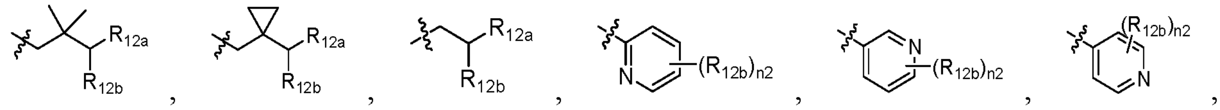
, , , , , ,

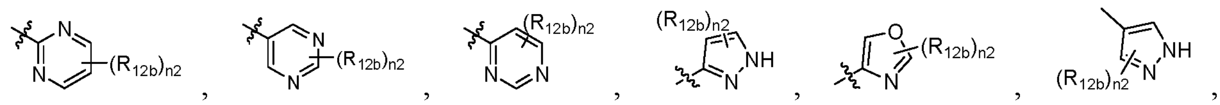
, , , , , ,

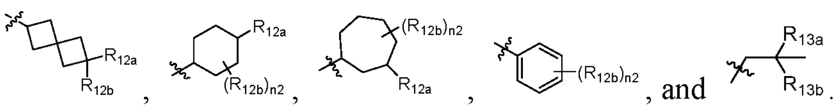
, , , , and .

$R_{12a}$ and $R_{12b}$ are each independently selected from $R_{8a}$, halogen, the oxo group, $-OR_{8a}$, $-SR_{8a}$, $-C(=O)R_{8a}$, $-OC(=O)R_{8a}$, $-C(=O)OR_{8a}$, $-C(=O)NR_{8a}R_{8b}$, $-NR_{8a}C(=O)R_{8b}$, $-NR_{8a}R_{8b}$, $-SO_2R_{8a}$, $-SO_2NR_{8a}R_{8b}$, $-NR_{8a}SO_2R_{8b}$, and -CN, and n2 is an integer selected from 0 to 6.

In some preferred embodiments of the present invention, $R_{12a}$ and $R_{12b}$ are each independently selected from $R_{8a}$, fluorine, the oxo group, $-OR_{8a}$, $-SR_{8a}$, $-C(=O)R_{8a}$, $-OC(=O)R_{8a}$, $-C(=O)OR_{8a}$, $-C(=O)NR_{8a}R_{8b}$, $-NR_{8a}C(=O)R_{8b}$, $-NR_{8a}R_{8b}$, $-SO_2R_{8a}$, $-SO_2NR_{8a}R_{8b}$, $-NR_{8a}SO_2R_{8b}$, and -CN, and n2 is an integer selected from 0 to 3.

In some more preferred embodiments of the present invention, $R_{12a}$ and $R_{12b}$ are each independently selected from fluorine, chlorine, the hydroxy group, the cyanogroup, the oxo group, $C_{1-4}$ alkyl, $C_{1-4}$ fluoroalkyl, $C_{1-4}$ deuterated alkyl, $C_{1-4}$ alkoxy, $C_{1-4}$ fluoroalkoxy, $C_{1-4}$ deuterated alkoxy, 3- to 6-membered cycloalkyl, 3- to 6-membered fluorocycloalkyl, the amino group, the dimethylamino group,

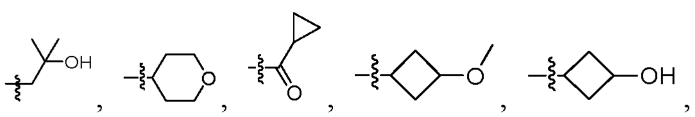
, , , , ,

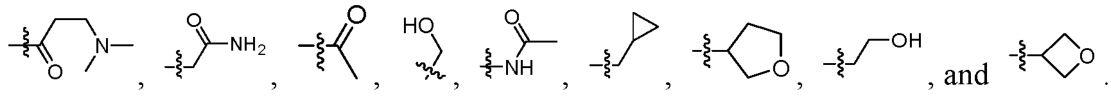
, , , , , , , , , and .

In some embodiments of the present invention, $R_6$ is selected from the following structures:

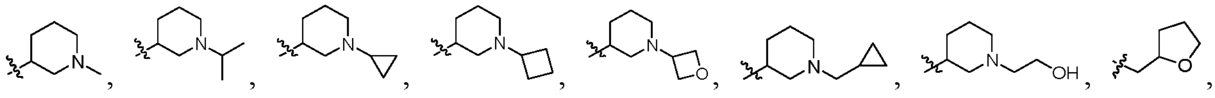
, , , , , , , ,

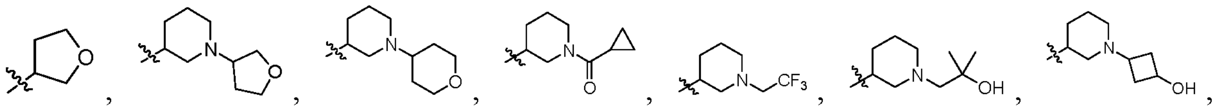
, , , , , , ,

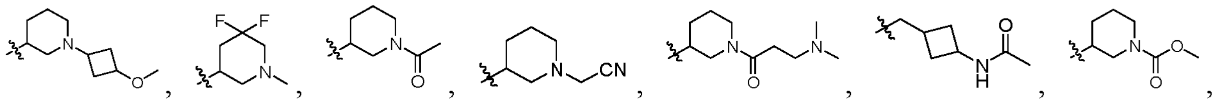
, , , , , , ,

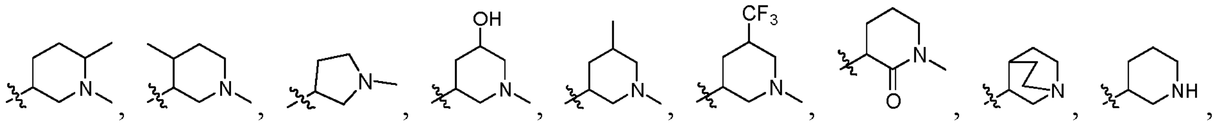
, , , , , , , , ,

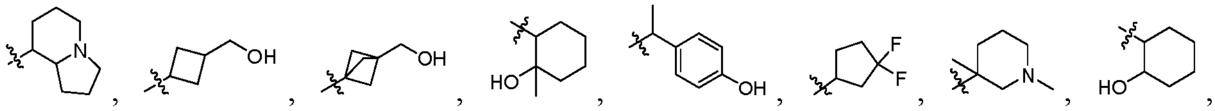
, , , , , , , , ,

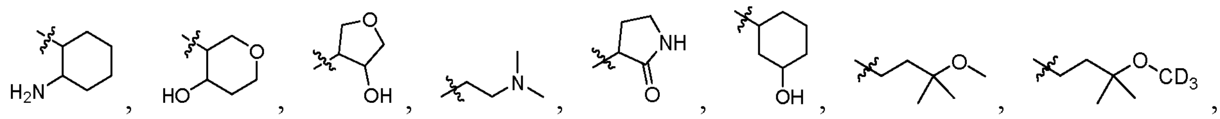
, , , , , , , ,

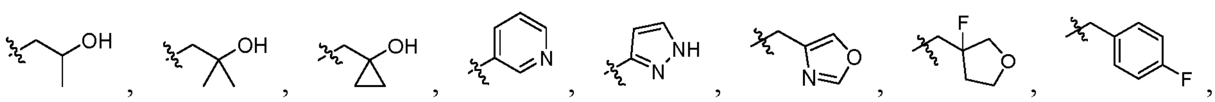
, , , , , , , ,

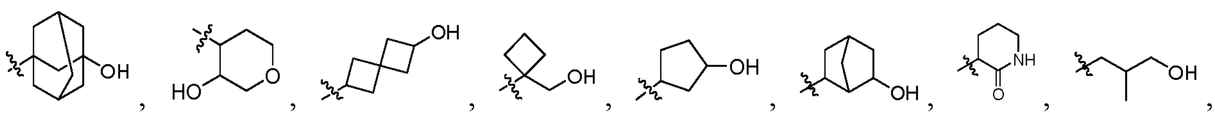
, , , , , , , ,

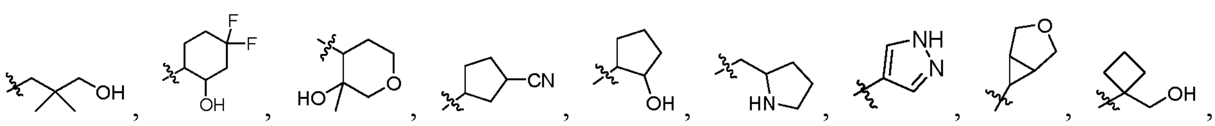
, , , , , , , , ,

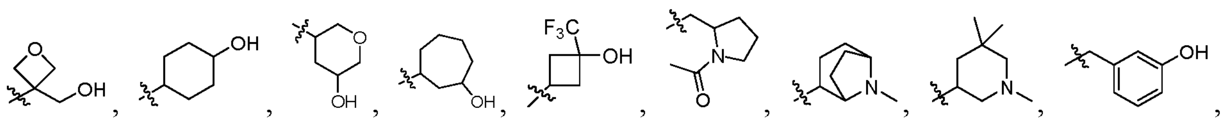
, , , , , , , , ,

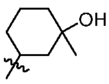 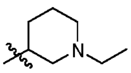, 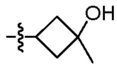, 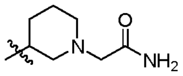, 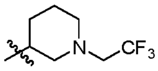, 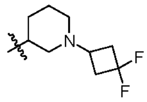, 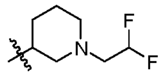,

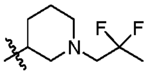, 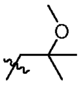, 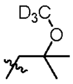, 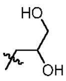, 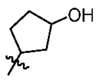, 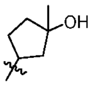, 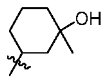, 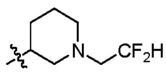, 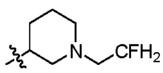,

and

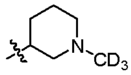.

The present invention further provides some specific compounds, where the specific compounds are selected from:

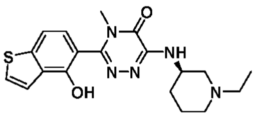, 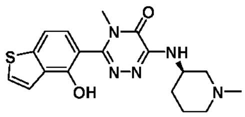, 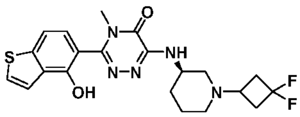, 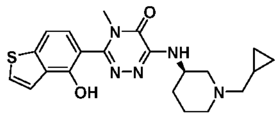,

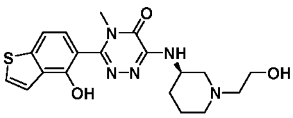, 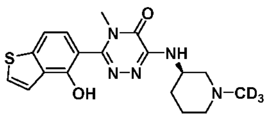, 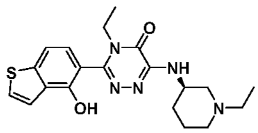, 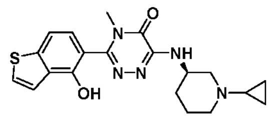,

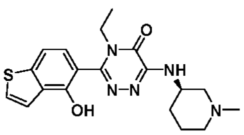, 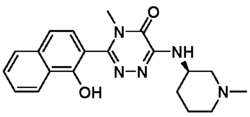, 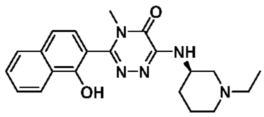, 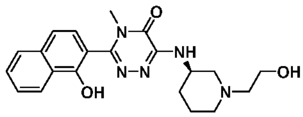,

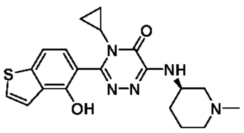, 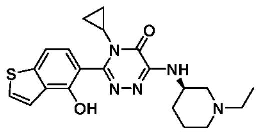, 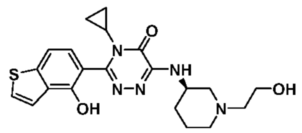, 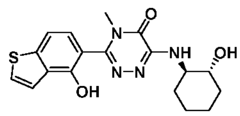,

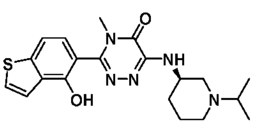, 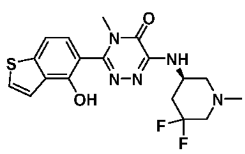, 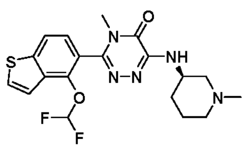, or 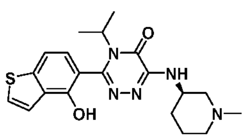.

The present invention further provides another similar compound; in some embodiments of the present invention, a structural unit

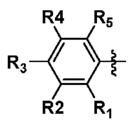

is selected from:

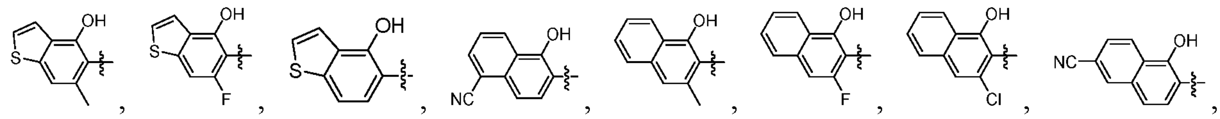

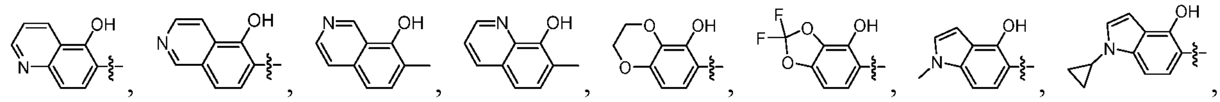

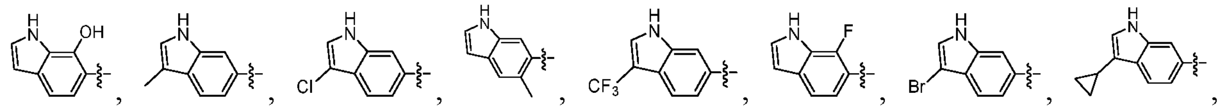

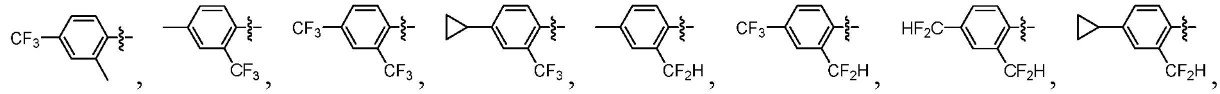

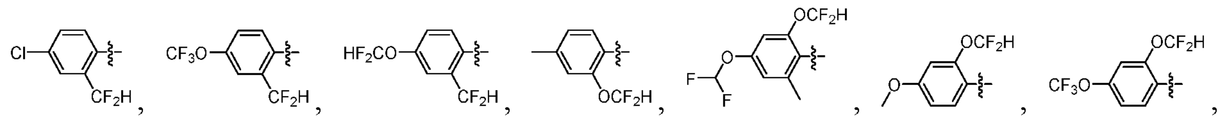

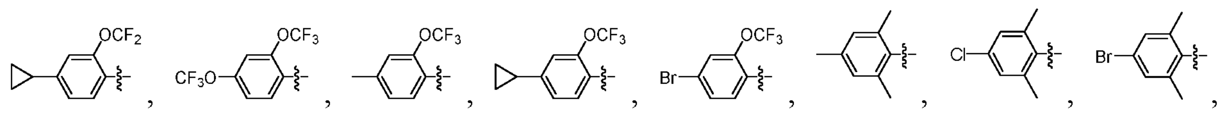

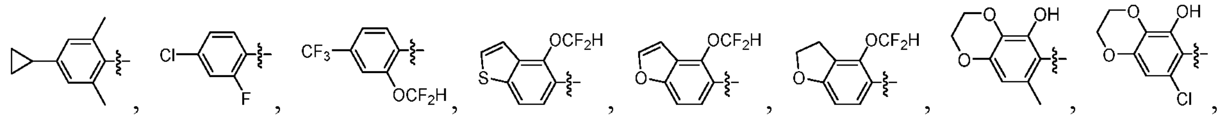

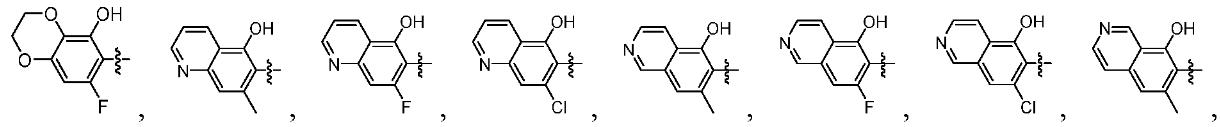

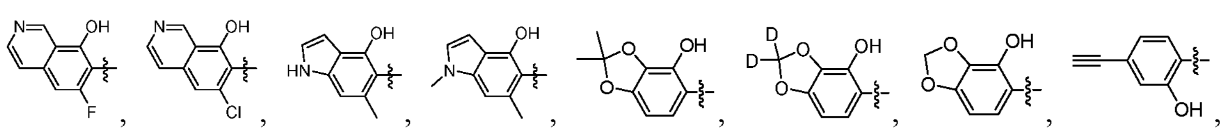

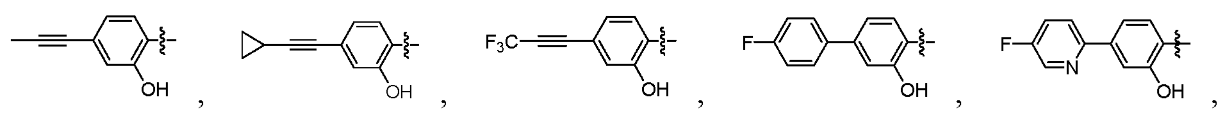

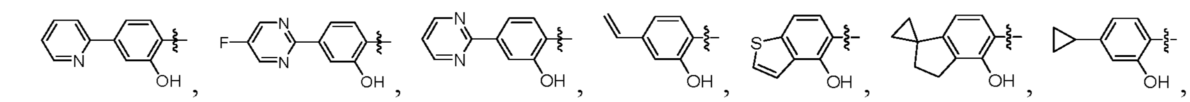

and

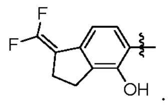
.

In some embodiments of the present invention, a structural unit

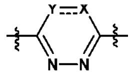

is selected from:

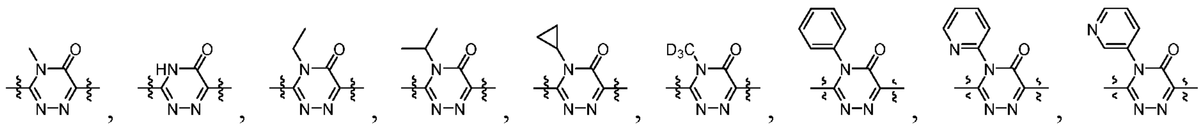
, , , , , , , , , ,

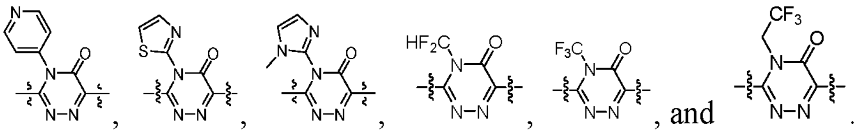
, , , , , and .

In some embodiments of the present invention, L is selected from O, -NH-, $-NH-CH_2-$, and $-NH-CH(CH_3)-$.

In some embodiments of the present invention, $R_6$ is selected from the following structures:

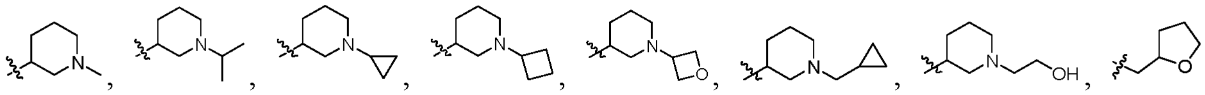
, , , , , , , ,

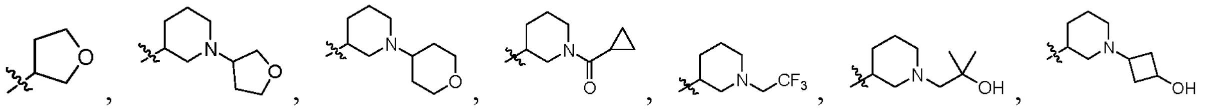
, , , , , , ,

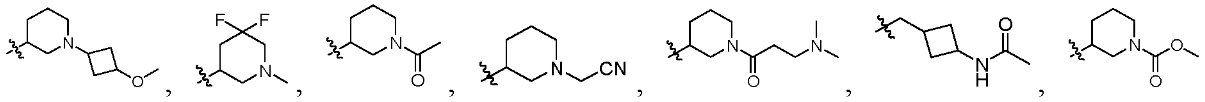
, , , , , , ,

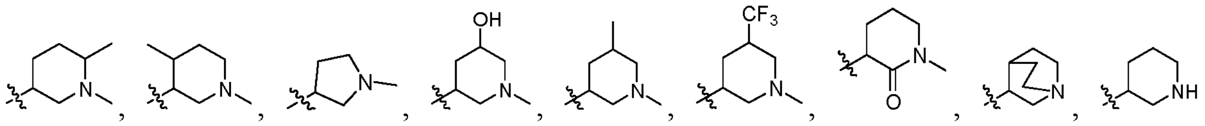
, , , , , , , , ,

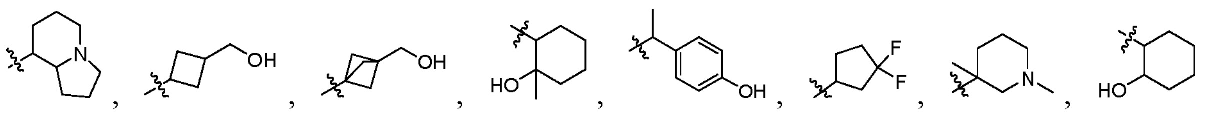
, , , , , , , ,

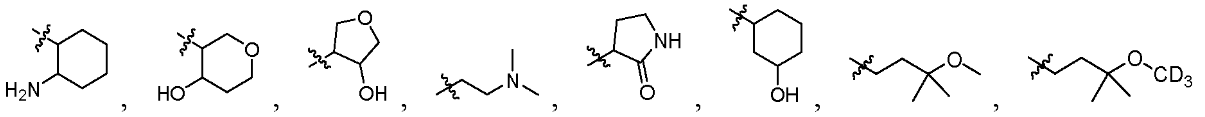
, , , , , , , ,

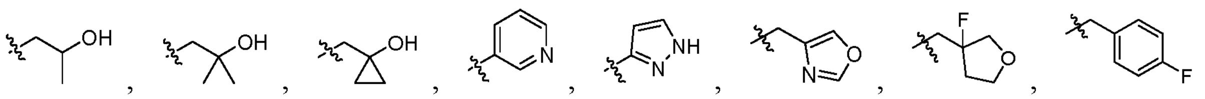
, , , , , , , ,

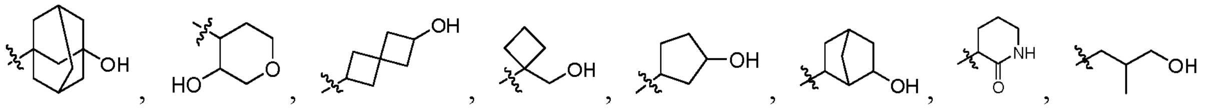
, , , , , , , ,

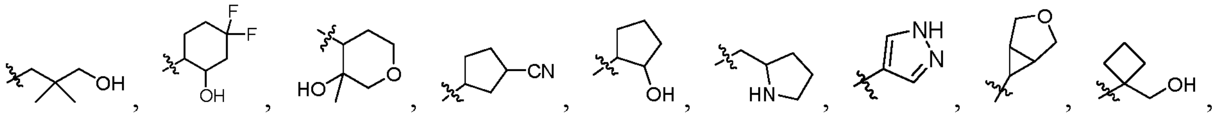
, , , , , , , , ,

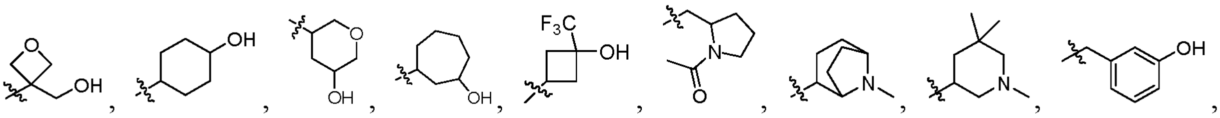
, , , , , , , , ,

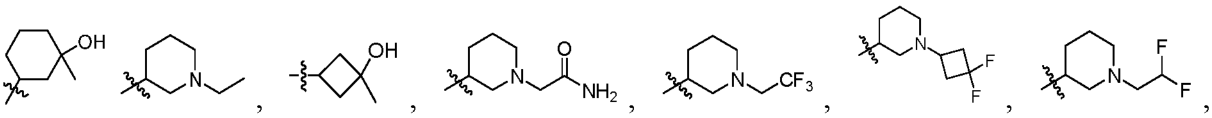
, , , , , ,

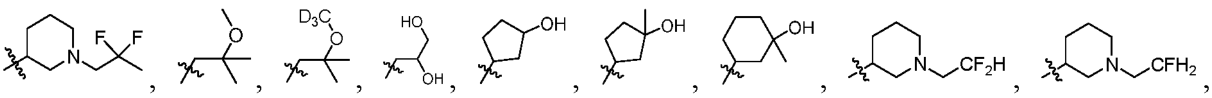
, , , , , , , , ,

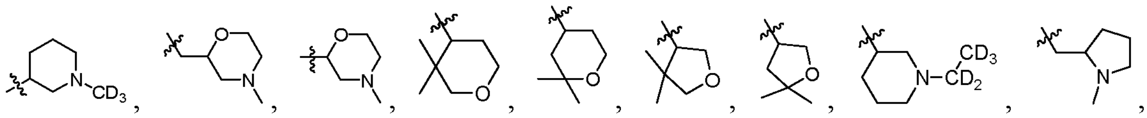
, , , , , , , , ,

and

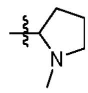
.

The present invention further provides some specific compounds, where the specific compounds are selected from:

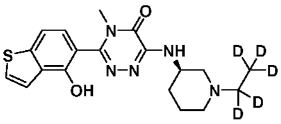
,
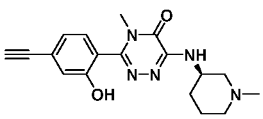
,
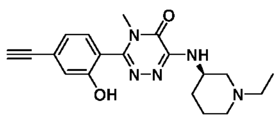
,
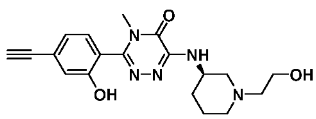
,

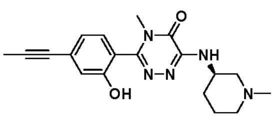
,
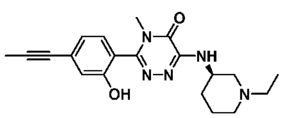
,
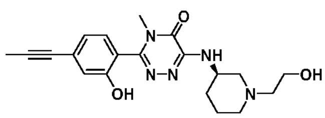
,
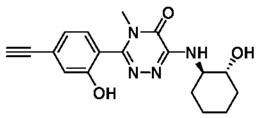
,

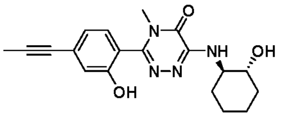
,
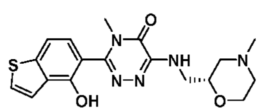
,
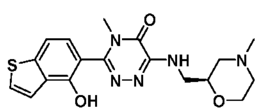
,
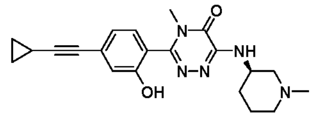
,

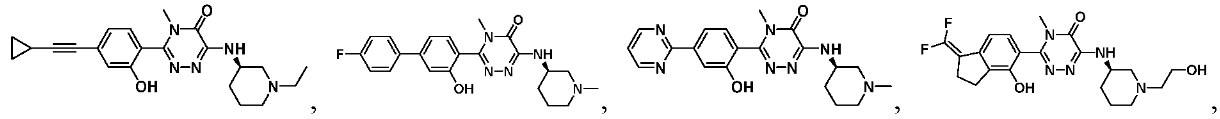

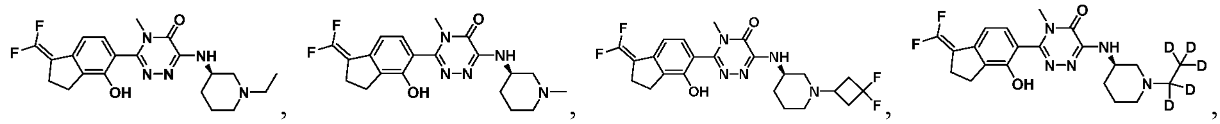

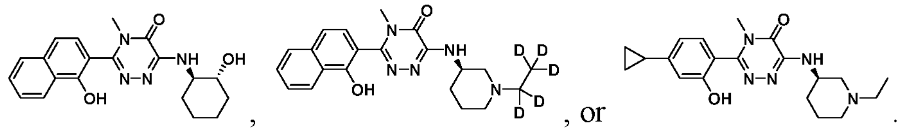

or

In some embodiments of the present invention, a structural unit

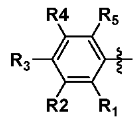

is selected from:

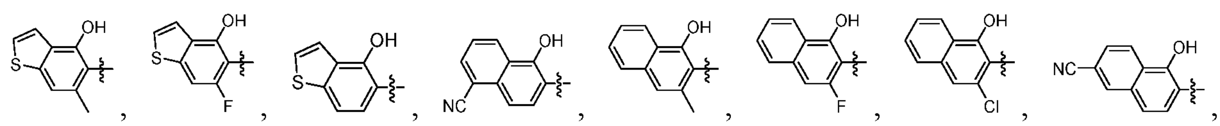

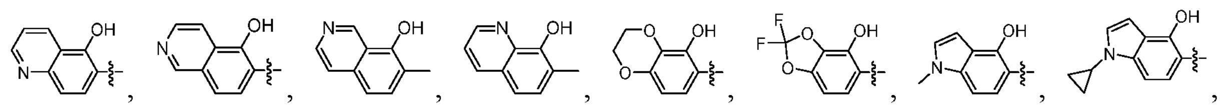

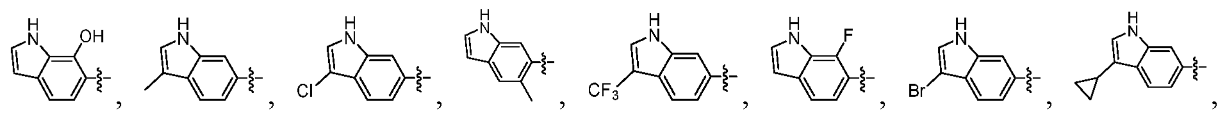

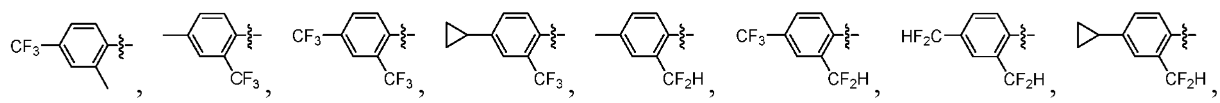

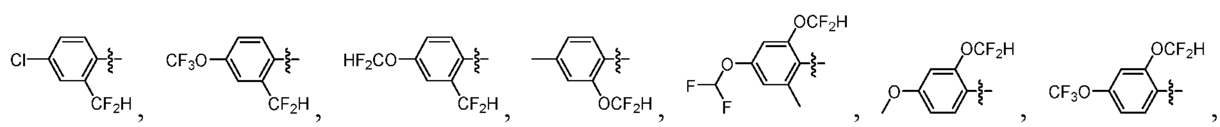

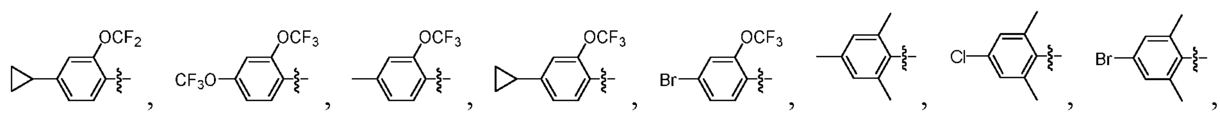

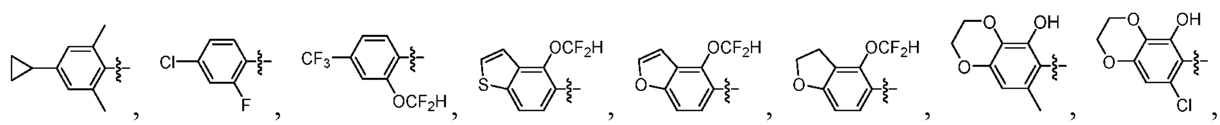

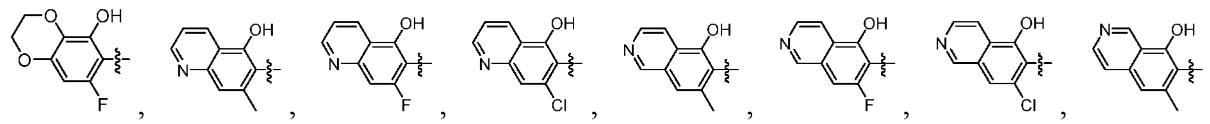

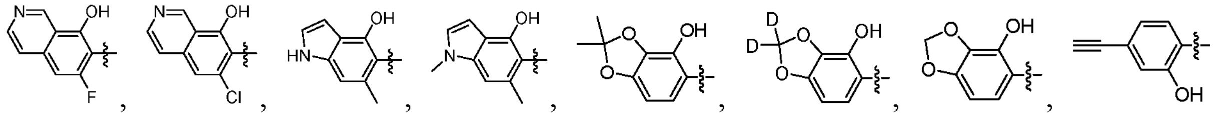

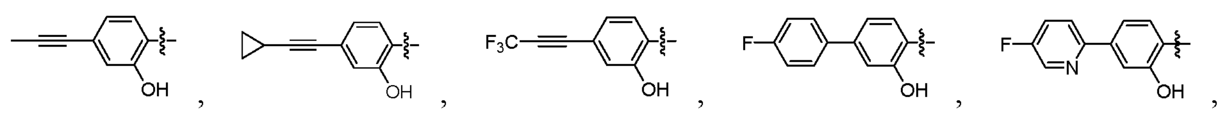

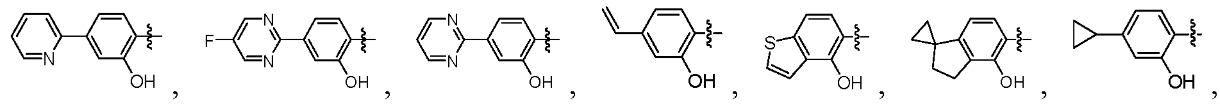

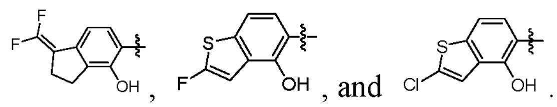

In some embodiments of the present invention, a structural unit

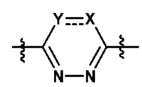

is selected from:

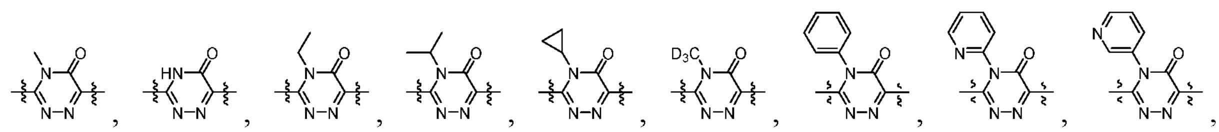

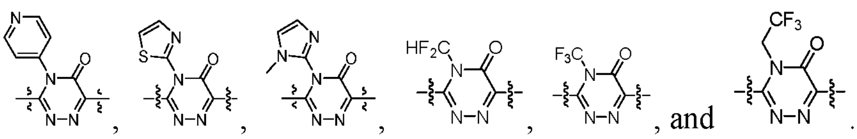

In some embodiments of the present invention, L is selected from O, -NH-, $-NH-CH_2-$, and $-NH-CH(CH_3)-$.

In some embodiments of the present invention, $R_6$ is selected from the following structures:

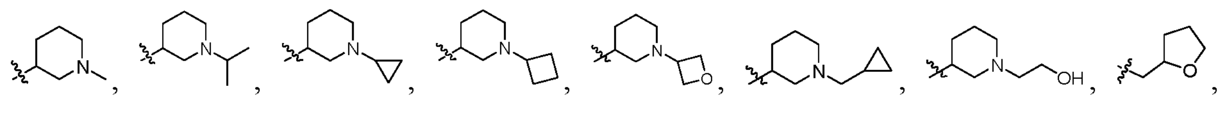

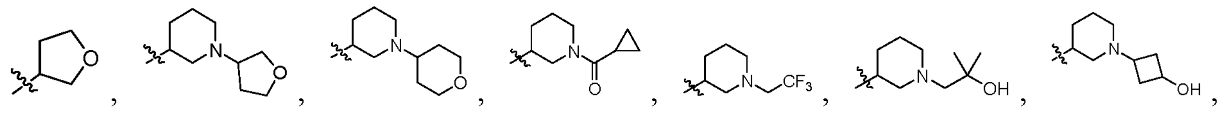

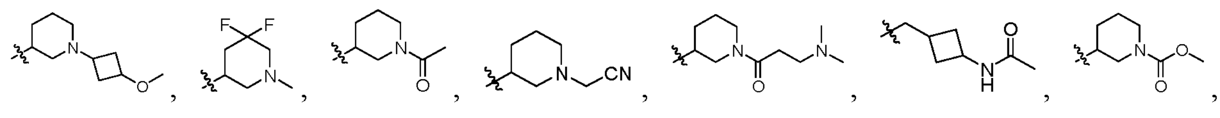

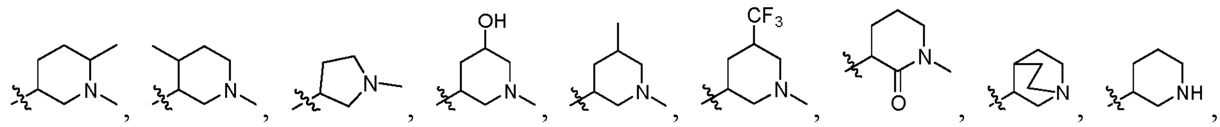
, , , , , , , , ,

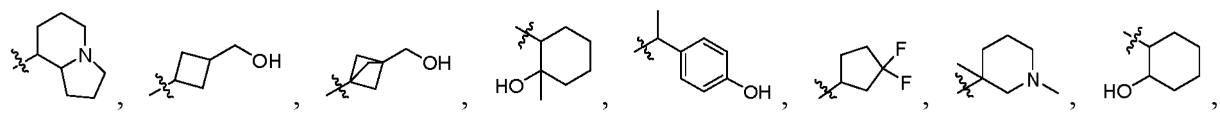
, , , , , , , ,

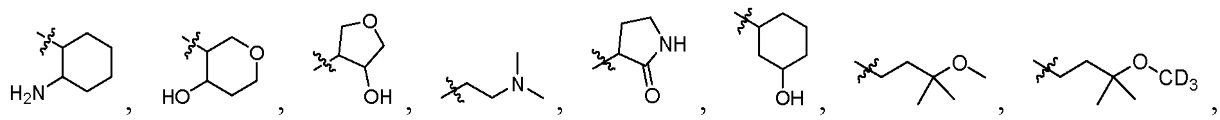
, , , , , , , ,

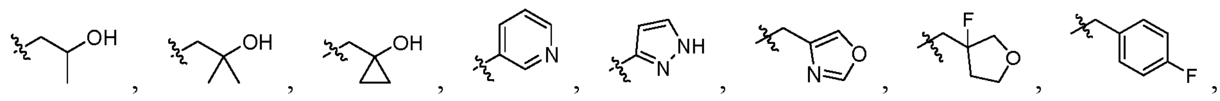
, , , , , , , ,

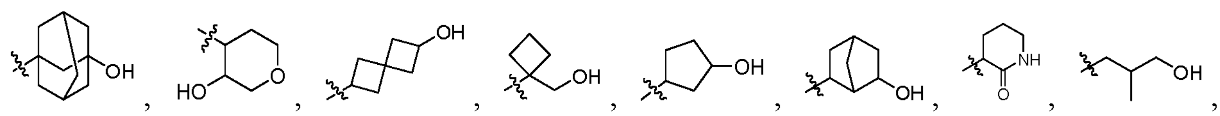
, , , , , , , ,

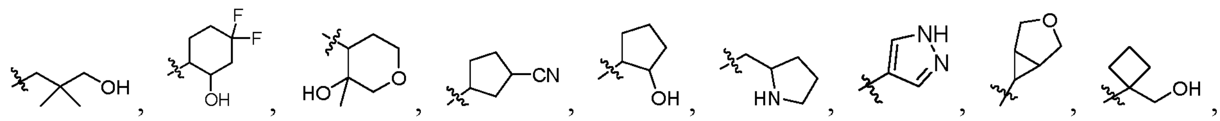
, , , , , , , , ,

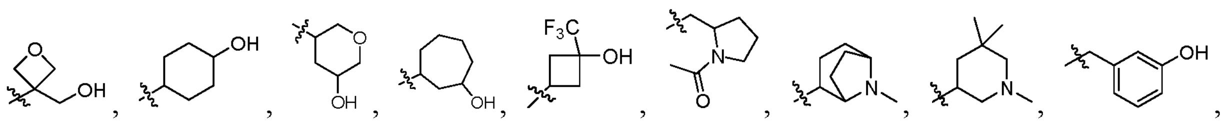
, , , , , , , , ,

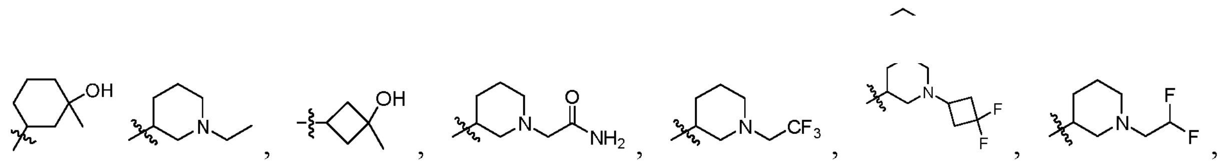
, , , , , ,

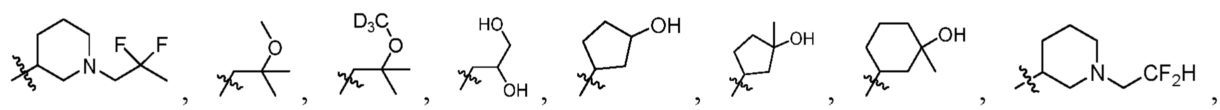
, , , , , , , ,

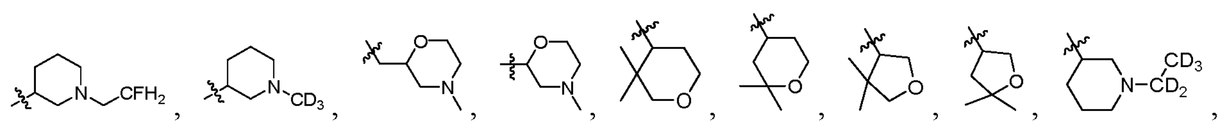
, , , , , , , , ,

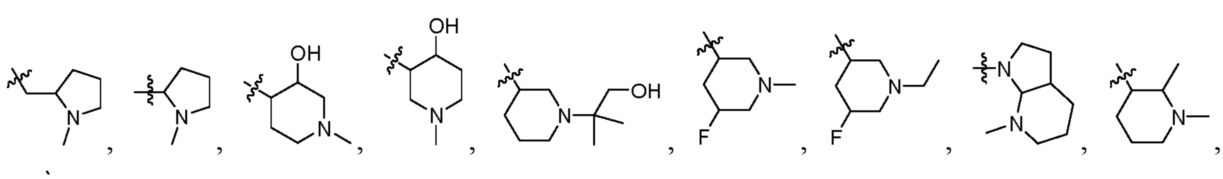
, , , , , , , , ,

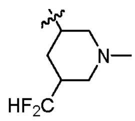, and 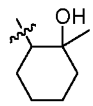.

The present invention further provides some specific compounds, where the specific compounds are selected from:

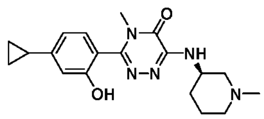, 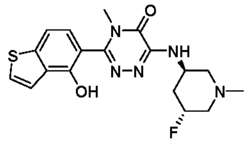, 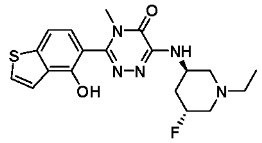, 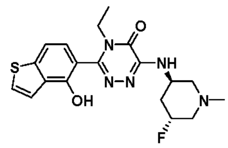,

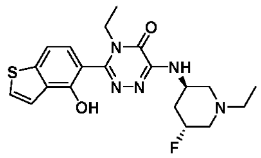, 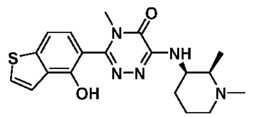, 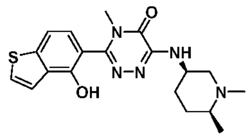, 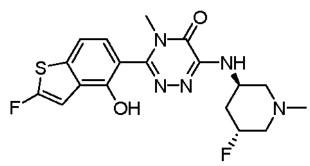,

or

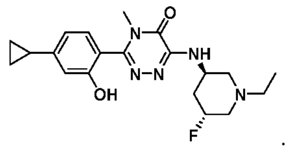.

In some embodiments of the present invention, a structural unit

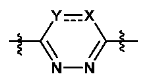

is selected from:

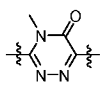, 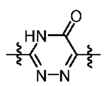, 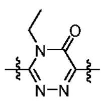, 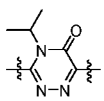, 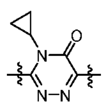, 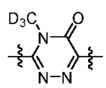, 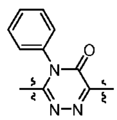, 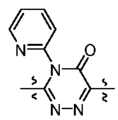, 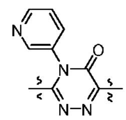,

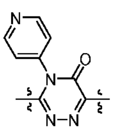, 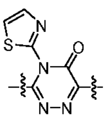, 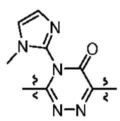, 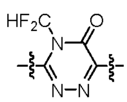, 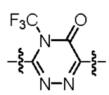, 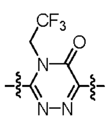, and 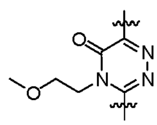.

In some embodiments of the present invention, $R_6$ is selected from the following structures:

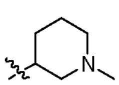, 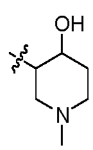, 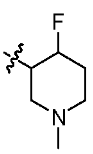, 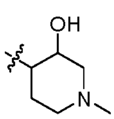, 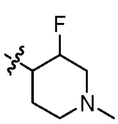, 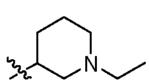, and 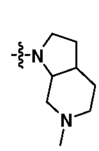.

The present invention further provides some specific compounds, where the specific compounds are selected from:

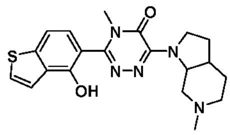, 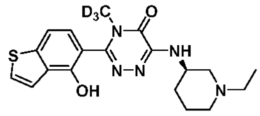, or 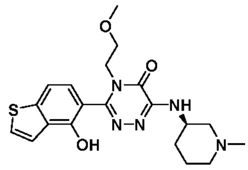.

In a second aspect, the present invention provides a pharmaceutical composition, where the aforementioned pharmaceutical composition takes the compound shown in formula **I, or** pharmaceutically acceptable salts, esters, stereoisomers, tautomers, polymorphs, solvates, nitrogen oxides, isotope markers, metabolites, or prodrugs thereof as active ingredients, together with a pharmaceutically acceptable carrier.

A further objective of the present invention is to provide a method for preparing the pharmaceutical composition of the present invention, and the method includes combining a compound containing formula I or a pharmaceutically acceptable form thereof, or a mixture thereof, with one or more pharmaceutically acceptable carriers.

Pharmaceutically acceptable carriers that can be used in the pharmaceutical composition of the present invention are pharmaceutically acceptable carriers, and examples of suitable pharmaceutically acceptable carriers are described in Remington's Pharmaceutical Sciences (2005).

The pharmaceutical composition can be administered in any form as long as the pharmaceutical composition can prevent, alleviate, or cure the symptoms of human or animal patients. For example, the pharmaceutical composition can be made into various suitable dosage forms according to an administration route.

In other some embodiments, the administration of the compound or pharmaceutical composition of the present invention can be combined with another treatment method. Another treatment method may be selected from, but not limited to, radiotherapy, chemotherapy, immunotherapy, or a combination thereof.

The present invention further relates to a pharmaceutical preparation, and the pharmaceutical preparation takes a compound of formula I or a pharmaceutically acceptable form thereof, or a mixture thereof, or a pharmaceutical composition of the present invention as active ingredients. **In** some embodiments, the pharmaceutical preparation is a solid preparation, a semi-solid preparation, a liquid preparation, or a gaseous preparation.

A further objective of the present invention is to provide a product, such as a kit. The product used herein includes but is not limited to medicine boxes and packages. The product of the present invention includes: (a) a first container; (b) a pharmaceutical composition in the first container, where the composition includes: a first therapeutic agent, and the first therapeutic agent includes: a compound containing formula **I,** a pharmaceutically acceptable form thereof, or a mixture thereof; (c) optional package instructions indicating that the pharmaceutical composition can be used for treating tumor diseases (as defined below); and (d) a second container.

The first container is a container for containing the pharmaceutical composition. The container can be used for preparation, storage, transportation and/or independent/bulk sales. The first container is intended to cover bottles, cans, vials, flasks, syringes, tubes (for example, for cream products), or any other container for preparing, containing, storing or dispensing pharmaceutical products.

The second container is a container for containing the first container and the optional packaging instructions. Examples of the second container include, but are not limited to, boxes (for example, cartons or plastic boxes), cases, cartons, bags (for example, paper bags or plastic bags), small bags, and burlap bags. The packaging instructions can be physically attached outside the first container via a cable tie, glue, staple or other attachment methods, or can be placed inside the second container without any physical tools for attachment to the first container. Alternatively, the packaging instructions are located outside the second container. Preferably, the package instructions located outside the second container are physically attached via the cable tie, glue, staple or other attachment methods. Alternatively, the package instructions may be adjacent to or in contact with the outside of the second container without physical attachment.

The package instructions are trademarks, labels, signs, and others, and list information related to the pharmaceutical composition located in the first container. The information listed is usually determined by a regulatory agency (such as the US Food and Drug Administration) that governs an area where the product is to be sold. Preferably, the packaging instructions specifically list indications for which the pharmaceutical composition is approved. The packaging instructions can be made of any material from which information contained therein or thereon can be read. Preferably, the package instructions are of printable materials (for example, paper, plastic, cardboard, foil, adhesive paper or plastic) on which required information can be impressed (for example, being printed or being coated).

In a third aspect, the present invention provides a use of the aforementioned compound of formula I, and specific compounds or pharmaceutically acceptable forms thereof, or the pharmaceutical composition of the present invention in preparation of drugs for prevention and/or treatment of NLRP3-related diseases.

The present invention provides a method for preventing or treating NLRP3-related diseases, and the method includes administering the above-mentioned compound of formula I, the pharmaceutically acceptable form thereof, or the pharmaceutical composition of the present invention to an individual in need.

The present invention provides a method for preventing or treating NLRP3-related diseases by using the compound of formula I or the pharmaceutically acceptable form thereof, or combining the pharmaceutical composition of the present invention with another treatment method, and the another treatment method includes but is not limited to radiotherapy, chemotherapy, immunotherapy, or a combination thereof.
In some embodiments, the NLRP3-related diseases comprise inflammatory diseases, autoimmune diseases, cardiovascular diseases, cancers, renal system diseases, gastrointestinal diseases, respiratory diseases, endocrine system diseases, or central nervous system diseases.
In some embodiments, the NLRP3-related diseases include: cryopyrin-associated periodic syndrome (CAPS), Muckle-Wells syndrome (MWS), familial cold autoinflammatory syndrome (FCAS), neonatal-onset multisystem inflammatory disease (NOMID), familial Mediterranean fever (FMF), non-alcoholic steatohepatitis, alcoholic liver disease, graft-versus-host disease, multiple sclerosis (MS), rheumatoid arthritis, type I/II diabetes mellitus and related complications (for example, nephropathy and retinopathy), psoriasis, Alzheimer's disease, atherosclerosis, gout, chronic kidney disease, sepsis, liver fibrosis, idiopathic pulmonary fibrosis, epilepsy, neuropathic pain, depressive disorders, Parkinson's disease, asthma, acute myocardial infarction, systemic lupus erythematosus, rheumatoid arthritis, Crohn's disease, ulcerative colitis, inflammatory bowel disease, rheumatoid arthritis, ankylosing spondylitis, bronchial asthma, acute respiratory distress syndrome, chronic obstructive pulmonary disease, or ischemic stroke.
In a further preferred embodiment, the compound of the present invention can be used in combination with radiotherapy, chemotherapy or immunotherapy, to prevent or treat the NLRP3-related diseases.

**Beneficial effects of the present invention are that:**

The present invention provides a triazine compound and use thereof, and the compound and the composition can be used to prepare NLRP3 inflammasome inhibitors, thereby providing a new way for treating NLRP3-related diseases.

Definition of terms:

Unless otherwise defined herein below, all technical and scientific terms used in this document are intended to have the meanings as commonly understood by those skilled in the art. The terms "including", "comprising", "having", "containing", or "relating to" and other variant forms thereof in this document are inclusive or open-ended and do not exclude other unenumerated elements or method steps. Those skilled in the art shall understand that the aforementioned terms such as "including" encompass the meaning of "consisting of".
In the present invention, "one", "a", "the", "at least one", and "one or more" can be used interchangeably. Therefore, for example, a composition comprising "a" pharmaceutically acceptable excipient may be interpreted to mean that the composition includes "one or more" pharmaceutically acceptable excipients.
For example, the expression "$C_{1-4}$" shall be understood to cover any sub-ranges therein and each point value, such as $C_{2-4}$, $C_{3-4}$, $C_{1-2}$, $C_{1-3}$, $C_{1-4}$, and others, as well as $C_1$, $C_2$, $C_3$, $C_4$, and others.
In the present invention, unless otherwise specified, halogen refers to fluorine, chlorine, bromine, or iodine.
In the present invention, unless otherwise specified, the term "alkyl" comprises straight-chain or branched monovalent saturated hydrocarbon groups. For example, alkyl groups include methyl, ethyl, propyl, isopropyl, n-butyl, isobutyl, sec-butyl, tert-butyl, n-pentyl, 3-(2-methyl) butyl, 2-pentyl, 2-methylbutyl, neopentyl, n-hexyl, 2-hexyl, 2-methylpentyl, and others. Similarly, $C_{1-4}$ in "$C_{1-4}$ alkyl" refers to a group having 1, 2, 3, or 4 carbon atoms arranged in a straight or branched chain form.
In the present invention, unless otherwise specified, the term "cycloalkyl", "carbocyclic" or "cycloalkylene" refers to a saturated or partially saturated, monocyclic or polycyclic (such as bicyclic) non-aromatic hydrocarbon group. Common cycloalkyl groups include, but are not limited to, monocyclic cycloalkyl groups such as cyclopropyl, cyclobutyl, cyclopentyl, cyclohexyl, cycloheptyl, cyclooctyl, cyclononyl, cyclobutenyl, cyclopentenyl, cyclohexenyl, and others; or bicyclic cycloalkyl groups, including fused, bridged, or spiro rings, such as bicyclo [1.1.1] pentyl, bicyclo [2.2.1] heptyl, bicyclo [3.2.1] octyl, bicyclo [5.2.0] nonyl, decahydronaphthyl, and others. For example, the term "$C_{3-12}$ cycloalkyl" refers to a cycloalkyl group having 3 to 12 ring carbon atoms (such as 3, 4, 5, 6, 7, 8, 9, 10, 11, or 12). The cycloalkyl or cycloalkylene group in the present invention is optionally substituted with one or more substituents described herein.
In the present invention, unless otherwise specified, the term "fluoroalkyl" refers to the aforementioned alkyl group, wherein one or more hydrogen atoms are substituted with fluorine atoms. For example, the term "$C_{1-4}$ fluoroalkyl" refers to a $C_{1-4}$ alkyl group optionally substituted with one or more (such as 1 to 3) fluorine atoms. Those skilled in the art shall understand that when there is more than one fluorine atom substituent, the fluorine atoms may be the same or different and may be located on the same or different C atoms. Examples of haloalkyl groups include, but are not limited to, $-CH_2F$, $-CHF_2$, $-CF_3$, $-C_2F_5$, $-CH_2CF_3$, and others. The fluoroalkyl group in the present invention is optionally substituted with one or more substituents described herein.
The present invention also encompasses all pharmaceutically acceptable isotopically labeled compounds that

are identical to the compounds of the present invention, except that one or more atoms are replaced by atoms having the same atomic number but an atomic mass or mass number different from the atomic mass or mass number predominant in nature. Examples of isotopes suitable for incorporation into the compounds of the present invention include, but are not limited to: isotopes of hydrogen (e.g., deuterium ($^2H$) and tritium ($^3H$)); isotopes of carbon (e.g., $^{13}C$ and $^{14}C$); isotopes of chlorine (e.g., $^{37}Cl$); isotopes of iodine (e.g., $^{125}I$); isotopes of nitrogen (e.g., $^{13}N$ and $^{15}N$); isotopes of oxygen (e.g., $^{17}O$ and $^{18}O$); isotopes of phosphorus (e.g., $^{32}P$); and isotopes of sulfur (e.g., $^{34}S$).

In present invention, the term "polymorph" refers to different solid crystalline phases of certain compounds of the present invention arising from the existence of two or more distinct molecular arrangements in the solid state. Certain compounds of the present invention may exist in more than one crystalline form, and the present invention is intended to encompass all such crystalline forms and mixtures thereof. Generally, crystallization results in the formation of solvates of the compounds of the present invention. As used in the present invention, the term "solvate" refers to an aggregate comprising one or more molecules of a compound of the present invention and one or more solvent molecules. The solvent may be water, in which case the solvate is a hydrate. Alternatively, the solvent may be an organic solvent. Therefore, the compounds of the present invention may exist as hydrates, including monohydrates, dihydrates, hemihydrates, sesquihydrates, trihydrates, tetrahydrates, and others, as well as corresponding solvated forms. The compounds of the present invention may form true solvates, but in some cases, they may also retain merely non-stoichiometric water or a mixture of water combined with a portion of non-stoichiometric solvents. The compounds of the present invention may be reacted in a solvent or precipitated or crystallized from a solvent. Solvates of the compounds of the present invention are also encompassed within the scope of the present invention. The present invention also encompasses all possible crystalline forms or polymorphs of the compounds of the present invention, which may be a single polymorph or a mixture of more than one polymorph in any ratio.

In the present invention, the term "stereoisomers" refer to isomers formed resulting from the presence of at least one asymmetric center. In compounds possessing one or more (e.g., one, two, three, or four) asymmetric centers, they can give rise to racemic mixtures, single enantiomers, diastereomer mixtures, and individual diastereomers. Certain individual molecules may also exist as geometric isomers (cis/trans). Similarly, the compounds of the present invention may exist as a mixture of two or more structurally different forms in rapid equilibrium (commonly referred to as tautomers). Representative examples of tautomers include keto-enol tautomers, phenol-keto tautomers, nitroso-oxime tautomers, and imine-enamine tautomers. It is to be understood that the scope of the present invention encompasses all such isomers or mixtures thereof in any ratio (e.g., 60%, 65%, 70%, 75%, 80%, 85%, 90%, 95%, 96%, 97%, 98%, and 99%).

In the present invention, pharmaceutically acceptable salts encompass acid addition salts and base addition salts thereof. The suitable acid addition salt is formed from an acid that forms a pharmaceutically acceptable salt. The suitable base addition salts are formed from a base that forms a pharmaceutically acceptable salt. For an overview of suitable salts, reference may be made to, for example, Remington's Pharmaceutical Sciences, Mack Publishing Company, Easton, Pa., (2005); and Handbook of Pharmaceutical Salts: Properties, Selection, and Use, Stahl and Wermuth (Wiley-VCH, Weinheim, Germany, 2002). Methods for preparing pharmaceutically acceptable salts of the compounds of the present invention are known to those skilled in the art. The term "pharmaceutically acceptable acid addition salt" refers to a salt formed with an inorganic acid or an organic acid that retains the bioavailability of the free base without other adverse effects. Inorganic acid salts include, but are not limited to, hydrochloride, hydrobromide, sulfate, nitrate, phosphate, and others; organic acid salts include, but are not limited to, formate, acetate, 2,2-dichloroacetate, trifluoroacetate, propionate, caproate, caprylate, caprate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalenedisulfonate, and others. These salts may be prepared by methods known in this patent. The term "pharmaceutically acceptable base addition salts" refer to salts formed with inorganic or organic bases that retain the biological effectiveness of the free acid without other adverse effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and others. Preferred inorganic salts are ammonium salts, sodium salts, calcium salts and magnesium salts. Salts derived from organic bases include, but are not limited to, salts of: primary amines, secondary amines, and tertiary amines, substituted amines, including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resins, and others. Preferred organic bases include isopropylamine, diethylamine, ethanolamine, trimethylamine, dicyclohexylamine, choline, and hexocaffeine. These salts may be prepared by methods known in this patent.

In the present invention, unless otherwise specified, the term "ester" refers to esters derived from the compounds described herein, including physiologically hydrolyzable esters (which can be hydrolyzed under physiological conditions

to release the compound of the present invention in free acid or alcohol form). The compounds of the present invention may also be esters themselves.

The compounds of the present invention may exist in the form of solvates (preferably hydrates), wherein the compounds of the present invention comprise a polar solvent, particularly such as water, methanol, or ethanol, as a structural element of the crystal lattice of the compounds. The amount of polar solvent, particularly water, may be present in a stoichiometric or non-stoichiometric ratio.

Those skilled in the art will understand that not all nitrogen heterocyclic rings are capable of forming N-oxides, as nitrogen requires an available lone pair of electrons to be oxidized into an oxide. Those skilled in the art will recognize nitrogen heterocyclic rings capable of forming N-oxides. Those skilled in the art will also recognize that tertiary amines are capable of forming N-oxides. The synthetic methods for preparing N-oxides of heterocyclic rings and tertiary amines are well-known to those skilled in the art, including oxidation of heterocyclic rings and tertiary amines using peroxy acids such as peroxyacetic acid and m-chloroperoxybenzoic acid (mCPBA), hydrogen peroxide, alkyl hydroperoxides such as tert-butyl hydroperoxide, sodium perborate, and dioxiranes such as dimethyldioxirane. These methods for preparing N-oxides have been extensively described and reviewed in the literature. See, for example: T.L. Gilchrist, Comprehensive Organic Synthesis, vol. 7, pp. 748-750 (A.R. Katritzky and A.J. Boulton, Eds., Academic Press); and G.W.H. Cheeseman and E.S.G Werstiuk, Advances in Heterocyclic Chemistry, vol. 22, pp. 390-392 (A.R. Katritzky and A.J. Boulton, Eds., Academic Press).

In the present invention, the term "metabolite" refers to a substance formed in the body when a compound of the present invention is administered. The metabolites of the compound may be identified using techniques well-established in the art, and their activity may be characterized through experimental methods. For example, such products may arise from oxidation, reduction, hydrolysis, amidation, deamidation, esterification, enzymolysis, and other reactions of the administered compound. Therefore, the present invention encompasses metabolites of the compounds of the present invention, including compounds produced by a method comprising contacting the compounds of the present invention with a mammal for a time sufficient to obtain their metabolites.

In the present invention, the term "prodrug" refers to certain derivatives of the compounds of the present invention that, when administered into or onto the body, can be converted, for example, by hydrolytic cleavage, into a compound of the present invention having the desired activity. Typically, such a prodrug will be a functional group derivative of the compound that is readily convertible in vivo to the desired therapeutically active compound. Additional information regarding the use of prodrugs may be found in Pro-drugs as Novel Delivery Systems, Volume 14, ACS Symposium Series (T. Higuchi and V. Stella). For example, the prodrug of the present invention may be prepared by substituting suitable functional groups present in the compounds of the present invention with certain moieties known to those skilled in the art as "pro-moieties" (e.g., as described in Design of Prodrugs by H. Bundgaard, Elsevier, 1985).

In the present application, the term "pharmaceutical composition" refers to a formulation of the compounds of the present invention with a medium generally accepted in the art for delivering biologically active compounds to mammals, such as humans. The medium comprises pharmaceutically acceptable carriers. An object of the pharmaceutical composition is to facilitate the administration to organisms, enhance the absorption of active ingredients, and thereby exert biological activity.

In the present application, the term "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authority or accepted for use in humans or domestic animals.

The terms "drug combination", "drug co-administration", "combination therapy", "administration of another therapy", "administration of another therapeutic agent", and others, as used herein, refer to a therapeutic regimen obtained by mixing or combining more than one active ingredient, encompassing both fixed and non-fixed combinations of active ingredients. The term "fixed combination" refers to the simultaneous administration to a patient of at least one compound described herein and at least one synergistic agent in the form of a single entity or dosage form. The term "non-fixed combination" refers to the simultaneous administration, co-administration, or sequential administration at variable intervals of at least one compound described herein and at least one synergistic agent as separate entities to a patient. These principles are also applied in cocktail therapy, such as the administration of three or more active ingredients.

In the present invention, unless otherwise specified, the term "tumor" includes but is not limited to leukemia, gastrointestinal stromal tumor, histiocytic lymphoma, non-small cell lung cancer, small cell lung cancer, pancreatic cancer, lung squamous cell carcinoma, lung adenocarcinoma, breast cancer, prostate cancer, liver cancer, skin cancer, epithelial cell carcinoma, cervical cancer, ovarian cancer, colorectal cancer, nasopharyngeal cancer, brain cancer, bone cancer, esophageal cancer, melanoma, renal cancer, oral cancer, and other diseases.

In the present invention, unless otherwise specified, the term "treatment" refers to reversing, alleviating, inhibiting the progression of a disorder or condition to which such term is applied, or one or more symptoms thereof, or preventing such disorder or condition or one or more symptoms thereof.

Without departing from the common general knowledge in the art, the aforementioned preferred technical

features may be freely combined in any manner, thereby yielding the various preferred embodiments of the present invention.

## DESCRIPTION OF THE EMBODIMENTS

The solution of the present invention will be explained with reference to embodiments. It will be understood by those skilled in the art that the following embodiments are merely intended for illustrating the present invention, instead of limiting the scope of the present invention. If technologies or conditions are not specified in the embodiments, technologies or conditions described in literatures in the art or product specifications should be followed.

Reagents and raw materials used in the embodiments of the present invention are all commercially available.

Table 1 Abbreviations used herein and meanings thereof

| | | | |
|---|---|---|---|
| AcOH | Acetic acid | PreHP LC | Preparative high-performance liquid chromatography |
| Aq or aq | Aqueous solution | m/z | Mass/charge |
| BOC or Boc | t-butyloxycarboryl | Me | Methyl |
| CPME | Cyclopentyl methyl ether | MeCN | Acetonitrile |
| DCE | 1,2-dichloroethane | MeOH | Methyl alcohol |
| DABC O | 1,4-diazabicyclo[2.2.2]octane | NMR | Nuclear magnetic resonance |
| DCM | Dichloromethane | THF | Tetrahydrofuran |
| DMA | N,N-dimethylacetamide | Sat or satd | Saturated |
| DMA P | 4-dimethylaminopyridine | rt | Room temperature |
| EA | Ethyl acetate | UV | Ultraviolet ray |
| PE | Petroleum ether | t-BuO H | Tertiary butanol |
| DMF | N,N-dimethylformamide | Cbz | Benzyloxycarbonyl |
| TFA | Trifluoroacetic acid | mmol | Millimole |
| TEA | Triethylamine | mg | Milligram |
| DME | 1,2-dimethoxyethane | min | Minute |
| DMS O | Dimethyl sulfoxide | mL or mL | Milliliter |
| DPPF or dppf | 1,1'-bis(diphenylphosphino)fer rocene | BINAP | 2,2'-bis(diphenylphosphino)-1,1'-binapht hyl |
| eq or equiv | Equivalent | *m-CPB* A | Metachloroperbenzoic acid |
| ESI | Electrospray ionization | TBS | Tert-butyldimethylsilane |
| Et | Ethyl | $Pd_2$(dba )3 | Tris(dibenzylideneacetone)dipalladium |
| G | Gram | Xantph os | 4,5-bis(diphenylphosphino)-9,9-dimethyl xanthene |
| H | Hour | NBS | N-bromosuccinimide |
| rt | Room temperature | TBAF | Tetrabutylammonium fluoride |
| Dioxa ne | Dioxane | LC-MS | Liquid chromatography-mass spectrometry (LC-MS) spectrometer |

The structure of the compound is determined by nuclear magnetic resonance (NMR) or mass spectrometry (MS). NMR measurements are performed on Bruker AVANCE-400 spectrometer using deuterated dimethyl sulfoxide (DMSO-$d_6$), deuterated chloroform ($CDCl_3$) and deuterated methanol ($CD_3OD$) as solvents. Tetramethylsilane (TMS) is employed as an internal reference standard. A chemical shift is reported in parts per million (ppm, $10^{-6}$).

MS measurements are performed on Agilent SQD (ESI) mass spectrometer (manufacturer: Agilent, signal: 6110).

HPLC measurements are performed on Agilent 1200DAD high pressure liquid chromatograph (Sunfirc C18,

150X 4.6 mm, 5 wn, column) and Waters 2695-2996 high pressure liquid chromatograph (Gimini C18, 150X 4.5 mm, 5 ym column).

Qingdao Haiyang GF254 silica gel plates are used for thin-layer chromatography, silica gel plates used for thin-layer chromatography (TLC) are 0.15 mm-0.2 mm, and silica gel plates used for thin-layer chromatography separation and purification products are 0.4 mm-0.5 mm.

Generally, silica gel with 100-200 meshes and 200-300 meshes from Qingdao Haiyang is used as a carrier for column chromatography.

Unless otherwise specified in the following embodiments, reactions are all carried out in argon atmosphere or nitrogen atmosphere. The argon atmosphere or nitrogen atmosphere refers to a reaction flask being connected to an argon balloon or a nitrogen balloon with a volume of about 1 L. Hydrogen atmosphere refers to a reaction flask being connected to a hydrogen balloon with a volume of about 1 L. Hydrogenation reaction is usually vacuumized and filled with hydrogen, and such operations are repeated three times.

**Intermediate INT1:**

2-[4-methoxybenzo[b]thiophen-5-yl]-4,4,5,5-tetramethyl-1,3,2-dioxaborolane

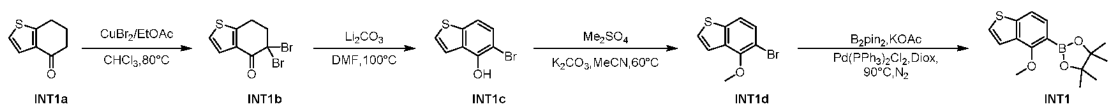


INT1a INT1b INT1c INT1d INT1

Step 1: $CuBr_2$ (146.5 g, 656 mmol) was added into EtOAc (250 mL) and the resulting mixture was stirred for 10 min at 80°C. Then, a compound INT1a (25.0 g, 164 mmol) was dissolved in chloroform (250 mL), the resulting solution was added to the above suspension, and the resulting mixture was refluxed at 80°C overnight. After the reaction was completed, the reaction mixture was concentrated under reduced pressure. The resulting residue was triturated with EtOAc (500 mL) for 0.5 h and filtered, and the filtrate was concentrated under reduced pressure to obtain a target compound INT1b (46.0 g, 148 mmol, light brown solid, yield: 90%), MS: $[M+H]^+$=309.0, 311.0, 313.0.

Step 2: the compound INT1b (45.0 g, 145 mmol) and $Li_2CO_3$ (26.8 g, 363 mmol) were added into DMF (450 mL) and the resulting mixture was stirred for 6 h at 100°C. After the reaction was completed, the reaction mixture was filtered, the filtrate was treated with a hydrochloric acid aqueous solution (900 mL, 0.5 N) and extracted with EtOAc (400 mL × 2), and an organic phase was washed with water (300 mL × 2), dried with anhydrous $Na_2SO_4$, filtered and concentrated to obtain a target compound **INT1c** (31.0 g, 135 mmol, light brown solid, yield: 93%), MS: $[M-H]^-$=227.0, 229.0.

Step 3: the compound **INT1c** (15.0 g, 65.5 mmol) and $K_2CO_3$ (18.1 g, 131 mmol) were added into MeCN (150 mL), followed by adding $Me_2SO_4$ (9.9 g, 78.6 mmol). Under nitrogen atmosphere, the resulting mixture was stirred overnight at 60°C. The reaction mixture was cooled to room temperature and filtered. Silica gel (30 g) was added to the filtrate, and then the resulting mixture was concentrated under reduced pressure, and purified by column chromatography (PE: EtOAc=10: 1) to obtain the target compound **INT1d** (13.7 g, 56.4 mmol, white solid, yield: 86%), $^1H$ NMR (400 MHz, $CDCl_3$) δ 7.51-7.46 (m, 2H), 7.45-7.42 (m, 2H) and 4.00 (s, 3H).

Step 4: the compound **INT1d** (13.7 g, 56.4 mmol), bis(pinacolato)diboron (17.2 g, 67.7 mmol), KOAc (11.1 g, 113 mmol), and $Pd(PPh_3)Cl_2$ (2.00 g, 2.82 mmol) were added into dioxane (137 mL), and the resulting mixture was reacted at 90°C for 12 h under nitrogen atmosphere. After the reaction was completed, the reaction mixture was filtered. Silica gel (30 g) was added to the filtrate, and then the resulting mixture was concentrated under reduced pressure, and purified by column chromatography (PE: EtOAc=30: 1) to obtain a target compound INT1 (7.6 g, 26.2 mmol, colorless solid, yield: 46%), $^1H$ NMR (400 MHz, $CDCl_3$) δ 7.67 (d, J=8.0 Hz, 1H), 7.61 (d, J=8.0 Hz, 1H), 7.49 (d, J=5.6 Hz, 1H), 7.35 (d, J=5.2 Hz, 1H), 4.00 (s, 3H), and 1.39 (s, 12H).

**Intermediate** INT2:

2-[2-(difluoromethoxy)-4-(trifluoromethyl)phenyl]-4,4,5-5-tetramethyl-1,3,2-dioxaborolane

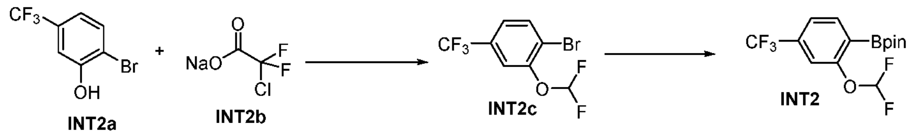

INT2a INT2b INT2c INT2

Step 1: the compound **INT2a** (5 g, 20.7 mmol), **INT2b** (7.1 g, 52 mmol), cesium carbonate (11.5 g, 41.5 mmol), water (20 mL) and DMF (80 mL) were added into a 250 mL three-necked flask sequentially, and were heated up to 120°C and stirred for 12 h. After cooled to room temperature, the reaction solution was extracted with ethyl acetate and layered. The organic phase was washed with water and saturated brine separately, dried with anhydrous sodium sulfate and filtered. The filtrate was concentrated and purified by column chromatography to obtain a compound **INT2c** (2.8 g, yellow solid).

Step 2: the compound **INT2c** (2.8 g, 9.6 mmol), bis(pinacolato)diboron (3.7 g, 14.4 mmol), potassium acetate (2.8 g, 29 mmol), Pd(dppf)$Cl_2$ (0.7 g, 0.96 mmol), and dioxane (30 mL) were added to a 100 mL three-necked flask under nitrogen atmosphere. The resulting mixture was reacted, heated up to 100°C and stirred for 16 h. After the reaction was completed, the reaction mixture was cooled to room temperature, and then filtered with celite. The filtrate was extracted with ethyl acetate. The organic phases were combined and then washed with water and saturated brine separately, dried with anhydrous sodium sulfate and filtered. The filtrate was concentrated and purified by column chromatography to obtain a compound **INT2** (1.7 g, white solid). $^1H$ NMR (400 MHz, Chloroform-d) δ 7.86 (d, J=7.7 Hz, 1H), 7.49 (d, J=7.8, 1H), 7.39 (s, 1H), 6.54 (t, J=72.5 Hz, 1H), 1.35 (s, 12H).

**Intermediate INT3:**

2-[1-(difluoromethylene)-4-methoxy-2,3-dihydro-1H-inden-5-yl]-4,4,5,5-tetramethyl-1,3,2-di oxaborolane

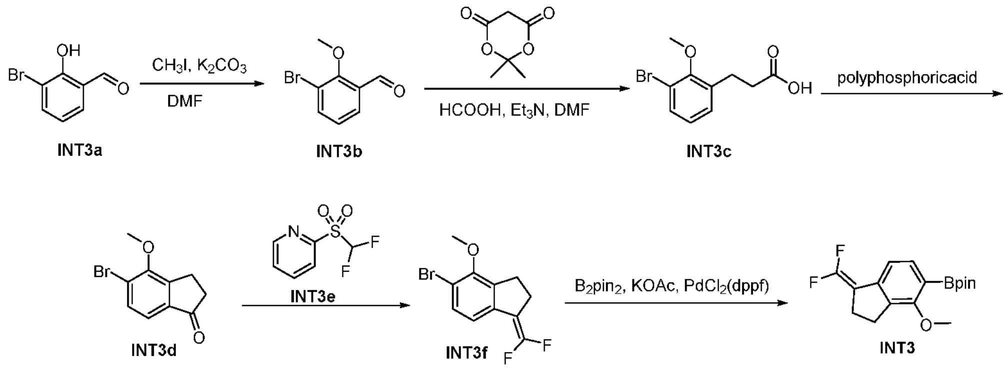


INT3a INT3b INT3c INT3d INT3e INT3f INT3

Step 1: the compound 3-bromo-2-hydroxybenzaldehyde (25.0 g, 104.65 mmol), $CH_3I$ (60.63 g, 373.14 mmol) and $K_2CO_3$ (34.38 g, 248.76 mmol) were dissolved in DMF (200 mL) and the resulting mixture was heated up to 50°C for 3 h. Upon the addition of water, the reaction mixture was extracted with ethyl acetate, the resulting organic phases were combined, dried with anhydrous sodium sulfate and filtered, the solvent was removed from the filtrate under reduced pressure to obtain a target compound **INT3a** (yellow oily liquid, 22.5 g, yield: 84%), and no further purification was required, MS/ESI$[M+H]^+$:215.1.

Step 2: $Et_3N$ (12.71 g, 125.58 mmol) was added into HCOOH (14.45 g, 313.95 mmol) dropwise at 0°C, the resulting mixture was reacted for 30 min at room temperature, and then a DMF (200 mL) solution in which the compound **INT3a** (22.5 g, 104.65 mmol) and 2,2-dimethyl-1,3-dioxane-4,6-dione (15.08 g, 104.65 mmol) were dissolved and added into the reaction mixture, and the resulting solution was heated up to 100°C and reacted overnight. NaOH aqueous solution was added to the reaction solution, and the resulting mixture was adjusted to pH=9, and extracted with ethyl acetate. HCl aqueous solution (3 M) was added to an aqueous phase, the resulting mixture was adjusted to pH=5, and extracted with ethyl acetate. After the organic phases were combined, the resulting mixture was dried with anhydrous sodium sulfate and filtered. The solvent was removed from the filtrate under reduced pressure, and the resulting residue was purified by column chromatography (PE:EA=5:1) to obtain a target compound **INT3b** (white solid, 20.3 g, yield: 75%), MS/ESI $[M+H]^+$:259.1.

Step 3: polyphosphoric acid (200 g) was heated up to 90°C, the compound **INT3b** (20.3 g, 78.34 mmol) was added into polyphosphoric acid. The resulting mixture was suspended for heating after one hour of reaction. The aqueous solution was added into the reaction mixture, and the resulting solution was cooled to room temperature and extracted with

dichloromethane. The organic phase was washed with saturated NaCl aqueous solution, dried with anhydrous sodium sulfate and filtered. The solvent was removed from the filtrate under reduced pressure, and the resulting residue was purified by column chromatography (PE: EA=5:1) to obtain a target compound **INT3c** (yellow solid, 9.7 g, yield: 51.3%), MS/ESI$[M+H]^+$:241.1.

Step 4: the compound **INT3c** (4.7 g, 19.50 mmol) and 2-[(difluoroMethyl)sulfonyl]pyridine (5.65 g, 29.25 mmol) were dissolved in DMF (30 mL), the DMF solution was cooled to -50°C, potassium tert-butoxide was added into the DMF solution under nitrogen atmosphere. The resulting mixture was heated up to -40°C and reacted for 3 h, and then a saturated ammonium chloride aqueous solution (26 mL) was added into the reaction mixture for quenching the reaction. 3N HCl (26 mL) was then added into the quenched solvent, the resulting mixture was heated to room temperature and extracted with ethyl acetate. The organic phases were combined, dried with anhydrous sodium sulfate and filtered, and the solvent was removed from the filtrate under reduced pressure, and the resulting residue was purified by column chromatography (PE: EA=20: 1) to obtain a target compound **INT3e** (yellow oily liquid, 1.92 g, yield: 35.8%). $^1H$ NMR (400 MHz, $CDCl_3$) δ 7.53 (d, J=7.7 Hz, 1H), 7.11 (d, J=7.7 Hz, 1H), 3.75 (s, 3H), 3.01-2.95 (m, 2H), 2.76-2.65 (m, 2H).

Step 5: under nitrogen atmosphere, the compound **INT3e** (1.92 g, 6.98 mmol), bis(pinacolato)diboron (2.66 g, 10.47 mmol), $PdCl_2$ (dppf) (0.57 g, 0.7 mmol) and potassium acetate (2.05 g, 20.94 mmol) were dissolved in a 1,4-dioxane solution. The resulting mixture was refluxed at 100°C overnight. After the reaction was completed, the reaction mixture was returned to room temperature, and extracted with water and ethyl acetate. The organic phases were combined, dried with anhydrous sodium sulfate and filtered. The solvent was removed from the filtrate under reduced pressure, and the resulting residue was purified by column chromatography (PE: EA=10: 1) to obtain a target compound **INT3** (yellow solid, 1.02 g, yield: 45.4%). $^1H$ NMR (400 MHz, $CDCl_3$) δ 7.60 (d, J=8.0 Hz, 1H), 7.12 (d, J=8.0 Hz, 1H), 3.81 (s, 3H), 2.97-2.82 (m, 2H), 2.62-2.51 (m, 2H), 1.30 (s, 12H).

**Intermediate** INT4:

2-(4-methoxybenzofuran-5-yl)-4,4,5,5-tetramethyl-1,3,2-dioxaborolane

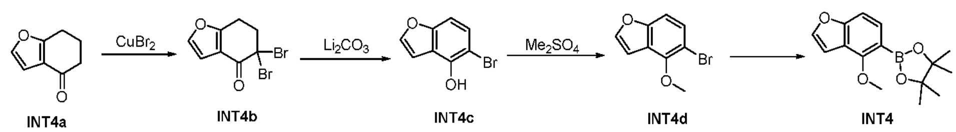


INT4a INT4b INT4c INT4d INT4

Step 1: $CuBr_2$ (66 g, 223 mmol) was added into EtOAc (120 mL) and the resulting mixture was stirred for 10 min at 80°C. Then, a compound INT4a (10 g, 136 mmol) was dissolved in chloroform (120 mL), the resulting solution was added to the above suspension, and the resulting mixture was refluxed at 80°C overnight. After the reaction was completed, the reaction mixture was concentrated under reduced pressure. The resulting residue was triturated with EtOAc (200 mL) for 0.5 h and filtered, and the filtrate was concentrated under reduced pressure to obtain a target compound **INT4b** (7.3 g). $^1H$ NMR (400 MHz, Chloroform-d) δ 7.39 (d, J=1.8 Hz, 1H), 6.76 (dt, J=4.7, 2.1 Hz, 1H), 3.12 (m, 2H), 3.05-2.98 (m, 2H).

Step 2: the compound **INT4b** (7.3 g, 25 mmol) and $Li_2CO_3$ (11 g, 150 mmol) were added into DMF (70 mL) and the resulting mixture was stirred for 6 h at 100°C. After the reaction was completed, the reaction mixture was filtered, the filtrate was adjusted to pH=1 using the hydrochloric acid aqueous solution and extracted with EtOAc (150 mL × 3), and an organic phase was washed with a saturated brine solution, dried with anhydrous $Na_2SO_4$, and filtered, and the filtrate was concentrated to obtain a target compound **INT4c** (5.5 g). $^1H$ NMR (400 MHz, Chloroform-*d*) δ 7.55 (d, *J*=2.2 Hz, 1H), 7.34 (d, *J*=8.7 Hz, 1H), 7.02 (dd, *J=8.7*, 0.9 Hz, 1H), 6.89 (dd, *J*=2.2, 1.0 Hz, 1H), 5.94 (s, 1H).

Step 3: the compound **INT4c** (5.5 g, 26 mmol), $K_2CO_3$ (7.17 g, 52 mmol), and $Me_2SO_4$ (4.1 g, 33 mmol) were added into MeCN (60 mL) sequentially, and the resulting mixture was stirred at 60°C overnight under a nitrogen atmosphere. The reaction mixture was cooled to room temperature and filtered. Silica gel was added to the filtrate, and then the resulting mixture was concentrated under reduced pressure, and purified by column chromatography (PE: EtOAc=10: 1) to obtain a target compound **INT4d** (5 g), $^1H$ NMR (400 MHz, Chloroform-*d*) δ 7.56 (d, *J*=2.3 Hz, 1H), 7.42 (d, *J*=8.7 Hz, 1H), 7.12 (dd, *J*=8.7, 1.0 Hz, 1H), 6.91 (dd, *J*=2.4, 1.0 Hz, 1H), 4.08 (s, 3H).

Step 4: the compound **INT4d** (1 g, 4.4 mmol), bis(pinacolato)diboron (2.2 g, 8.8 mmol), KOAc (1.7 g, 17.6 mmol), and Pd(PPh)$Cl_2$ (322 mg, 0.44 mmol) were added into dioxane (15 mL), and the resulting mixture was reacted for 12 h at 90°C under nitrogen atmosphere. After the reaction was completed, the reaction mixture was filtered. Silica gel was added to the filtrate, and then the resulting mixture was concentrated under reduced pressure, and purified by column chromatography (PE: EtOAc=10: 1) to obtain a target compound **INT4** (410 mg), $^1H$ NMR (400 MHz, Chloroform-*d*) δ 7.63 (d, *J*=8.3 Hz, 1H), 7.54 (d, *J*=2.2 Hz, 1H), 7.22 (dd, *J*=8.3, 1.0 Hz, 1H), 6.90 (dd, *J*=2.2, 1.0 Hz, 1H), 4.05 (s, 3H), 1.37 (s, 12H).

Intermediate **INT5**:

6-bromo-2-(4-methoxybenzyl)-4-methyl-1,2,4-triazine-3,5(2H,4H)-dione

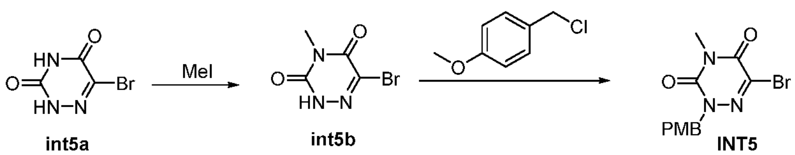


int5a

int5b

INT5

Step 1: the compound **INT5a** (50 g, 260 mmol) and methyl iodide (37 g, 260 mmol) were dissolved in ultra-dry DMF (250.0 mL), DIEA (36 g, 281 mmol) was added into the reaction flask, and the resulting mixture was reacted for 3 h at room temperature. After most of raw material was reacted, the reaction mixture was extracted four times with EA and the saturated brine solution, and the organic phase was concentrated, and then purified by column chromatography with the silica gel (PE/EA 8:1 to 6:1) to obtain a white solid compound **INT5b** (39 g).

Step 2: the compound **INT5b** (39g, 189.3 mmol) and 4-methoxychlorobenzyl (40 g, 265 mmol) were dissolved in ultra-dry DMF (500.0 mL), $K_2CO_3$ (52 g, 378.6 mmol) was added into the reaction flask, and the resulting mixture was reacted for 8 h at room temperature. After the reaction of raw materials was confirmed complete by TLC, the reaction mixture was extracted three times with 200 mL ethyl acetate and water, and the organic phase was washed twice with 200 mL saturated brine solution, dried with anhydrous sodium sulfate, and concentrated to obtain a compound **INT5.** $^1$H NMR (400 MHz, DMSO-d6) δ 7.36-7.20 (m, 2H), 7.00-6.82 (m, 2H), 5.01(s, 2H), 3.74(s, 3H), 3.20(s, 3H).

**Intermediate INT6:**

2-(4-cyclopropyl-2-methoxyphenyl)-4,4,5,5-tetramethyl-1,3,2-dioxaborolane

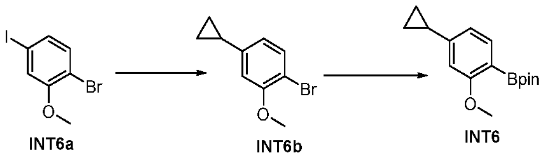

INT6a

INT6b

INT6

Step 1: the compound **INT6a** (500 mg, 1.6 mmol), cyclopropylboronic acid (505 mg, 5.87 mmol) and potassium carbonate (883 mg, 6.39 mmol) were mixed in dioxane (10 mL) and water (5 mL), followed by adding Pd(dppf)$Cl_2$ (65.9 mg,0.080 mmol) under nitrogen atmosphere, and then the resulting mixture was stirred at 120°C under nitrogen atmosphere and fully reacted. After cooled to room temperature, the reaction mixture was diluted with ethyl acetate (20 mL), and the organic phase was washed with brine (30 mL), dried with anhydrous sodium sulfate, and filtered. The filtrate was concentrated in vacuum to obtain a crude product compound **INT6b,** and the compound **INT6b** was directly used in a next step.

Step 2: the compound **INT6b** (1.2 g, 4.4 mmol), bis(pinacolato)diboron (2.2 g, 8.8 mmol), KOAc (1.7 g, 17.6 mmol), and Pd(dppf)$Cl_2$ (322 mg, 0.44 mmol) were added into dioxane (15 mL), and the resulting mixture was reacted for 12 h at 90°C under nitrogen atmosphere. After the reaction was completed, the reaction mixture was filtered. Silica gel was added to the filtrate, and then the resulting mixture was concentrated under reduced pressure, and purified by column chromatography (PE: EtOAc=10: 1) to obtain a target compound **INT6** (0.82g).

**Intermediate INT7:**

6-bromo-3-chloro-4-methyl-1,2,4-triazin-5(4H)-ketone

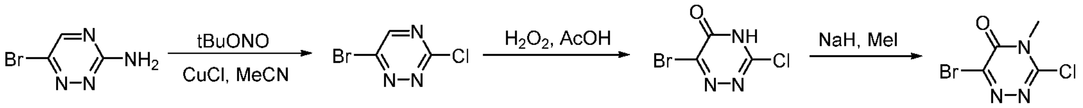


INT7a INT7b INT7c INT7

Step 1: the compound **INT7a** (10 g, 57.15 mmol) and cuprous chloride (11 g, 111.11 mmol) were placed in a sealed tube, tert-butyl nitrite (12 g, 116.50 mmol) and acetonitrile (35 mL) were also added into the sealed tube, and the sealed tube was quickly sealed. The resulting mixture was heated up to 80°C and reacted for 4 h. After the reaction was completed, the reaction mixture was concentrated. The resulting residue was purified by column chromatography, and 10% EA: PE elution product was concentrated to obtain a light yellow solid product **INT7b** (5.31 g). LC-MS: ESI $[M+H]^+$=193.0.

Step 2: the compound **INT7b** (5.31 g, 25.24 mmol) and acetic acid (25 mL) were added into the reaction flask, the reaction flask was placed in an ice bath, hydrogen peroxide (5 mL) was slowly dropped into the reaction flask, and the resulting mixture was slowly returned to room temperature and reacted overnight. After the reaction was completed, a sodium thiosulfate aqueous solution was added into the reaction mixture for quenching the reaction. Then, the reaction mixture was extracted with EA (ethyl acetate) and 5% TEA (triethylamine). The organic phase was concentrated under reduced pressure. The resulting residual was purified by column chromatography to obtain a light yellow solid product **INT7c** (5.12 g). LC-MS: ESI$[M+H]^+$=209.1.

Step 3: the compound **INT7c** (3 g, 14.26 mmol) and anhydrous DMF (30 mL) were placed in the three-necked bottle under nitrogen atmosphere, the resulting mixture was reacted and placed in the ice bath, NaH (1.15 g, 28.79 mmol) was slowly added into the reaction mixture, the resulting mixture was returned to room temperature and reacted for 1 h, methyl iodide (2.02 g, 14.26 mmol) was slowly dropped into the reaction mixture, and the resulting mixture was reacted overnight under room temperature. After the reaction was completed, water was added into the reaction mixture for quenching the reaction, the reaction mixture was extracted with EA, washed with the saturated brine solution, dried and concentrated, and the resulting residue was purified by column chromatography to obtain a white solid product **INT7** (0.8 g). LC-MS: ESI$[M+H]^+$=224.0. $^1$H NMR (400 MHz, DMSO-$d_6$) $\delta$ 3.45 (s, 3H).

**Intermediate INT8:**

2-(4-(methoxymethoxy)benzo[b]thiophen-5-yl)-4,4,5,5-tetramethyl-1,3,2-dioxaborolane

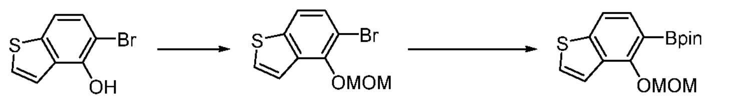

INT1c INT8a INT8

Step 1: the compound **INT1c** (220 g, 960 mmol) and diisopropylethylamine (186 g, 1440 mmol) were added into dichloromethane (1.1 L), followed by bromomethyl methyl ether (132 g, 1056 mmol) slowly added dropwise at 0°C, and the resulting mixture was stirred for 1 h at room temperature. After the reaction was completed, a saturated ammonium chloride aqueous solution (300 mL $\times$ 3) was added into the reaction mixture for washing, and the organic phase was dried with anhydrous sodium sulfate, then concentrated under reduced pressure, and purified by column chromatography (PE: EtOAc=30: 1) to obtain a target compound **INT8a** (183 g, 670 mmol, light yellow oily substance, yield: 70%), $^1$H NMR (400 MHz, $CDCl_3$) $\delta$ 7.55-7.45 (m, 3H), 7.43 (d, *J*=5.5 Hz, 1H), 5.27 (s, 2H), 3.70 (s, 3H).

Step 2: the compound **INT8a** (20.0 g, 73.2 mmol), bis(pinacolato)diboron (22.3 g, 87.8 mmol), KOAc (14.3 g, 146 mmol), and $Pd(PPh_3)Cl_2$ (2.57 g, 3.66 mmol) were added into anhydrous dioxane (200 mL), and the resulting mixture was reacted for 12 h at 100°C under nitrogen atmosphere. After the reaction was completed, the reaction mixture was filtered. The filtrate was concentrated under reduced pressure, and purified by column chromatography (PE: EtOAc=30: 1) to obtain a target compound **INT8** (14.5 g, 45.3 mmol, colorless solid, yield: 62%). $^1$H NMR (400 MHz, $CDCl_3$) $\delta$ 7.71 (d, *J*=8.1 Hz, 1H), 7.63 (d, *J*=8.1 Hz, 1H), 7.55 (d, *J*=5.5 Hz, 1H), 7.35 (d, *J*=5.6 Hz, 1H), 5.25 (s, 2H), 3.62 (s, 3H), 1.37 (s, 12H).

**Embodiment 1:**

(R)-3-(1-(difluoromethylene)-4-hydroxy-2,3-dihydro-1H-inden-5-yl)-6-((1-(2-hydroxyethyl)p iperidin-3-yl)amino)-4-methyl-1,2,4-triazin-5(4H)-ketone

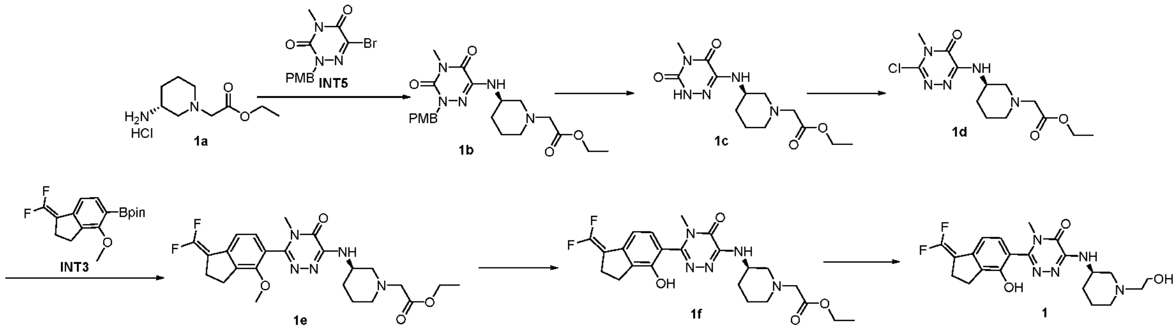

Step 1: the compound **INT5** (8.00 g, 24.54 mmol), **1a** (12.71 g, 49.08 mmol), $Pd(OAc)_2$ (0.56 g, 2.45 mmol), BINAP (1.53 g, 2.45 mmol), cesium carbonate (23.93 g, 73.62 mmol), and dioxane (180 mL) were added into the reaction flask, and then stirred for 15 h at 100°C under nitrogen atmosphere. After the reaction was completed, the reaction mixture was cooled to room temperature and directly stirred with silica gel, and the resulting mixture was purified by silica gel column chromatography (petroleum ether: ethyl acetate=1: 0-2: 1) to obtain a compound **1b** (7.00 g, 66.7%), MS/ESI $[M+H]^+$=432.0.

Step 2: the compound **1b** (7.00 g, 16.24 mmol) and dichloromethane (60 mL) were added in the reaction flask and cooled to 0°C, followed by slowing dropping trifluoromethanesulfonic acid (6 mL) into the reaction flask, and the resulting mixture was stirred for 15 h at room temperature. After the reaction was completed, the reaction mixture was cooled to 0°C-5°C with acetonitrile (60 mL), solid sodium bicarbonate was added into the cooled mixture until no large amounts of bubbles were generated, then the pH of the cooled mixture was adjusted to be 7-8 with ammonia water, the pH-adjusted mixture was dried with anhydrous sodium sulfate, filtered and concentrated, and the obtained crude product was purified by silica gel column chromatography (petroleum ether: ethyl acetate=1: 0-1: 2) to obtain a compound **1c** (4.05 g, 80.5%), MS/ESI$[M+H]^+$=312.0.

Step 3: the compound **1c** (4.00 g, 12.86 mmol) was dispersed in phosphorus oxychloride (25 mL) and the resulting mixture was stirred for 12 h at 110°C under nitrogen atmosphere. After the reaction was completed, the solvent was directly concentrated, the resulting residue was dispersed in acetonitrile (60 mL) and dichloromethane (100 mL), the resulting mixture was cooled to 0°C-5°C, solid sodium bicarbonate was added into the cooled mixture until no large amounts of bubbles were generated, then the pH of the cooled mixture was adjusted to be 7-8 with the ammonia water, and the pH-adjusted mixture was dried with anhydrous sodium sulfate, filtered and concentrated, and the obtained crude product was purified by silica gel column chromatography (dichloromethane: methanol=1: 0-10: 1) to obtain a compound **1d** (3.00 g, 71.4%), MS/ESI$[M+H]^+$=330.0.

Step 4: the compound **1d** (2.12g, 6.43 mmol), **INT3** (1.80 g, 5.59 mmol), $Pd(dppf)Cl_2$ (409 mg, 0.06 mmol), cesium carbonate (5.45g, 16.77 mmol), dioxane (60 mL) and water (6 mL) were added into the reaction flask and stirred for 15 h at 100°C under nitrogen atmosphere. After the reaction was completed, the reaction mixture was cooled to room temperature, and directly mixed with the silica gel, and the resulting mixture was purified by silica gel column chromatography (petroleum ether: ethyl acetate=1: 0-0: 1) to obtain a compound **1e** (1.10 g, 40.3%), MS/ESI$[M+H]^+$=490.1.

Step 5: the compound **1e** (1.10 g, 2.25 mmol) and dichloromethane (50 mL) were added into the reaction flask, and cooled to -10°C under nitrogen atmosphere, followed by adding 1 M dichloromethane solution of boron tribromide (6.71 mL, 6.71 mmol), and the resulting mixture was reacted for 2 h at a constant temperature. After the reaction was completed, methanol (10 mL) was added into the reaction mixture for quenching the reaction, and the solvent was concentrated directly. The resulting residue was purified by silica gel column chromatography (dichloromethane: methanol=1: 0-9: 1) to obtain a compound **1f** (498 mg, 46.6%), MS/ESI$[M+H]^+$=476.0.

Step 6: the compound **1f** (479 mg, 1.01 mmol) and ethanol (25 mL) were added in the reaction flask. Under nitrogen atmosphere, lithium borohydride solid (177 mg, 8.07 mmol) was added into the reaction mixture in batches, and the resulting mixture was reacted at 55°C for 12 h. After the reaction was completed, saturated ammonium chloride (20 mL) was added into the reaction mixture for quenching the reaction, followed by adding 50 mL ethyl acetate. The aqueous phase was further extracted three times with ethyl acetate (30), and the organic phases were combined, dried with anhydrous sodium sulfate, filtered and concentrated. The resulting residue was purified by Pre-HPLC (FA system) to obtain a compound **1** (14 mg, 3.2%). $^1$H NMR (400 MHz, Methanol-$d_4$) $\delta$ 8.39 (br s, 2H), 7.13 (d, *J*=8.0 Hz, 1H), 6.98 (dd, *J*=8.0, 1.6 Hz, 1H), 4.26-4.14 (m, 1H), 3.72 (t, *J*=5.2 Hz, 2H), 3.45-3.35 (m, 1H), 3.23 (s, 3H), 3.17-3.07 (m, 1H), 2.98-2.87

(m, 4H), 2.82-2.64 (m, 4H), 2.01-1.86 (m, 2H), 1.81-1.58 (m, 2H). MS/ESI[M+H]$^+$=434.0.

**Embodiment 2:**

(*R*)-3-(1-(difluoromethylene)-4-hydroxy-2,3-dihydro-1*H*-inden-5-yl)-6-((1-ethylpiperidin-3-y 1)amino)-4-methyl-1,2,4-triazin-5(4*H*)-ketone

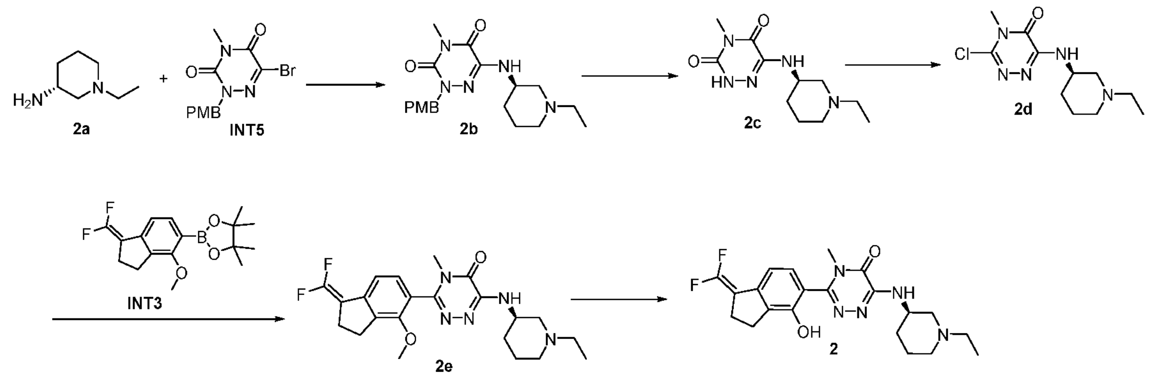


2a INT5 2b 2c 2d 2e 2

Step 1: the compound **INT5** (1.50 g, 4.60 mmol), **2a** (1.13 g, 6.90 mmol), BINAP Pd G3 (0.46 g, 0.46 mmol), cesium carbonate (4.48 g, 13.8 mmol), and dioxane (30 mL) were added into the reaction flask, and then stirred for 15 h at 100°C under nitrogen atmosphere. After the reaction was completed, the reaction mixture was cooled to room temperature, and directly mixed with the silica gel, and the resulting mixture was purified by silica gel column chromatography (dichloromethane: methyl alcohol=1: 0- 35: 1) to obtain a compound **2b** (1.41 g, 82.4%), MS/ESI[M+H]$^+$=374.0.

Step 2: the compound **2b** (1.41 g, 3.79 mmol) and dichloromethane (10 mL) were added in the reaction flask and cooled to 0°C, followed by slowing dropping trifluoromethanesulfonic acid (3 mL) into the reaction flask, and the resulting mixture was stirred for 15 h at room temperature. After the reaction was completed, the reaction mixture was cooled to 0°C-5°C with acetonitrile (10 mL), solid sodium bicarbonate was added into the cooled mixture until no large amounts of bubbles were generated, then the pH of the cooled mixture was adjusted to be 7-8 with ammonia water, the pH-adjusted mixture was dried with anhydrous sodium sulfate, filtered and concentrated, and the obtained crude product was purified by silica gel column chromatography (dichloromethane: methyl alcohol=1: 0-4: 1) to obtain a compound **2c** (850 mg, 89.5%), MS/ESI[M+H]$^+$=254.0.

Step 3: the compound **2c** (850 mg, 3.36 mmol) was dispersed in phosphorus oxychloride (10 mL) and the resulting mixture was stirred for 12 h at 110°C under nitrogen atmosphere. After the reaction was completed, the solvent was directly concentrated, the resulting residue was dispersed in acetonitrile (10 mL) and dichloromethane (10 mL), the resulting mixture was cooled to 0°C-5°C, solid sodium bicarbonate was added into the cooled mixture until no large amounts of bubbles were generated, then the pH of the cooled mixture was adjusted to be 7-8 with the ammonia water, and the pH-adjusted mixture was dried with anhydrous sodium sulfate, filtered and concentrated and the obtained crude product was purified by silica gel column chromatography (dichloromethane: methanol=1: 0-10: 1) to obtain a compound **2d** (605 mg, 65.9%), MS/ESI[M+H]$^+$=272.0.

Step 4: the compound **2d** (200mg, 0.74 mmol), **INT3** (261 mg, 0.81 mmol), Pd(dppf)$Cl_2$ (54 mg, 0.07 mmol), cesium carbonate (720mg, 2.21 mmol), dioxane (12 mL) and water (2 mL) were added into the reaction flask and stirred for 15 h at 100°C under nitrogen atmosphere. After the reaction was completed, the reaction mixture was cooled to room temperature, and directly mixed with the silica gel, and the resulting mixture was purified by silica gel column chromatography (dichloromethane: methanol=1: 0- 10: 1) to obtain a compound **2e** (99 mg, 31.1%), MS/ESI[M+H]$^+$=432.1.

Step 5: the compound **2e** (99 mg, 0.23 mmol) and dichloromethane (6 mL) were added into the reaction flask, and cooled to -10°C under nitrogen atmosphere, followed by adding 1M dichloromethane solution of boron tribromide(0.69 mL, 0.69 mmol), and the resulting mixture was reacted for 2 h at a constant temperature. After the reaction was completed, methanol (4 mL) was added into the reaction mixture for quenching the reaction, and the solvent was concentrated directly. The resulting residue was purified by silica gel column chromatography (dichloromethane: methanol=1: 0-9: 1) to obtain a compound **2** (70 mg, 73.2%). $^1$H NMR (400 MHz, DMSO-$d_6$) $\delta$ 9.78 (br s, 1H), 9.58 (br s, 1H), 7.24 (d, *J*=8.0 Hz, 1H), 7.07-6.97 (m, 1H), 4.36-4.23 (m, 1H), 3.57-3.48 (m, 3H), 3.26-3.18 (m, 5H), 3.08-2.98 (m, 2H), 2.87-2.80 (m, 3H), 2.05-1.93 (m, 2H), 1.88-1.65 (m, 2H), 1.28-1.20 (m, 3H), MS/ESI[M+H]$^+$[M+H]$^+$=418.0.

**Embodiment 3:**

(*R*)-6-((1-ethylpiperidin-3-yl)amino)-3-(4-hydroxybenzo[b]thiophen-5-yl)-4-methyl-1,2,4-tria zin-5(4*H*)-ketone

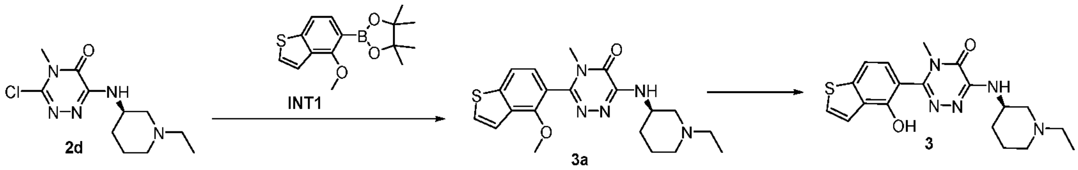


(*R*)-6-((1-ethylpiperidin-3-yl)amino)-3-(4-hydroxybenzo[b]thiophen-5-yl)-4-methyl-1,2, 4-triazin-5(4*H*)-ketone was prepared by referring to Embodiment 2, to obtain a compound (*R*)-6-((1-ethylpiperidin-3-yl)amino)-3-(4-hydroxybenzo[b]thiophen-5-yl)-4-methyl-1,2,4-tria zin-5(4*H*)-ketone (70 mg, 80.6%), $^1$H NMR (400 MHz, DMSO-$d_6$) δ 10.46 (br s, 1H), 9.60 (br s, 1*H*), 7.80-7.73 (m, 2H), 7.65 (d, *J*=8.0 Hz, 1H), 7.30 (d, *J*=8.0 Hz, 1H), 4.37-4.27 (m, 1H), 3.58-3.48 (m, 3H), 3.29-3.18 (m, 5H), 2.92-2.82 (m, 1H), 2.05-1.96 (m, 2H), 1.88-1.68 (m, 2H), 1.28-1.24 (m, 3H), MS/ESI $[M+H]^+[M+H]^+$=386.0.

**Embodiment 4:**

(*R*)-3-(4-hydroxybenzo[b]thiophen-5-yl)-4-methyl-6-((1-methylpiperidin-3-yl)amino)-1,2,4-tr iazin-5(4H)-ketone

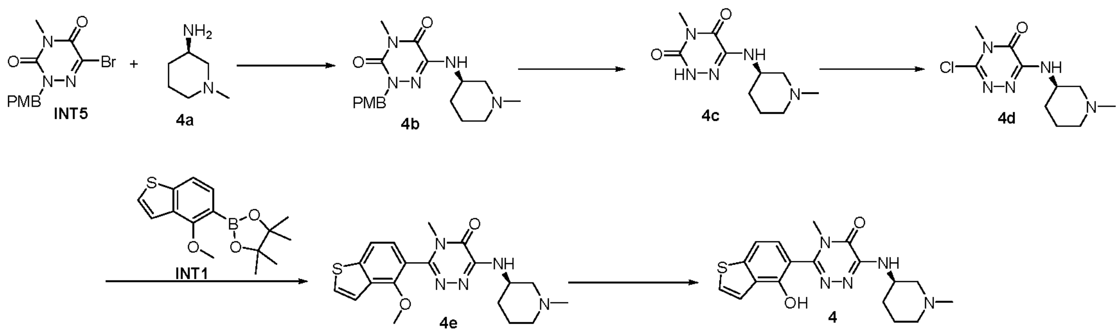


Step 1: the compound **INT5** (3.00 g, 9.20 mmol), **4a** (1.15 g, 10.1 mmol), $Cs_2CO_3$ (6.01 g, 18.4 mmol), BINAP (573 mg, 0.92 mmol) and $Pd(OA)_2$(208 mg, 0.92 mmol) were weighed and added to dioxane (60 mL), and the resulting mixture was replaced three times with $N_2$, and then heated up to 110°C, and stirred overnight. After the reaction was completed, the reaction mixture was mixed with silica gel and concentrated under reduced pressure, and purified by column chromatography (DCM: MeOH=10: 1) to obtain a target compound **4b** (3.30 g, 9.18 mmol, light yellow viscous substance, yield: 99%), MS/ESI$[M+H]^+$=360.2.

Step 2: the compound **4b** (3.30 g, 9.18 mmol) was dissolved in dichloromethane (30 mL), followed by slowly adding TfOH (4.13 g, 27.5 mmol) at room temperature, and the resulting mixture was stirred overnight. After the reaction was completed, the pH of the reaction mixture was adjusted to about 8 with ammonia water, the pH-adjusted reaction mixture was dried with anhydrous $Na_2SO_4$ and filtered, the filtrate was mixed with silica gel, and the resulting mixture was concentrated under reduced pressure and purified by column chromatography (DCM: MeOH=5: 1) to obtain a target compound **4c** (770 mg, 3.22 mmol, light yellow solid, yield: 35%), MS/ESI$[M+H]^+$=240.1.

Step 3: $POCl_3$ (4 mL) was added to the compound **4c** (770 mg, 3.22 mmol), and the resulting mixture was heated to 110°C, and stirred overnight. After the reaction was completed, an excessive amount of $POCl_3$ was concentrated under pressure to obtain the target compound **4d** (1.5 g, 3.30 mmol, light brown solid, yield: 100%), MS/ESI$[M+H]^+$=258.2.

Step 4: the compound **4d** (1.1 g, 2.42 mmol), **INT1** (772 mg, 2.66 mmol), $Cs_2CO_3$ (3.94 g, 12.1 mmol), and $Pd(dppf)Cl_2$ (176 mg, 0.24 mmol) were weighed and added to dioxane (20 mL) and water (4 mL), and the resulting mixture was replaced three times with $N_2$, heated up to 100°C, and stirred overnight. After the reaction was completed, the reaction mixture was mixed with silica gel and concentrated under reduced pressure, and purified by column chromatography (DCM: MeOH=10: 1) to obtain a target compound **4e** (550 mg, 1.43 mmol, light yellow viscous substance, yield: 59%), MS/ESI$[M+H]^+$=386.2.

Step 5: the compound **4e** (550 mg, 1.43 mmol) was dissolved in DCM (5.5 mL) and cooled to 0°C, followed by adding $BBr_3$ (4.29 mL, 4.29 mmol, 1.0 M) dropwise, and the resulting mixture was stirred for 1 h at room temperature. After

the reaction was completed, a proper amount of methanol was added into the reaction mixture for quenching the reaction, the quenched mixture was concentrated under reduced pressure to obtain the crude product, the crude product was dissolved in methanol (5 mL), and the resulting solvent was purified to obtain the target compound **4** (295 mg, 0.79 mmol, light yellow powder, yield: 55%). $^1$H NMR (400 MHz, MeOD) δ 8.46 (s, 1H), 7.63 (d, *J*=5.6 Hz, 1H), 7.58 (dd, *J*=6.9, 4.3 Hz, 2H), 7.29 (d, *J*=8.3 Hz, 1H), 4.30 (dt, *J*=13.0, 4.7 Hz, 1H), 3.52 (d, *J*=11.5 Hz, 1H), 3.36 (s, 3H), 3.25-3.15 (m, 1H), 3.02-2.80 (m, 2H), 2.75 (s, 3H), 2.07 (dd, *J*=21.0, 10.1 Hz, 2H), 1.94-1.69 (m, 2H). MS/ESI[M+H]$^+$=372.2.

**Embodiment 5:**

(*R*)-6-((1-(3,3-difluorocyclobutyl)piperidin-3-yl)amino)-3-(4-hydroxybenzo[b]thiophen-5-yl)-4-methyl-1,2,4-triazin-5(4H)-ketone

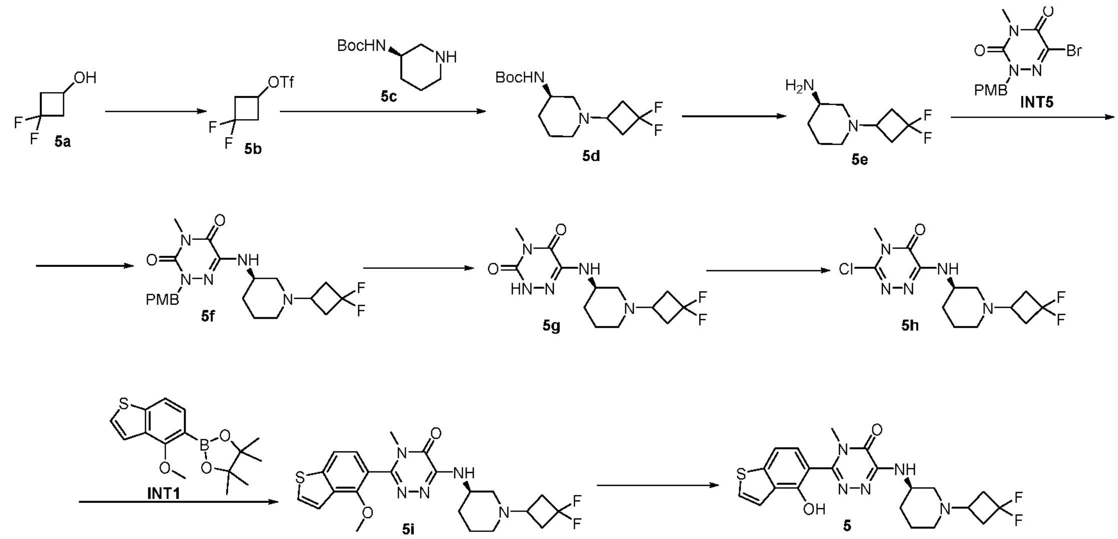


Step 1: the compound 5a (25.0 g, 231 mmol), pyridine (21.6 g, 277 mmol) and dichloromethane (125 mL) were weighed into a flask and cooled to 0°C, followed by slowly dropping $Tf_2O$ (71.7 g, 254 mmol), and the resulting mixture was stirred at room temperature and reacted for 1 h. The reaction mixture was filtered, and a filter cake was washed with dichloromethane (20 mL), the filtrate was collected to obtain the dichloromethane solution of the target compound **5b** (light yellow).

Step 2: the compound **5c** (50.9 g, 254 mmol) and triethylamine (70.1 g, 693 mmol) were dissolved in dichloromethane (250 mL), the dichloromethane solution of the above compound **5b** was added into the solvent dropwise at room temperature, and the resulting mixture was stirred overnight at room. After the reaction was completed, the reaction mixture was mixed with silica gel and concentrated under reduced pressure, and purified by column chromatography (PE: EtOAc=3: 1, iodine color development) to obtain a target compound **5d** (20.0 g, 68.9 mmol, light yellow solid, two-step yield: 30%), MS/ESI[M+H]$^+$=291.1.

Step 3: the compound **5d** (20.0 g, 68.9 mmol) was dissolved in ethyl acetate (100 mL), followed by adding HCl/EtOAc (86 mL, 345 mmol, 4.0 M), and the resulting mixture was stirred for 1 h at room temperature. After the reaction was completed, the reaction mixture was concentrated under reduced pressure to obtain a target compound **5e** (15.6 g, 59.3 mmol, white solid, yield: 86%), MS/ESI[M+H]$^+$=191.2.

(*R*)-6-((1-(3,3-difluorocyclobutyl)piperidin-3-yl)amino)-3-(4-hydroxybenzo[b]thiophen-5-yl)-4-methyl-1,2,4-triazin-5(4H)-ketone was prepared by referring to Embodiment 2. $^1$H NMR (400 MHz, $CDCl_3$) δ 7.62 (d, *J*=5.5 Hz, 1H), 7.47-7.31 (m, 2H), 7.21 (d, *J*=8.5 Hz, 1H), 6.31 (s, 1H), 4.24 (s, 1H), 3.60 (s, 3H), 2.67 (dd, *J*=11.5, 7.9 Hz, 3H), 2.52-2.33 (m, 4H), 2.23 (dd, J=16.5, 9.0 Hz, 1H), 1.83-1.70 (m, 4H), 1.66-1.61 (m, 1H). MS/ESI[M+H]$^+$=448.3.

**Embodiment 6:**

(*R*)-6-((1-(ethyl-*d*5)piperidin-3-yl)amino)-3-(4-hydroxybenzo[b]thiophen-5-yl)-4-methyl-1,2, 4-triazin-5(4H)-ketone

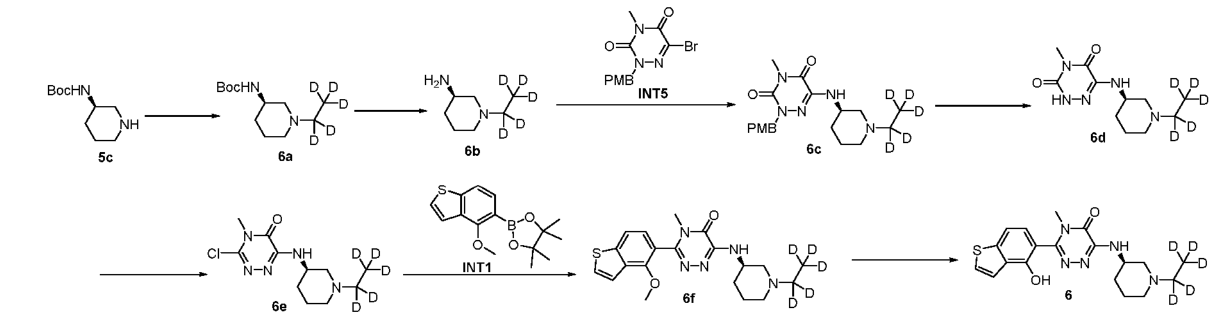


Step 1: the compound **5c** (10.0 g, 49.9 mmol) and $K_2CO_3$ (13.8 g, 99.8 mmol) were added into MeCN (100 mL), followed by adding deuterated bromoethane (6.83 g, 59.9 mmol), and the resulting mixture was stirred overnight at the room temperature. After the reaction was completed, the reaction mixture was filtered, the filtrate was concentrated under reduced pressure, the filter cake was dissolved in EtOAc (100 mL) and washed with water (30 mL $\times$ 3), the organic phase was collected and dried with anhydrous $Na_2SO_4$, filtered and concentrated under reduced pressure to obtain a target compound **6a** (10.1 g, 43.3 mmol, light brown solid, yield: 87%). $^1H$ NMR (400 MHz, $CDCl_3$) $\delta$ 4.99 (s, 1H), 3.73 (s, 1H), 2.54-2.21 (m, 4H), 1.74-1.52 (m, 4H), 1.45 (s, 9H).

Step 2: the compound **6a** (10.1 g, 43.3 mmol) was dissolved in dioxane (50 mL), followed by slowly adding HCl/dioxane (65 mL, 4.0 M), and the resulting mixture was stirred for 1 h at room temperature. The reaction mixture was concentrated under reduced pressure to obtain a target compound **6b** (9.20 g, 44.6 mmol, white solid, yield: 100%).

(*R*)-6-((1-(ethyl-*d*5)piperidin-3-yl)amino)-3-(4-hydroxybenzo[b]thiophen-5-yl)-4-methyl -1,2,4-triazin-5(4H)-ketone was prepared by referring to Embodiment 2. $^1H$ NMR (400 MHz, MeOD) $\delta$ 8.51 (s, 1H), 7.64 (d, *J*=5.6 Hz, 1H), 7.58 (dd, *J*=6.9, 4.5 Hz, 2H), 7.30 (d, *J*=8.2 Hz, 1H), 4.35 (t, *J*=8.8 Hz, 1H), 3.65 (d, J=9.4 Hz, 1H), 3.36 (s, 3H), 3.30 (d, *J*=4.5 Hz, 1H), 3.09-2.80 (m, 2H), 2.09 (dd, *J*=26.7, 9.0 Hz, 2H), 1.96-1.71 (m, 2H). MS/ESI[M+H]$^+$=391.5.

**Embodiment 7:**

(*R*)-6-((1-(ethyl-*d*5)piperidin-3-yl)amino)-3-(1-hydroxynaphthalen-2-yl)-4-methyl-1,2,4-triazi n-5(4H)-ketone

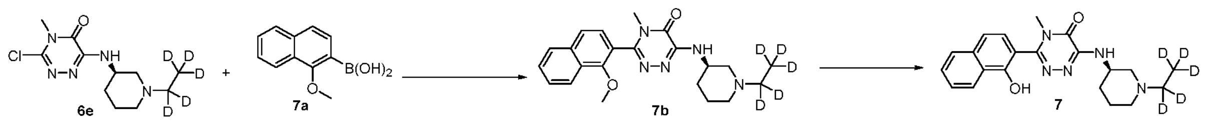


(*R*)-6-((1-(ethyl-*d*5)piperidin-3-yl)amino)-3-(1-hydroxynaphthalen-2-yl)-4-methyl-1,2,4-triazin-5(4*H*)-ketone (60 mg, 19.5%) was prepared by referring to Embodiment 2. $^1H$ NMR (400 MHz, DMSO-$d_6$) $\delta$ 10.14 (br s, 1H), 8.29 (d, *J*=8.0 Hz, 1H), 7.92 (d, *J*=7.6 Hz, 1H), 7.68-7.45 (m, 3H), 7.38 (d, *J*=8.4 Hz, 1H), 6.83 (br s, 1H), 4.16-4.01 (m, 1H), 3.19 (s, 3H), 2.95-2.74 (m, 1H), 2.65-2.54 (m, 1H), 2.29-2.09 (m, 2H), 1.84-1.45 (m, 4H). MS/ESI[M+H]$^+$=385.0.

**Embodiment 8:**

(*R*)-6-((1-(cyclopropylmethyl)piperidin-3-yl)amino)-3-(4-hydroxybenzo[*b*]thiophen-5-yl)-4-methyl-1,2,4-triazin-5(4H)-ketone

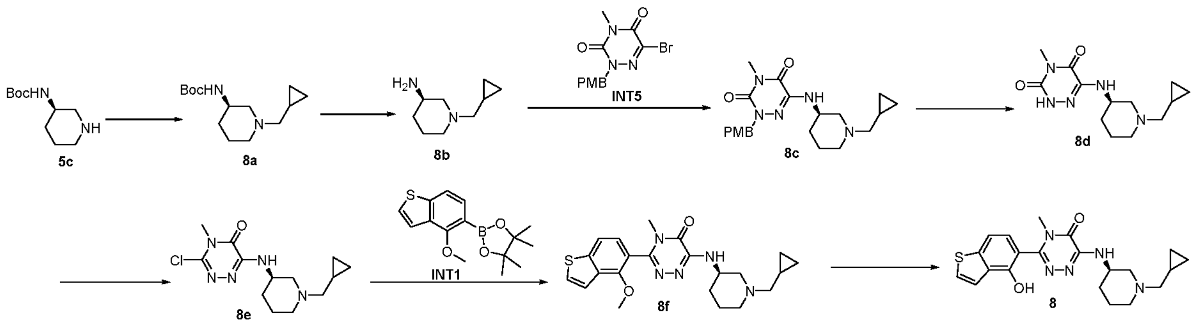


Step 1: the compound 5c (1.0 g, 5 mmol) and bromomethyl cyclopropane (675 mg, 5 mmol) were dissolved in 10 mL anhydrous acetonitrile, followed by adding potassium carbonate (759 mg, 5.5 mmol), and the reaction solution was stirred overnight at room temperature. The generation of target products was confirmed by an LC-MS. The reaction solution was filtered, the filtrate was concentrated under reduced pressure. The resulting residue was diluted with ethyl acetate, the organic phase was washed three times with water and saturated brine solution, dried with anhydrous sodium sulfate, and filtered, and the filtrate was concentrated under reduced pressure to obtain a crude product. The crude product was purified by methanol/dichloromethane to obtain a target product 8a (300 mg, yield: 24%). MS/ESI[M+H]$^+$=255.6.

Step 2: the compound **8a** (300 mg, 1.18 mmol) was completely dissolved in anhydrous DCM (5 mL), followed by adding hydrochloric acid dioxane solution (4 M, 5 mL). The resulting mixture was reacted for 2 h at room temperature, and the complete reaction of the raw materials was confirmed by the LC-MS. The reaction solution was concentrated under reduced pressure to obtain a compound **8b** (267 mg, yield: 100%), MS/ESI[M+H]$^+$=155.1.

(*R*)-6-((1-(cyclopropylmethyl)piperidin-3-yl)amino)-3-(4-hydroxybenzo[*b*]thiophen-5-yl )-4-methyl-1,2,4-triazin-5(4H)-ketone (128 mg, 39.1%) was synthesized by referring to Embodiment 2. $^1$H NMR (400 MHz, DMSO-$d_6$) δ 10.41 (br s, 1H), 9.54 (br s, 1H), 7.79-7.70 (m, 2H), 7.63 (d, *J*=8.0 Hz, 1H), 7.28 (d, *J*=8.4 Hz, 1H), 4.41-4.29 (m, 1H), 3.72-3.54 (m, 2H), 3.23 (s, 3H), 3.15-3.02 (m, 2H), 2.97-2.80 (m, 2H), 2.06-1.94 (m, 2H), 1.86-1.68 (m, 2H), 1.14-1.01 (m, 1H), 0.72-0.58 (m,2H), 0.46-0.33 (m, 2H). MS/ESI[M+H]$^+$=412.0.

**Embodiment 9:**

(*R*)-3-(4-hydroxybenzo[b]thiophen-5-yl)-6-((1-(2-hydroxyethyl)piperidin-3-yl)amino)-4-meth yl-1,2,4-triazin-5(4H)-ketone

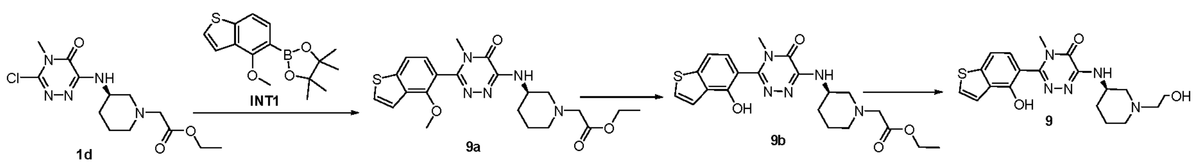


(*R*)-3-(4-hydroxybenzo[b]thiophen-5-yl)-6-((1-(2-hydroxyethyl)piperidin-3-yl)amino)-4-methyl-1,2,4-triazin-5(4H)-ketone was prepared by referring to Embodiment 1. MS/ESI[M+H]$^+$=402.2. $^1$H NMR (400 MHz, Methanol-$d_4$) δ 7.52 (d, *J*=5.6 Hz, 1H), 7.47-7.40 (m, 2H), 7.18 (d, *J*=8.0 Hz, 1H), 4.12-4.02 (m, 1H), 3.64-3.57 (m, 2H), 3.24 (s, 3H), 3.01-2.91 (m, 1H), 2.66-2.57 (m, 1H), 2.55-2.47 (m, 2H), 2.44-2.26 (m, 2H), 1.87-1.67 (m, 2H), 1.65-1.47 (m, 2H).

**Embodiment 10:**

(*R*)-3-(4-hydroxybenzo[b]thiophen-5-yl)-4-methyl-6-((1-(methyl-d3)piperidin-3-yl)amino)-1, 2,4-triazin-5(4H)-ketone

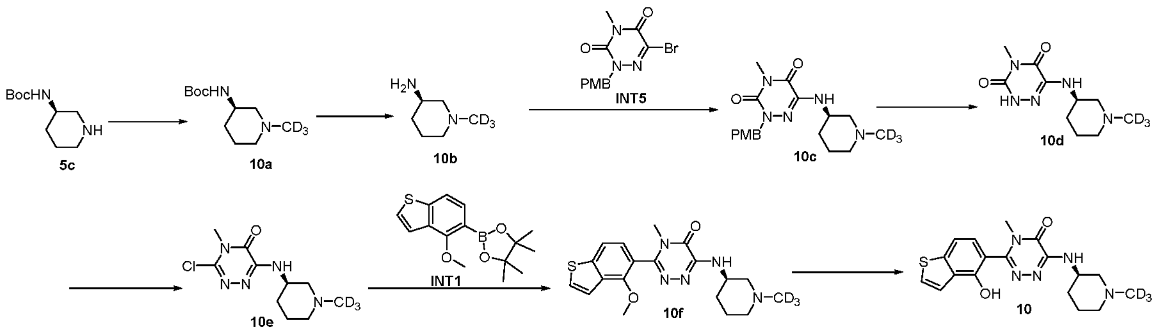


Step 1: the compound **5c** (2 g, 10 mmol), $CD_3I$ (1.5 g, 10 mmol), $K_2CO_3$ (2.76 g, 20 mmol) and acetonitrile (30 mL) were added into a drying flask as solvents, and reacted overnight at room temperature, upon the addition of water, the reaction mixture was extracted three times with ethyl acetate, the organic phases were combined, dried with anhydrous sodium sulfate, and filtered, and the filtrate was concentrated under reduced pressure. The resulting residue was purified by column chromatography to obtain a compound 10a (white solid, 2.03 g). MS/ESI$[M+H]^+$=218.2.

Step 2: the compound **10a** (2.03 g, 9.35 mmol), 1,4-dioxane (10 mL), and HCl (4 M, in dioxane) (9.35 mL) were added into the drying flask, and reacted for 3 h at room temperature. The reaction mixture was concentrated under reduced pressure to remove the excess solvent to obtain a compound **10b** (white solid, 1.32 g), and the compound **10b** can be directly used in next reaction.

Step 3: the compound **10b** (1.32 g, 7 mmol), **INT5** (2.28 g, 7 mmol), $Pd(OAc)_2$ (157.2 mg, 0.7 mmol), BINAP (435.9 mg, 0.7 mmol), and $Cs_2CO_3$ (6.6 g, 35 mmol) were added into the drying flask, under nitrogen atmosphere, the resulting mixture was reacted overnight at 100°C with dioxane (35 mL) as a solvent, after the completion of the reaction was confirmed by TLC, the reaction mixture was filtered and concentrated under reduced pressure, the resulting residue was purified by column chromatography to obtain a compound **10c** (brown oily liquid, 0.95 g). MS/ESI$[M+H]^+$=363.2.

Step 4: the compound **10c** (0.95 g, 2.6 mmol), DCM (10 mL) and TfOH (1.17 g, 7.8 mmol) were added into the drying flask, and reacted overnight at room temperature, after the completion of the reaction was confirmed by TLC, the pH of the reaction mixture was adjusted to pH=8-9 with dry $NaHCO_3$ and a small amount of ammonia water, and the reaction mixture was dried with anhydrous $Na_2SO_4$ and filtered, the filtrate was concentrated under reduced pressure, and the resulting residue was purified by column chromatography to obtain a compound **10d** (brown oily liquid, 600 mg). MS/ESI $[M+H]^+$=243.2.

Step 5: the compound **10d** (600 mg, 2.5 mmol) and $POCl_3$ (6 mL) were added into the drying flask, and the resulting mixture was reacted overnight at 100°C, after the reaction was completed, the reaction mixture was concentrated under reduced pressure to remove excess $POCl_3$, the PH of the reaction mixture was adjusted to 8-9 with dry $NaHCO_3$ and a small amount of ammonia water, and the reaction mixture was dried with anhydrous $Na_2SO_4$ and filtered,,the filtrate was concentrated under reduced pressure, and the resulting residue was purified by column chromatography to obtain a compound **10e** (light brown solid, 487 mg). MS/ESI$[M+H]^+$=261.1.

Step 6: the compound **10e** (200 mg, 0.77 mmol), **INT1** (268 mg, 0.93 mmol), $Pd(dppf)Cl_2$ (58.6 mg, 0.08 mmol), $Cs_2CO_3$ (1.17 g, 3.6 mmol) and 1,4-dioxane/$H_2O$ (6 mL/1 mL) were added into the drying flask, and reacted overnight at 100°C. After the completion of the reaction was confirmed by TLC, the reaction solution was returned to room temperature and filtered, and the filtrate was concentrated under reduced pressure. The resulting residue was purified by column chromatography to obtain a compound **10f** (yellow oily liquid, 108 mg). MS/ESI$[M+H]^+$=389.2.

Step 7: under nitrogen atmosphere, the compound **10f** (108 mg, 0.28 mmol) and DCM (2 mL) were added into the drying flask and cooled at -10°C, followed by adding $BBr_3$ (1 M, 1 mL) dropwise, and the resulting mixture was reacted for 3 h. After MeOH was added into the reaction solution for quenching the reaction, the reaction solution was returned to room temperature and concentrated under reduced pressure. The resulting residue was purified by column chromatography to obtain a compound 10 (light yellow solid, 15.6 mg). $^1H$ NMR (400 MHz, $CDCl_3$) δ 7.55 (d, *J*=5.5 Hz, 1H), 7.35-7.28 (m, 2H), 7.14 (d, *J*=8.5 Hz, 1H), 4.15 (s, 1H), 3.51 (s, 3H), 2.64-2.38 (m, 4H), 2.28-2.09 (m, 4H), 1.59-1.48 (m, 1H).

**Embodiment 11:**

(*R*)-3-(4-cyclopropyl-2-hydroxyphenyl)-6-((1-ethylpiperidin-3-yl)amino)-4-methyl-1,2,4-triaz in-5(4H)-ketone

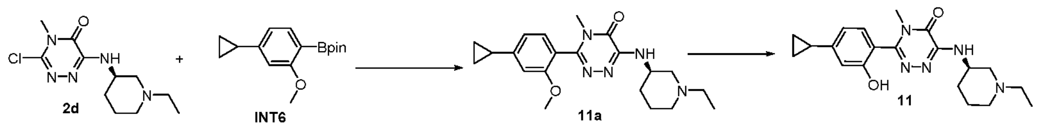


Step 1: the compound **2d** (150 mg, 0.32 mmol), **INT6** (104.2 mg, 0.38 mmol), Pd(dppf)$Cl_2$ (22 mg, 0.03 mmol), and $Cs_2CO_3$ (417.3 mg, 1.28 mmol) were added into the drying flask, under nitrogen atmosphere, the resulting mixture was reacted overnight at 100°C with dioxane as a solvent. After the completion of the reaction was confirmed by TLC, the reaction mixture was filtered, and the filtrate was concentrated under reduced pressure to remove the excess solvent, the resulting residue was purified by column chromatography to obtain a compound **11a** (brown oily liquid, 79 mg). MS/ESI $[M+H]^+$=384.2.

Step 2: under nitrogen atmosphere, the compound **11a** (79 mg, 0.21 mmol) and DCM (1 mL) were added into the drying flask and cooled at-10°C, followed by adding $BBr_3$ (1 M, 0.84 mL) dropwise, and the resulting mixture was reacted for 3 h. After MeOH was added into the reaction solution for quenching the reaction, the reaction solution was returned to room temperature and concentrated under reduced pressure. The resulting residue was purified by column chromatography to obtain a compound 11 (off-white solid, 4.5 mg). $^1$H NMR (400 MHz, $CD_3OD$) $\delta$ 7.61 (d, *J*=4.1 Hz, 1H), 7.16-7.08 (m, 2H), 4.25-4.19 (m, 1H), 3.34 (s, 3H), 2.95-2.75 (m, 4H), 2.15-2.02 (m, 4H), 1.97-1.78 (m, 3H), 1.79-1.62 (m, 2H), 1.33-1.24 (m, 5H), 1.23-1.12 (m, 1H).

**Embodiment 12:**

(*R*)-4-ethyl-6-((1-ethylpiperidin-3-yl)amino)-3-(4-hydroxybenzo[b]thiophen-5-yl)-1,2,4-triazi n-5(4H)-ketone

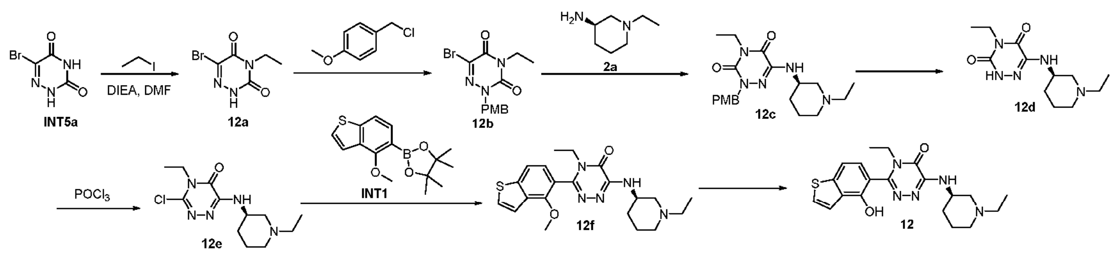


Step 1: the compound **INT5a** (200 mg, 1.04 mmol) was added into the reaction flask, then, DMF (10 mL) and DIEA (269 mg, 2.08 mmol) were added into the reaction flask, followed by slowly dropping iodoethane (163 mg, 1.04 mmol), and the resulting mixture was reacted for 3 h at room temperature, after the completion of the reaction was confirmed by LC-MS, water was added into the reaction mixture, the reaction mixture was extracted with EA and re-extracted with the water phase, the organic phases were combined and concentrated, and the resulting residue was purified by column chromatography and concentrated to obtain a product **12a** (200 mg). LC-MS: ESI$[M+H]^+$=221.0.

Step 2: the compound **12a** (200 mg, 0.91 mmol), potassium carbonate (251 mg, 1.82 mmol) and PMBCl (213 mg, 1.36 mmol) were added in the reaction flask, followed by adding DMF (10 mL), and the resulting mixture was heated to 60°C and reacted for 3 h. After the completion of the reaction was confirmed by LC-MS, water was added into the reaction mixture, the reaction mixture was extracted with EA and re-extracted with the water phase, the organic phases were combined and concentrated, and the resulting residue was purified by column chromatography and concentrated to obtain a product **12b** (110 mg). LC-MS: ESI$[M+H]^+$=341.1.

Step 3: the compound **12b** (110 mg, 0.32 mmol), **2a** (83 mg, 0.65 mmol), palladium acetate (6 mg, 0.03 mmol), BINAP (35 mg, 0.06 mmol), cesium carbonate (185 g, 0.57 mmol), and dioxane (10 mL) were added into the reaction flask, and then stirred for 15 h at 100°C under nitrogen atmosphere. After the reaction was completed, the reaction mixture was cooled to room temperature and concentrated, and the resulting residue was purified by silica gel column chromatography to obtain a product **12c** (69 mg), LC-MS:ESI$[M+H]^+$=387.0.

Step 4: the compound **12c** (69 mg, 0.18 mmol) was added in the reaction flask, and added dichloromethane (5 mL), and cooled to 0°C, followed by slowing dropping trifluoromethanesulfonic acid (1 mL) into the reaction flask, and the resulting mixture was stirred for 15 h at room temperature. After the reaction was completed, the reaction mixture was cooled to 0°C-5°C with acetonitrile (10 mL), solid sodium bicarbonate was added into the cooled mixture until no large amounts of bubbles were generated, then the pH of the cooled mixture was adjusted to be 7-8 with ammonia water, the mixture was dried with anhydrous sodium sulfate, filtered and concentrated under reduced pressure, and the obtained crude product was purified by silica gel column chromatography to obtain a compound **12d** (43 mg), LC-MS:ESI[M+H]+=268.1.

Step 5: the compound **12d** (43 mg, 0.16 mmol) was dispersed in phosphorus oxychloride (10 mL) and the resulting mixture was stirred for 12 h at 110°C under nitrogen atmosphere. After the reaction was completed, the solvent was directly concentrated, the resulting residue was dispersed in acetonitrile (10 mL) and dichloromethane (10 mL), the resulting mixture was cooled to 0°C-5°C, solid sodium bicarbonate was added into the cooled mixture until no large amounts of bubbles were generated, then the pH of the cooled mixture was adjusted to be 7-8 with the ammonia water, and the mixture was dried with anhydrous sodium sulfate, filtered and concentrated under reduced pressure, and the obtained crude product was purified by silica gel column chromatography to obtain a compound **12e** (30 mg), LC-MS:ESI [M+H]+=286.1.

Step 6: the compound **12e** (30 mg, 0.10 mmol), **INT1** (46 mg, 0.16 mmol), Pd(dppf)$_2$Cl$_2$ (7 mg, 0.01 mmol), cesium carbonate (68 mg, 0.21 mmol), dioxane (8 mL)/water (2 mL) were added into the reaction flask and stirred for 15 h at 100°C under nitrogen atmosphere. After the reaction was completed, the reaction mixture was cooled to room temperature, and the resulting residue was purified by silica gel column chromatography to obtain a compound **12f** (22 mg), LC-MS:ESI[M+H]+=414.0.

Step 7: the compound 12f (22 mg, 0.05 mmol) was added into the reaction flask and added DCM (10 mL) and cooled to -10°C, followed by slowly dropping BBr$_3$ (67 mg, 0.27 mmol), and the resulting mixture was reacted for 3 h at -10°C After the completion of the reaction was confirmed by MS, methanol was added into the reaction mixture for quenching the reaction, the reaction solution was concentrated, and the resulting residue was purified by silica gel column chromatography to obtain a compound **12** (5 mg). LC-MS:ESI[M+H]+=400.1. $^1$H NMR (400 MHz, DMSO-*d*$_6$) δ 10.26 (s, 1H), 7.73 (d, *J*=8.0 Hz, 1H), 7.71 (d, *J*=8.0 Hz, 1H), 7.59 (d, *J*=8.0 Hz, 1H), 7.30 (d, *J*=8.0 Hz, 1H), 3.83 (q, *J*=8.0, 4.0 Hz, 2H), 2.38-2.34 (m, 4H), 1.67-1.55 (m, 4H), 1.49-1.45 (m, 2H), 1.14 (t, *J*=8.0 Hz 3H), 1.02 (t, *J*=8.0 Hz 3H).

**Embodiment 13:**

(*R*)-3-(1-(difluoromethylene)-4-hydroxy-2,3-dihydro-1H-inden-5-yl)-4-methyl-6-((1-methylpi peridin-3-yl)amino)-1,2,4-triazin-5 (4H)-ketone

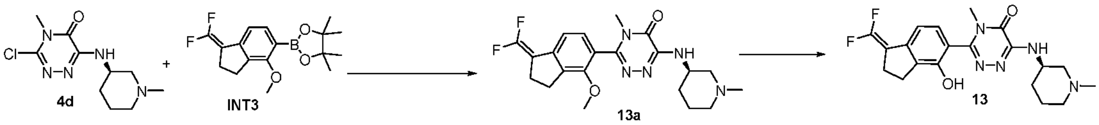

(*R*)-3-(1-(difluoromethylene)-4-hydroxy-2,3-dihydro-1H-inden-5-yl)-4-methyl-6-((1-met hylpiperidin-3-yl) amino)-1,2,4-triazin-5 (4H)-ketone was prepared by referring to Embodiment 2. MS/ESI[M+H]+=404.2. $^1$H NMR (400 MHz,CD$_3$OD) δ 7.28 (d, *J*=7.7 Hz, 1H), 7.13 (d, *J*=7.5 Hz, 1H), 4.33-4.28 (m, 1H), 3.36 (s, 3H), 3.12-3.03 (m, 2H), 2.95-2.91 (m, 2H), 2.84-2.81 (m, 1H), 2.27(s, 3H), 2.25-2.09 (m, 3H), 2.04-1.94 (m, 2H), 1.82-1.74 (m, 1H), 1.42-1.34 (m, 2H).

**Embodiment 14:**

(*R*)-6-((1-(3,3-Difluorocyclobutyl)piperidin-3-yl)amino)-3-(1-(difluoromethylene)-4-hydroxy -2,3-dihydro-1H-inden-5-yl)-4-methyl-1,2,4-triazin-5(4H)-ketone

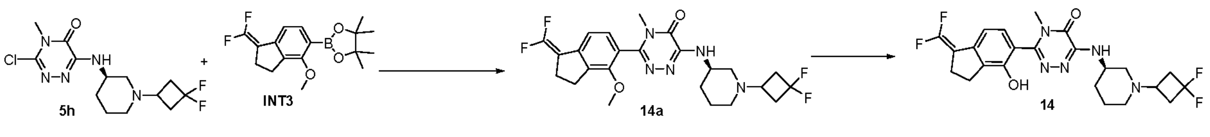

(*R*)-6-((1-(3,3-Difluorocyclobutyl)piperidin-3-yl)amino)-3-(1-(difluoromethylene)-4-hyd roxy-2,3-dihydro-1H-inden-5-yl)-4-methyl-1,2,4-triazin-5(4H)-ketone was prepared by referring to Embodiment 2. MS/ESI[M+H]+=480.2. $^1$H NMR (400 MHz, CD$_3$OD) δ 7.08 (d, *J*=7.9 Hz, 1H), 6.86 (d, *J*=7.8 Hz, 1H), 4.02 (s, 1H), 3.25 (S, 3H), 2.96-2.90 (m, 2H), 2.76-2.72 (m, 2H), 2.65-2.52 (m, 2H), 2.47-2.24 (m, 3H), 2.12-2.07 (m, 1H), 1.98-1.92 (m, 1H), 1.59-1.46 (m, 3H), 1.25-1.23 (m, 2H), 1.20-1.18 (m, 4H).

**Embodiment 15:**

(*R*)-3-(1-(difluoromethylene)-4-hydroxy-2,3-dihydro-1H-inden-5-yl)-6-((1-(ethyl-d5)piperidi n-3-yl)amino)-4-methyl-1,2,4-triazin-5(4H)-ketone

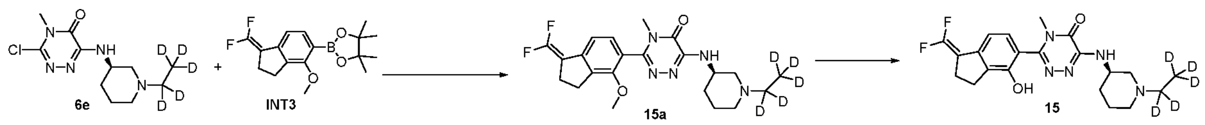

6e + INT3 → 15a → 15

Step 1: under nitrogen atmosphere, the compound **6e** (132 mg, 0.5 mmol), **INT3** (193.2 mg, 0.6 mmol), Pd(dppf) $Cl_2$ (36.6 mg, 0.05 mmol), $Cs_2CO_3$ (652 mg, 2 mmol) and dioxane/$H_2O$ (5 mL/1 mL) were added into the drying flask, and reacted overnight at 100°C. After the completion of the reaction was confirmed by TLC, the reaction solution was returned to room temperature and filtered, and the filtrate was concentrated under reduced pressure. The resulting residue was purified by column chromatography to obtain a compound **15a** (brown oily liquid, 108 mg). MS/ESI$[M+H]^+$=437.2.

Step 2: under nitrogen atmosphere, the compound **15a** (108 mg, 0.25 mmol) and DCM (2 mL) were added into the drying flask and cooled at -10°C, followed by adding $BBr_3$ (1 M, 1 mL) dropwise, and the resulting mixture was reacted for 3 h, after MeOH was added into the reaction solution for quenching the reaction, the reaction solution was returned to room temperature and concentrated under reduced pressure. The resulting residue was purified to obtain a compound **15** (light yellow solid, 42.5 mg). $^1H$ NMR (400 MHz,$CD_3OD$) δ 7.28 (d, *J*=7.7 Hz, 1H), 7.13 (d, *J*=7.5 Hz, 1H), 4.33-4.28 (m, 1H), 3.38 (s, 3H), 3.10-3.04 (m, 2H), 2.94-2.91 (m, 2H), 2.84-2.80 (m, 1H), 2.25-2.09 (m, 3H), 2.04-1.94 (m, 2H), 1.82-1.72 (m, 1H), 1.34-1.31 (m, 2H).

**Embodiment 16:**

(*R*)-6-((1-cyclopropylpiperidin-3-yl)amino)-3-(4-hydroxybenzo[b]thiophen-5-yl)-4-methyl-1, 2,4-triazin-5(4H)-ketone

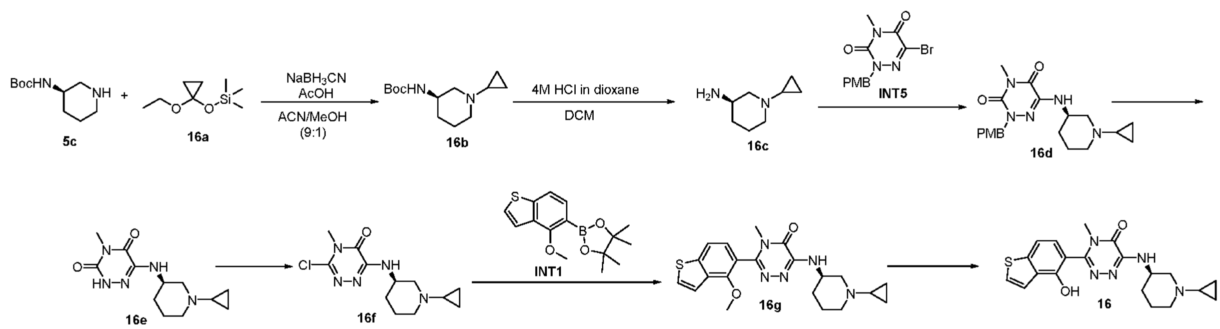


5c + 16a → 16b → 16c → 16d → 16e → 16f → 16g → 16

Step 1: the compound **5c** (400 mg, 2 mmol) and **16a** (522 mg, 3 mmol) were dissolved in 9 mL tetrahydropyran and 1 mL methanol, followed by adding sodium cyanoborohydride (378 mg, 6 mmol) and glacial acetic acid (1.44 g, 24 mmol) sequentially at room temperature under nitrogen atmosphere, and the reaction solution was stirred for 16 h at 65°C. After the completion of the reaction was confirmed by TLC, the reaction solution was filtered, and the filtrate was concentrated under reduced pressure. The resulting residue was diluted with a saturated sodium bicarbonate solution and the diluent was extracted three times with DCM, the organic phases were combined and washed with the saturated brine solution, dried with anhydrous sodium sulfate, and filtered, and the filtrate was concentrated under reduced pressure to obtain a crude product. The crude product was purified by methanol/dichloromethane to obtain a compound **16b** (300 mg, yield: 62.5%), MS/ESI$[M+H]^+$=241.3.

Step 2: the compound **16b** (300 mg, 1.25 mmol) was completely dissolved in anhydrous DCM (5 mL), followed by adding hydrochloric acid dioxane solution (4 M, 10 mL). The resulting mixture was reacted for 2 h at room temperature, and the complete reaction of the raw materials was confirmed by the LC-MS. The reaction solution was concentrated under reduced pressure to obtain a compound **16c** (270 mg), MS/ESI$[M+H]^+$=141.1.

Step 3: the compound **INT5** (300 mg, 0.92 mmol), **16c** (193.2 mg, 1.38 mmol), $Pd(OAc)_2$ (20.2 mg, 0.09 mmol), BINAP (56.1 mg, 0.09 mmol), and $Cs_2CO_3$ (1.47 g, 4.5 mmol) were added into the drying flask, under nitrogen atmosphere, the resulting mixture was reacted overnight at 100°C with dioxane (10 mL) as a solvent. After the completion of the reaction

was confirmed by TLC, the reaction mixture was filtered and concentrated under reduced pressur, the resulting residue was purified by column chromatography to obtain a compound **16d** (yellow oily liquid, 385 mg). MS/ESI[M+H]$^+$=386.2.

Step 4: the compound **16d** (325 mg, 0.84 mmol), DCM (4 mL) and TfOH (378.2 mg, 2.52 mmol) were added into the drying flask and reacted overnight at room temperature, after the completion of the reaction was confirmed by TLC, the pH of the reaction mixture was adjusted to 8-9 with dry $NaHCO_3$ and a small amount of ammonia water, and the reaction mixture was dried with anhydrous $Na_2SO_4$ and filtered, the filtrate was concentrated under reduced pressure, and the resulting residue was purified by column chromatography to obtain a compound **16e** (brown solid, 201 mg). MS/ESI [M+H]$^+$=266.2.

Step 5: the compound **16e** (201 mg, 0.81 mmol) and $POCl_3$ (5 mL) were added into the drying flask, and the resulting mixture was reacted overnight at 100°C, after the reaction was completed, the reaction mixture was concentrated under reduced pressure to remove excess $POCl_3$, the pH of the reaction mixture was adjusted to 8-9 with dry NaHCO3 and a small amount of ammonia water, and the reaction mixture was dried with anhydrous Na2SO4 and filtered, the filtrate was concentrated under reduced pressure, and the resulting residue was purified by column chromatography to obtain a compound **16f** (light brown solid, 102 mg). MS/ESI[M+H]$^+$=284.1.

Step 6: under nitrogen atmosphere, the compound **16f** (102 mg, 0.36 mmol), the compound **INT1** (125.3 mg, 0.43 mmol), Pd(dppf)$Cl_2$ (29.3 mg, 0.04 mmol), Cs2CO$_3$ (469.4 mg, 1.44 mmol) and 1,4-dioxane/$H_2O$ (4 mL/0.8 mL) were added into the drying flask and reacted overnight at 100°C. After the completion of the reaction was confirmed by TLC, the reaction solution was returned to room temperature and filtered, and the filtrate was concentrated under reduced pressure. The resulting residue was purified by column chromatography to obtain a compound **16g** (bright yellow oily liquid, 87 mg). MS/ESI[M+H]$^+$=412.2.

Step 7: under nitrogen atmosphere, the compound **16g** (87 mg, 0.21 mmol) and DCM (2 mL) were added into the drying flask and cooled at -10°C, followed by adding $BBr_3$ (1 M, 0.84 mL) dropwise, and the resulting mixture was reacted for 3 h. After MeOH was added into the reaction solution for quenching the reaction, the reaction solution was returned to room temperature and concentrated under reduced pressure. The resulting residue was purified by column chromatography to obtain a compound 16 (off-white solid, 22.1 mg). $^1$H NMR (400 MHz, $CD_3OD$) δ 7.52 (d, *J=5.5* Hz, 1H), 7.48-7.44 (m, 2H), 7.19 (d, *J*=8.3 Hz, 1H), 4.01 (s, 1H), 3.23 (s, 3H), 3.15-3.02 (m, 2H), 2.97-2.80 (m, 2H), 2.06-1.94 (m, 2H), 1.86-1.68 (m, 2H), 1.14-1.01 (m, 1H), 0.72-0.58 (m, 2H), 0.46-0.33 (m, 2H). MS/ESI[M+H]$^+$=398.2.

**Embodiment 17:**

(*R*)-4-ethyl-3-(4-hydroxybenzo[b]thiophen-5-yl)-6-[(1-methylpiperidin-3-yl)amino]-1,2,4-tria zin-5(4H)-ketone

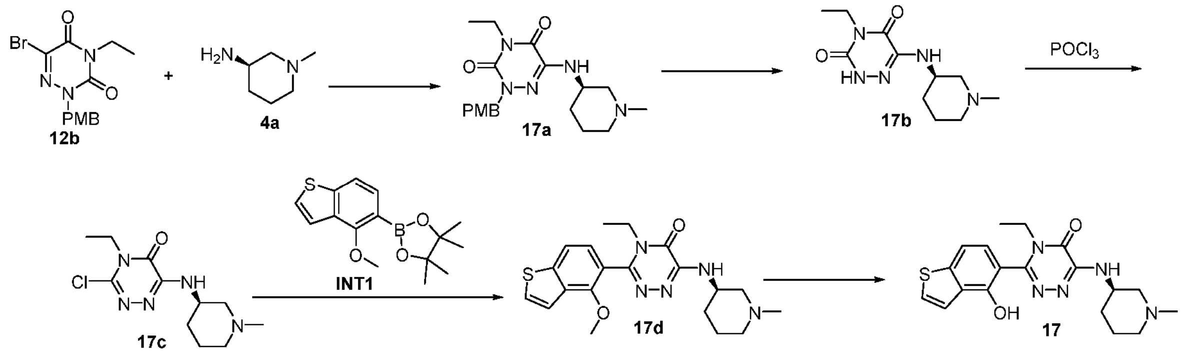


(*R*)-4-ethyl-3-(4-hydroxybenzo[b]thiophen-5-yl)-6-[(1-methylpiperidin-3-yl)amino]-1,2, 4-triazin-5(4H)-ketone was prepared by referring to Embodiment 12. $^1$H NMR (400 MHz, DMSO ) δ 10.22 (s, 1H), 7.73 (d, J=4.0 Hz, 2H), 7.59 (d, J=8.0 Hz, 1H), 7.29 (d, J=8.0 Hz, 1H), 4.14 (s, 1H), 3.72 (q, J=8.0, 4.0 Hz, 2H), 3.05-2.95 (m, 1H), 2.74-2.68 (m, 1H), 2.41 (s, 3H), 1.85-1.73 (m, 2H), 1.68-1.55 (m, 2H), 1.00 (t, J =8.0 Hz, 3H). MS/ESI[M+H]$^+$=386.1.

**Embodiment 18:**

(*R*)-3-(1-hydroxy-2-naphthyl)-4-methyl-6-((1-methylpiperidin-3-yl)amino)-1,2,4-triazin-5(4H )-ketone

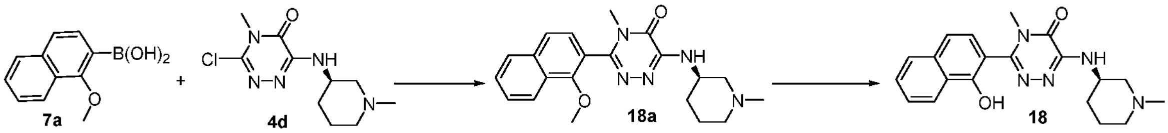
7a + 4d → 18a → 18

(*R*)-3-(1-hydroxy-2-naphthyl)-4-methyl-6-((1-methylpiperidin-3-yl)amino)-1,2,4-triazin-5(4H)-ketone was prepared by referring to Embodiment 2. MS/ESI[M+H]$^+$=366.2.

**Embodiment 19:**

(*R*)-6-((1-ethylpiperidin-3-yl)amino)-3-(1-hydroxynaphthalen-2-yl)-4-methyl-1,2,4-triazin-5( 4H)-ketone

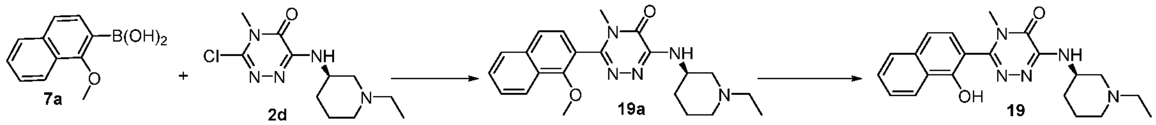
7a + 2d → 19a → 19

(*R*)-6-((1-ethylpiperidin-3-yl)amino)-3-(1-hydroxynaphthalen-2-yl)-4-methyl-1,2,4-triazi n-5(4H)-ketone was prepared by referring to Embodiment 2. MS/ESI[M+H]$^+$=380.2.

**Embodiment 20:**

(*R*)-6-((1-(2-hydroxyethyl)piperidin-3-yl)amino]-3-(1-hydroxynaphthalen-2-yl)-4-methyl-1,2, 4-triazin-5(4H)-ketone

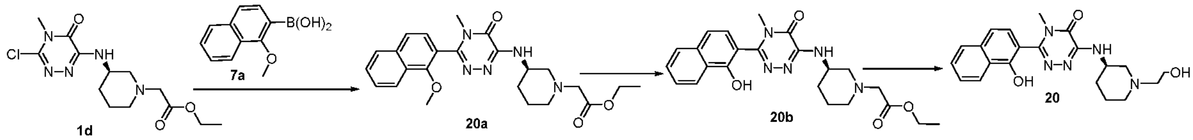
1d, 7a, 20a, 20b, 20

(*R*)-6-((1-(2-hydroxyethyl)piperidin-3-yl)amino]-3-(1-hydroxynaphthalen-2-yl)-4-methyl -1,2,4-triazin-5(4H)-ketone was prepared by referring to Embodiment 1. MS/ESI[M+H]$^+$=396.2.

**Embodiment 21:**

(*R*)-4-cyclopropyl-3-(4-hydroxybenzo[b]thiophen-5-yl)-6-((1-methylpiperidin-3-yl)amino)-1, 2,4-triazin-5(4H)-ketone

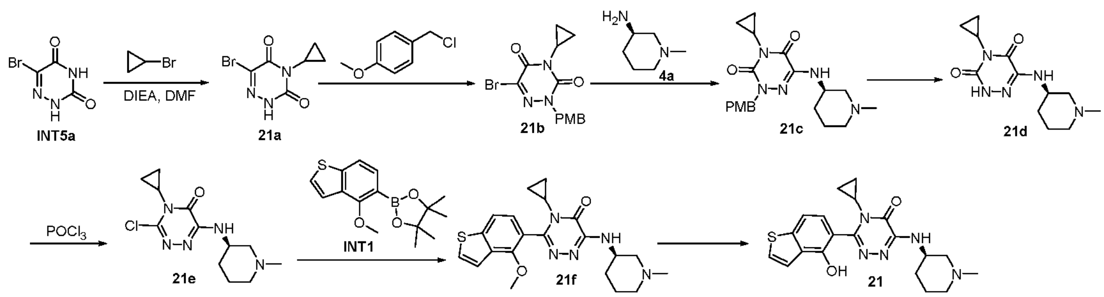

INT5a, 21a, 21b, 4a, 21c, 21d, 21e, INT1, 21f, 21

(*R*)-4-cyclopropyl-3-(4-hydroxybenzo[b]thiophen-5-yl)-6-((1-methylpiperidin-3-yl)amin o)-1,2,4-triazin-5(4H)-ketone was prepared by referring to Embodiment 12. MS/ESI[M+H]$^+$=398.2.

**Embodiment 22:**

(*R*)-4-cyclopropyl-6-((1-ethylpiperidin-3-yl)amino)-3-(4-hydroxybenzo[b]thiophen-5-yl)-1,2, 4-triazin-5(4H)-ketone

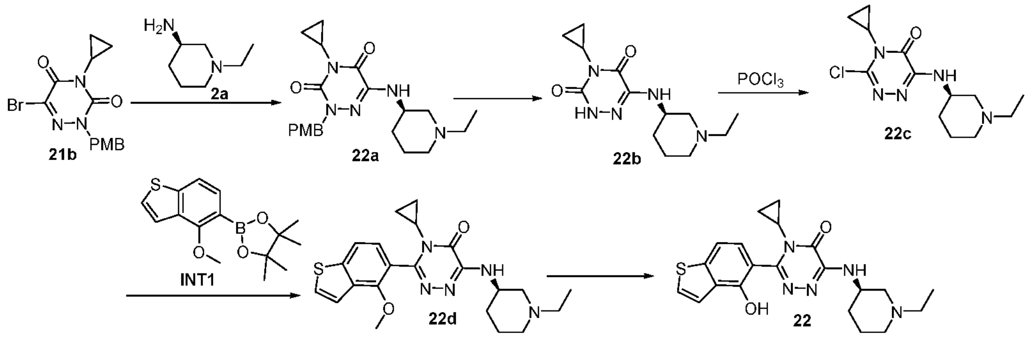


(*R*)-4-cyclopropyl-6-((1-ethylpiperidin-3-yl)amino)-3-(4-hydroxybenzo[b]thiophen-5-yl) -1,2,4-triazin-5(4H)-ketone was prepared by referring to Embodiment 12. MS/ESI$[M+H]^+$=412.2.

**Embodiment 23:**

(*R*)-4-cyclopropyl-3-(4-hydroxybenzo[b]thiophen-5-yl)-6-((1-(2-hydroxyethyl)piperidin-3-yl) amino)-1,2,4-triazin-5(4H)-ketone

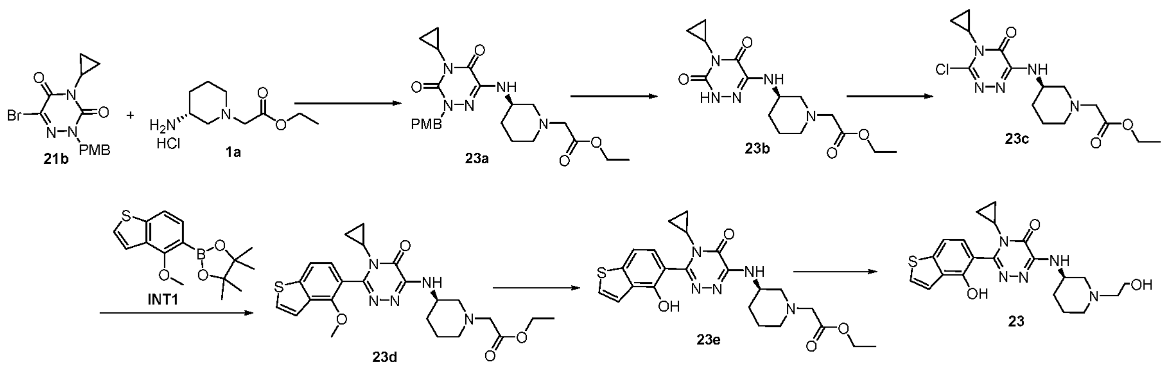


(*R*)-4-cyclopropyl-3-(4-hydroxybenzo[b]thiophen-5-yl)-6-((1-(2-hydroxyethyl)piperidin-3-yl)amino)-1,2,4-triazin-5(4H)-ketone was prepared by referring to Embodiment 11. MS/ESI$[M+H]^+$=428.2.

**Embodiment 24:**

(*R*)-3-(4-ethynyl-2-hydroxyphenyl)-4-methyl-6-((1-methylpiperidin-3-yl)amino)-1,2,4-triazin -5(4H)-ketone

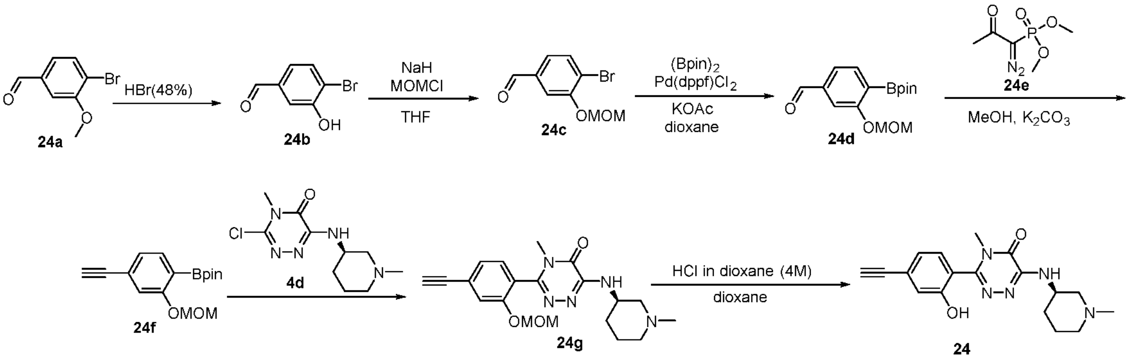


Step 1: the compound **24a** (2.0 g, 9.35 mmol) and HBr (48%, 18 mL) were added into the drying flask, and the resulting mixed solution was stirred overnight at 100°C. After the reaction was completed, the mixed solution was returned to room temperature, quenched with water and extracted three times with ethyl acetate, the resulting organic phases were combined, dried with anhydrous sodium sulfate and filtered, and the filtrate was concentrated under reduced pressure. The resulting residue was purified by column chromatography to obtain a compound **24b** (1.13 g, 60%). LC-MS: ESI $[M+H]^+$=200.9.

Step 2: the compound **24b** (1.13 g, 5.65 mmol) was added into the drying flask and dissolved in THF (20 mL), the resulting solution was cooled to 0°C, followed by adding NaH (60%, 339.2 mg, 8.48 mmol), the resulting mixture was stirred for 20 min, followed by adding MOMCl (797.1 mg, 8.48 mmol), the resulting solution was reacted for 4 h, quenched with a saturated $NH_4Cl$ aqueous solution, dispensed, extracted three times with ethyl acetate, the resulting organic phases were combined, dried with anhydrous sodium sulfate, and filtered,and the filtrate was concentrated under reduced pressure. The resulting residue was purified by column chromatography to obtain a compound **24c** (1.24 g, 90%). LC-MS: ESI $[M+H]^+$=245.1.

Step 3: the compound **24c** (1.24 g, 5.08 mmol), $(Bpin)_2$ (1.55 g, 6.1 mmol), $Pd(dppf)Cl_2$ (36.6 mg, 0.05 mmol), KOAc (1.0 g, 10.2 mmol), and 1,4-dioxane (20 mL) were added into the drying flask and stirred overnight at 100°C. After the reaction was completed, the mixed solution was returned to room temperature and extracted three times with ethyl acetate upon the addition of water, the resulting organic phases were combined, dried with anhydrous sodium sulfate and filtrated, and the filtrate was concentrated under reduced pressure. The resulting residue was purified by column chromatography to obtain a compound 24d (684 mg, 46%). LC-MS: ESI$[M+H]^+$=293.1.

Step 4: $K_2CO_3$ (646.8 mg, 4.68 mmol) and compound **24e** (539.2 mg, 2.81 mmol) were added into the solution of the compound **24d** (684 mg, 2.34 mmol) in MeOH (8 mL) at room temperature, the resulting mixture was reacted for 3 h at room temperature, and the reaction mixture was concentrated under reduced pressure to remove the excess solvent, dissolved in water, extracted with DCM, dried with anhydrous sodium sulfate, and filtered, and the filtrate was concentrated under reduced pressure. The resulting residue was purified by column chromatography to obtain a compound **24f** (366 mg, 54%). LC-MS: ESI$[M+H]^+$=289.2.

Step 5: the compound **24f** (182 mg, 0.63 mmol), **16c** (206.0 mg, 0.76 mmol), $Pd(dppf)Cl_2$ (43.9 mg, 0.06 mmol), $Cs_2CO_3$ (616.1 mg, 1.89 mmol) and 1,4-dioxane (20 mL) were added into the drying flask and reacted overnight at 100°C, after the reaction was completed, the mixed solution was returned to room temperature and filtered, and the filtrate was concentrated under reduced pressure. The resulting residue was purified by column chromatography to obtain a compound 24g (87 mg, 35%). LC-MS: ESI$[M+H]^+$=384.2.

Step 6: the compound 24g (87 mg, 0.22 mmol),1,4-dioxane (3 mL), and HCl (4 M, in dioxane) (0.3 mL) were added into the drying flask, and reacted for 3 h at room temperature, the reaction mixture was concentrated under reduced pressure to remove the excess solvent to obtain a compound **24** (9.8 mg, 13%). LC-MS: ESI$[M+H]^+$=340.2. $^1H$ NMR (400 MHz, $CD_3OD$) $\delta$ 7.30 (d, *J*=7.8 Hz, 1H), 7.05 (d, *J*=7.8 Hz, 1H), 7.01 (s, 1H), 4.20-4.13 (m, 1H), 3.32 (s, 3H),3.04-2.88 (m, 1H), 2.72-2.54 (m, 1H), 2.32 (s, 3H), 2.30-2.19 (m, 2H), 1.98-1.86 (m, 1H), 1.84-1.75 (m, 1H), 1.70-1.55 (m, 2H), 1.34-1.32 (m, 1H).

## Embodiment 25:

(*R*)-6-((1-ethylpiperidin-3-yl)amino)-3-(4-ethynyl-2-hydroxyphenyl)-4-methyl-1,2,4-triazin-5 (4H)-ketone

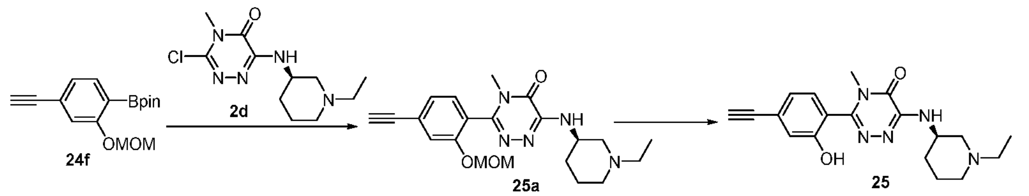


(*R*)-6-((1-ethylpiperidin-3-yl)amino)-3-(4-ethynyl-2-hydroxyphenyl)-4-methyl-1,2,4-tria zin-5(4H)-ketone was prepared by referring to Embodiment 24. MS/ESI[M+H]$^+$=354.2. $^1$H NMR (400 MHz, $CD_3OD$) δ 7.33 (d, *J*=7.8 Hz, 1H), 7.11-7.00 (m, 2H), 4.20-4.13 (m, 1H), 3.60 (s, 1H), 3.35 (s, 1H), 3.10-3.02 (m, 1H), 2.77-2.68 (m, 1H), 2.52 (dd, *J*=14.2, 7.0 Hz, 2H), 2.34-2.21 (m, 1H), 2.02-1.92 (m, 1H), 1.86-1.78 (m, 1H), 1.75-1.67 (m, 1H), 1.64-1.54 (m, 1H), 1.36-1.31 (m,1H), 1.14 (t, *J*=7.2 Hz, 3H).

**Embodiment 26:**

(*R*)-3-(4-ethynyl-2-hydroxyphenyl)-6-((1-(2-hydroxyethyl)piperidin-3-yl)amino)-4-methyl-1, 2,4-triazin-5(4H)-ketone

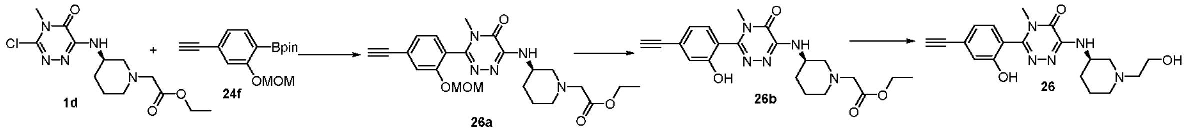


(*R*)-3-(4-ethynyl-2-hydroxyphenyl)-6-((1-(2-hydroxyethyl)piperidin-3-yl)amino)-4-meth yl-1,2,4-triazin-5(4H)-ketone was prepared by referring to Embodiment 1. MS/ESI[M+H]$^+$=370.2.

**Embodiment 27:**

(*R*)-3-(2-hydroxy-4-(prop-1-yn-1-yl)phenyl)-4-methyl-6-((1-methylpiperidin-3-yl)amino)-1,2, 4-triazin-5(4H)-ketone

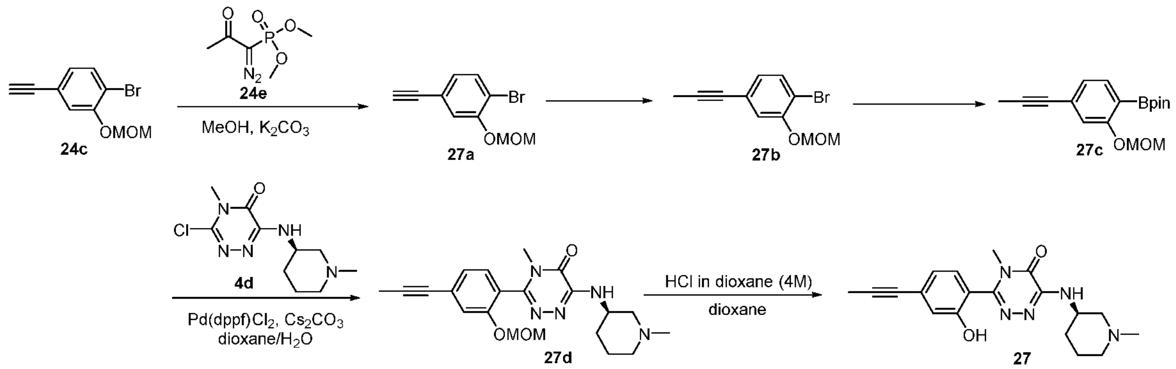


Step 1: the compound **24c** (8 g, 30.88 mmol, 1.0 eq), **24e** (8.9 g, 46.32 mmol, 1.5 eq) and anhydrous potassium carbonate (8.5 g, 61.76 mmol, 2.0 eq) were added into methanol (150 mL) and the resulting mixture was reacted at room temperature for 3 h. No raw materials were detected by TLC, the reaction mixture was concentrated under reduced pressure, and extracted with ethyl acetate (30 mL x 3) upon the addition of water (30 mL). The resulting organic phases were combined, dried with anhydrous sodium sulfate, and filtered. The filtrate was concentrated under reduced pressure, and the crude product was purified by silica gel column chromatography (EA: PE=1: 150) to obtain a compound 27a (3 g, yield: 38.5%).

Step 2: the compound **27a** (500 mg, 1.96 mmol, 1.0 eq) was dissolved in anhydrous THF (10 mL), and the resulting solvent was cooled to -70°C, followed by adding 1.6 mol/L n-butyl lithium THF solution (1.35 mL, 1.1 eq) dropwise, the resulting mixture was reacted for 1 h at -75°C to -70°C, followed by adding methyl iodide (834.6 mg, 5.88 mmol, 3.0 eq), the resulting solvent was normally heated to room temperature and reacted for 16 h. After the completion of the reaction was confirmed by LC-MS, the saturated ammonium chloride aqueous solution (50 mL) was added into the reaction

mixture, the resulting solvent was extracted with ethyl acetate (50 mL x 2), and the resulting organic phases were combined, dried with anhydrous sodium sulfate and filtered, the filtrate was concentrated under reduced pressure, and the crude product was purified by silica gel column chromatography (EA: PE=1: 100) to obtain a compound 27b (485 mg, yield: 91.9%).

Step 3: the compound **27b** (1.28 g, 5.0 mmol), $(Bpin)_2$ (1.55 g, 6.0 mmol), $Pd(dppf)Cl_2$ (36.6 mg, 0.05 mmol), KOAc (1.0 g, 10.2 mmol), and 1,4-dioxane (20 mL) were added into the drying flask and stirred overnight at 100°C. After the reaction was completed, the mixed solution was returned to room temperature and extracted three times with ethyl acetate upon the addition of water, the resulting organic phases were combined, dried with anhydrous sodium sulfate and filtrated, and the filtrate was concentrated under reduced pressure. The resulting residue was purified by column chromatography to obtain a compound 27c (684 mg, 46%). LC-MS: ESI$[M+H]^+$=303.1.

(*R*)-3-(2-hydroxy-4-(prop-1-yn-1-yl)phenyl)-4-methyl-6-((1-methylpiperidin-3-yl)amino )-1,2,4-triazin-5(4H)-ketone was prepared by referring to Embodiment 24. LC-MS: ESI$[M+H]^+$=354.2. $^1H$ NMR (400 MHz, DMSO-$d_6$) δ 10.36 (br s, 1H), 7.31 (d, *J*=8.0 Hz, 1H), 7.06-6.93 (m, 2H), 6.82 (d, *J*=8.0 Hz, 1H), 4.18-3.91 (m, 1H), 3.23 (s, 3H), 2.79-2.70 (m, 1H), 2.54-2.45 (m, 1H), 2.24 (s, 3H), 2.21-2.08 (m, 5H), 1.79-1.52 (m, 4H).

**Embodiment 28:**

(*R*)-6-((1-ethylpiperidin-3-yl)Jamino)-3-(2-hydroxy-4-(prop-1-yn-1-yl)phenyl)-4-methyl-1,2,4-triazin-5(4H)-ketone

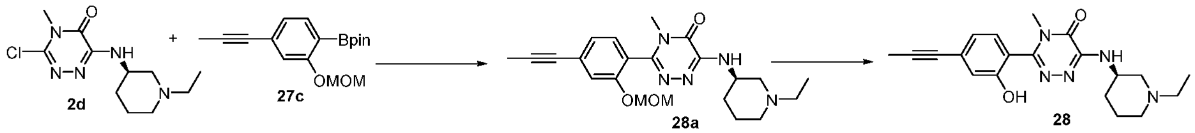

(*R*)-6-((1-Ethylpiperidin-3-yl)amino)-3-(2-hydroxy-4-(prop-1-yn-1-yl)phenyl)-4-methyl-1,2,4-triazin-5(4H)-ketone was prepared by referring to Embodiment 27. MS/ESI$[M+H]^+$=368.2. $^1H$ NMR (400 MHz, Methanol-$d_4$) δ 7.25 (d, *J*=8.0 Hz, 1H), 6.95 (dd, *J*=8.0, 1.2 Hz, 1H), 6.91 (d, *J*=1.2 Hz, 1H), 4.60 (br s, 1H), 4.23-4.06 (m, 1H), 3.32 (s, 3H), 3.09-2.98 (m, 1H), 2.75-2.63 (m, 1H), 2.56-2.41 (m, 2H), 2.35-2.19 (m, 2H), 2.03 (s, 3H), 1.99-1.91 (m, 1H), 1.86-1.77 (m, 1H), 1.74-1.52 (m, 2H), 1.12 (d, *J*=7.2 Hz, 3H).

**Embodiment 29:**

(R)-6-((1-(2-hydroxyethyl)piperidin-3-yl)amino)-3-(2-hydroxy-4-(prop-1-yn-1-yl)phenyl)-4-methyl-1,2,4-triazin-5(4H)-ketone

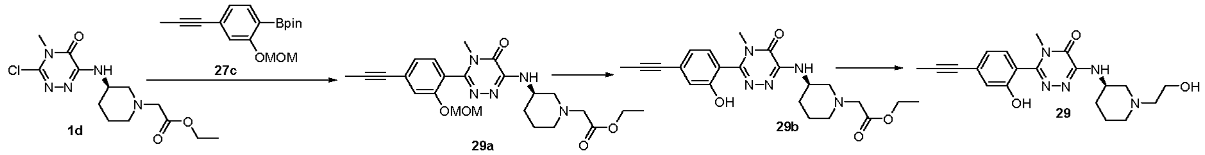

(R)-6-((1-(2-hydroxyethyl)piperidin-3-yl)amino)-3-(2-hydroxy-4-(prop-1-yn-1-yl )phenyl )-4-methyl-1,2,4-triazin-5(4H)-ketone was prepared by referring to Embodiment 1. MS/ESI$[M+H]^+$=384.2.

**Embodiment 30:**

3-(4-hydroxybenzo[b]thiophen-5-yl)-6-(((1R,2R)-2-hydroxycyclohexyl)amino)-4-methyl-1,2, 4-triazin-5(4H)-ketone.

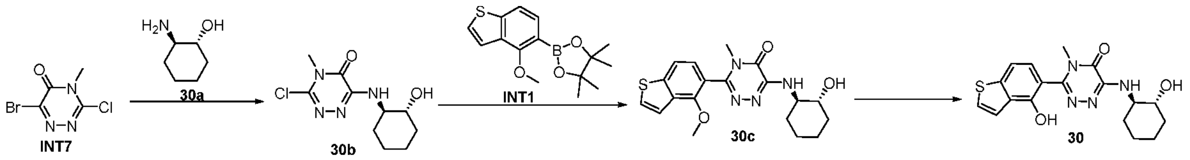


Step 1: the compound **INT7** (444 mg, 2.00 mmol), **30a** (230 mg, 2.00 mmol), and DIPEA (1.03 g, 8 mmol) were added into the drying flask, added 1,4-dioxane (15 mL) as a solven, the resulting mixture was reacted overnight at 80°C. After the reaction was completed, the reaction mixture was concentrated under reduced pressure to remove the excess solvent, the resulting residue was purified by column chromatography to obtain a compound **30b** (526 mg). MS/ESI $[M+H]^+$=259.1.

Step 2: the compound **30b** (163 mg, 0.63 mmol), **INT1** (250.9 mg, 0.76 mmol), Pd(dppf)$Cl_2$ (43.9 mg, 0.06 mmol), and $Cs_2CO_3$ (782.4 mg, 2.4 mmol) were added into the drying flask, under nitrogen atmosphere, the resulting mixture was reacted overnight at 100°C with 1,4-dioxane/$H_2O$ (6 mL/0.5 mL) as a solvent. After the completion of the reaction was confirmed by TLC, the reaction mixture was filtered, and the filtrate was concentrated under reduced pressure to remove the excess solvent, the resulting residue was purified by column chromatography to obtain a compound **30c** (202 mg). MS/ESI$[M+H]^+$=387.1.

Step 3: under nitrogen atmosphere, the compound **30c** (66 mg, 0.17 mmol) and DCM (1.5 mL) were added into the drying flask and cooled at -10°C, followed by adding $BBr_3$ (1 M, 0.7 mL) dropwise, and the resulting mixture was reacted for 3 h. After MeOH was added into the reaction solution for quenching the reaction, the reaction solution was returned to room temperature and concentrated under reduced pressure. The resulting residue was purified by column chromatography to obtain a compound 30 (33.5 mg). MS/ESI$[M+H]^+$=373.2. $^1$H NMR (400 MHz, DMSO-$d_6$) $\delta$ 7.66-7.60 (m, 2H), 7.49 (d, $J$=8.4 Hz, 1H), 7.19 (d, $J$=8.4 Hz, 1H), 6.65 (d, $J$=7.2 Hz, 1H), 4.75-4.64 (m, 1H), 3.63-3.52 (m, 1H), 3.49-3.40 (m, 1H), 3.13 (s, 3H), 2.09-1.99 (m, 1H), 1.91-1.79 (m, 1H), 1.66-1.51 (m, 2H), 1.28-1.10 (m, 4H).

**Embodiment 31:**

3-(4-ethynyl-2-hydroxyphenyl)-6-(((1R,2R)-2-hydroxycyclohexyl)amino)-4-methyl-1,2,4-tria zin-5(4H)-ketone

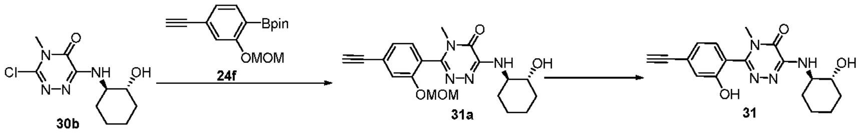


3-(4-ethynyl-2-hydroxyphenyl)-6-(((1R,2R)-2-hydroxycyclohexyl)amino)-4-methyl-1,2, 4-triazin-5(4H)-ketone was prepared by referring to Embodiment 24. MS/ESI$[M+H]^+$=341.2.

**Embodiment 32:**

3-(2-hydroxy-4-(prop-1-yn-1-yl)phenyl)-6-(((1*R*,2*R*)-2-hydroxycyclohexyl)amino)-4-methyl-1,2,4-triazin-5(4H)-ketone.

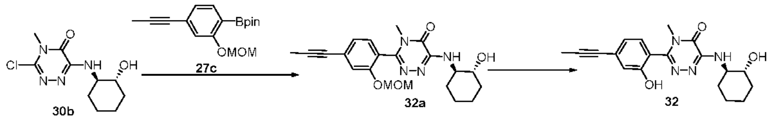


3-(2-hydroxy-4-(prop-1-yn-1-yl)phenyl)-6-(((1R,2R)-2-hydroxycyclohexyl)amino)-4-methyl-1,2,4-triazin-5(4H)-ketone was prepared by referring to Embodiment 24. MS/ESI$[M+H]^+$=355.2.

**Embodiment 33:**

6-(((1*R*,2*R*)-2-hydroxycyclohexyl)amino)-3-(1-hydroxynaphthalene-2-yl)-4-methyl-1,2,4-tria zine-5(4H)-ketone

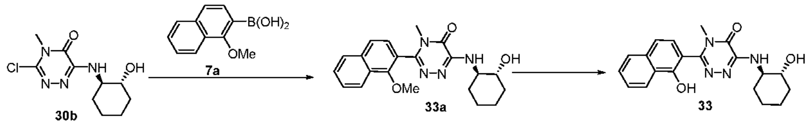


6-(((1R,2R)-2-hydroxycyclohexyl)amino)-3-(1-hydroxynaphthalene-2-yl)-4-methyl-1,2,4 -triazine-5(4H)-ketone was prepared by referring to Embodiment 30. MS/ESI[M+H]$^+$=367.2.

**Embodiment 34:**

(*R*)-3-(4-hydroxybenzo[b]thiophen-5-yl)-4-methyl-6-(((4-methylmorpholino-2-yl)methyl)ami no)-1,2,4-triazine-5(4H)-ketone

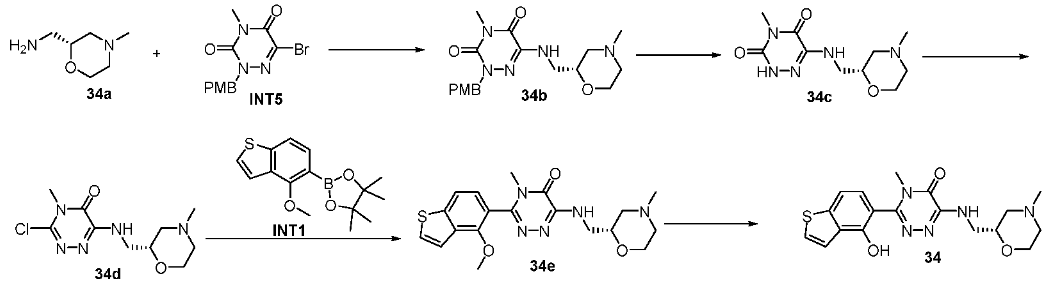


(*R*)-3-(4-hydroxybenzo[b]thiophen-5-yl)-4-methyl-6-(((4-methylmorpholino-2-yl)methyl )amino)-1,2,4-triazine-5(4H)-ketone was prepared by referring to Embodiment 4. MS/ESI[M+H]$^+$=388.2. $^1$H NMR (400 MHz, $CD_3OD$) δ 7.53 (d, *J*=5.7 Hz, 1H), 7.43-7.37 (m, 2H), 7.21 (d, *J*=8.3 Hz, 1H), 4.03 (s, 1H), 3.62-3.57 (m, 2H), 3.52-3.47 (m, 2H), 3.35 (s,1H), 3.25 (s, 3H), 2.68-2.62 (m, 2H), 2.63-2.54 (m, 2H), 2.48-2.42 (m, 2H), 2.18 (s, 3H).

**Embodiment** 35:

(*S*)-3-(4-hydroxybenzo[b]thiophen-5-yl)-4-methyl-6-(((4-methylmorpholino-2-yl)methyl)ami no)-1,2,4-triazine-5(4H)-ketone

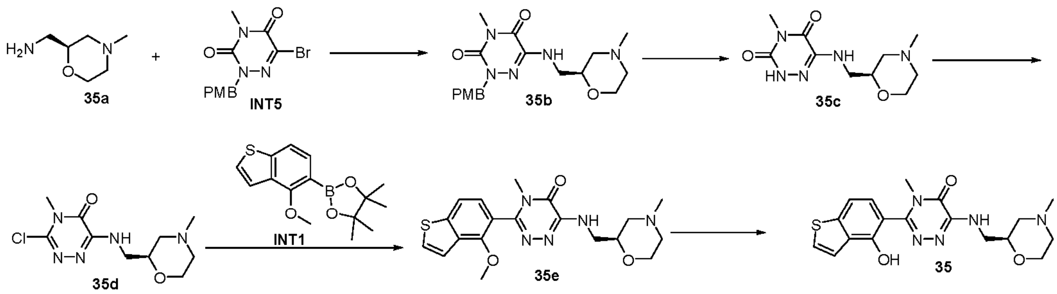


(*S*)-3-(4-hydroxybenzo[b]thiophen-5-yl)-4-methyl-6-(((4-methylmorpholino-2-yl)methyl )amino)-1,2,4-triazine-5(4H)-ketone was prepared by referring to Embodiment 4. MS/ESI[M+H]$^+$=388.2. $^1$H NMR (400 MHz, $CD_3OD$) δ 7.53 (d, *J*=5.7 Hz, 1H), 7.43-7.37 (m, 2H), 7.21 (d, *J*=8.3 Hz, 1H), 4.03 (s, 1H), 3.62-3.57 (m, 2H), 3.52-3.47 (m, 2H), 3.35 (s,1H), 3.25 (s, 3H), 2.68-2.62 (m, 2H), 2.63-2.54 (m, 2H), 2.48-2.42 (m, 2H), 2.18 (s, 3H).

**Embodiment 36:**

(*R*)-3-(4-hydroxybenzo[b]thiophen-5-yl)-4-methyl-6-(((1-isopropylpiperidin-3-yl)amino)-1,2, 4-triazin-5(4H)-ketone

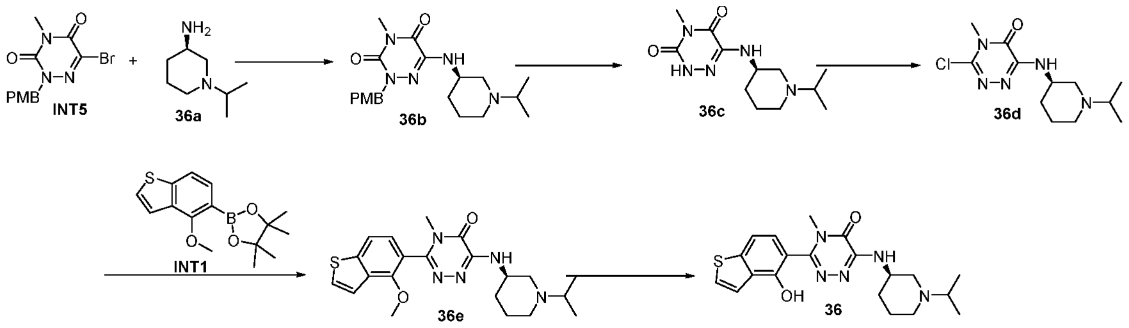

INT5, 36a, 36b, 36c, 36d, INT1, 36e, 36

(*R*)-3-(4-hydroxybenzo[b]thiophen-5-yl)-4-methyl-6-(((1-isopropylpiperidin-3-yl)amino) -1,2,4-triazin-5(4H)-ketone was prepared by referring to Embodiment 4. MS/ESI[M+H]+=400.2. $^1$H NMR (400 MHz, DMSO-$d_6$) δ 8.16 (s, 1H), 7.76 - 7.64 (m, 2H), 7.58 (d, *J*=8.2 Hz, 1H), 7.27 (d, *J*=8.2 Hz, 1H), 6.84 (d, *J*=8.4 Hz, 1H), 4.11-4.03 (m, 1H), 3.19 (s, 3H), 2.91-2.83 (m, 2H), 2.69-2.61 (m, 1H), 2.45-2.36 (m, 2H), 1.79-1.51 (m, 4H), 1.02 (dd, *J*=6.5, 1.8 Hz, 6H).

**Embodiment 37:**

(*R*)-6-((5,5-difluoro-1-methylpiperidin-3-yl)amino)-3-(4-hydroxybenzb[b]thiophen-5-yl)-4-m ethyl-1,2,4-triazin-5(4H)-ketone

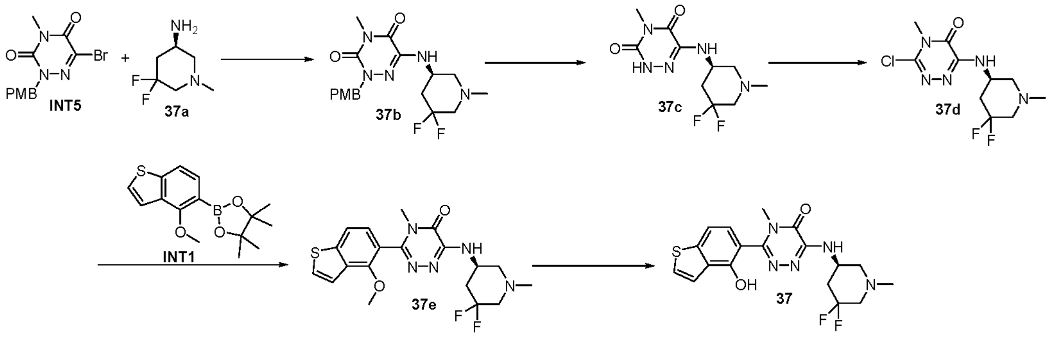

INT5, 37a, 37b, 37c, 37d, INT1, 37e, 37

(*R*)-6-((5,5-difluoro-1-methylpiperidin-3-yl)amino)-3-(4-hydroxybenzb[b]thiophen-5-yl) -4-methyl-1,2,4-triazin-5(4H)-ketone was prepared by referring to Embodiment 4. MS/ESI[M+H]+=408.2. $^1$H NMR (400 MHz, $CD_3OD$) δ 7.52 (d, *J=5.5* Hz, 1H), 7.48-7.44 (m, 2H), 7.19 (d, *J*=8.3 Hz,1 H), 4.01 (s, 1H), 3.23 (s, 3H), 2.87 (s,1H), 2.68-2.62 (m, 2H), 2.63-2.54 (m, 2H), 2.18 (s, 3H), 2.15-2.12(m, 2H).

**Embodiment 38:**

(R)-3-(4-(difluoromethoxy)benzo[b]thiophen-5-yl)-4-methyl-6-((1-methylpiperidin-3-yl)amin o)-1,2,4-triazin-5(4H)-ketone

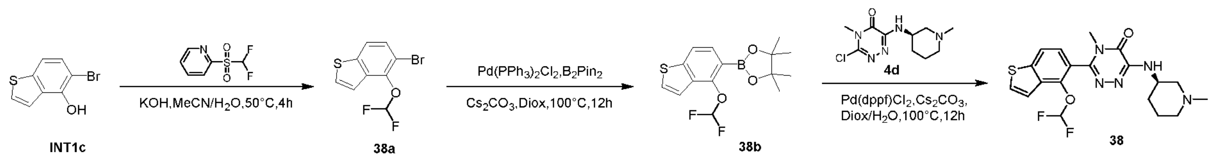


INT1c, 38a, 38b, 4d, 38

Step 1: 5-bromobenzo[b]thiophen-4-ol **INT1c** (1.00 g, 4.37 mmol), 2-(difluoromethyl)sulfonyl)pyridine (1.69 g, 8.74 mmol), KOH (1.23 g, 21.85 mmol), dioxane (10 mL), and water (2.5 mL) were added in the reaction flask, and stirred for 4 h at 50°C. After the reaction was completed, the reaction system was layered, the upper organic phases were collected, directly mixed with the silica gel, and purified by silica gel column chromatography (petroleum ether: ethyl acetate=30:1) to obtain a compound **38a** (1.10 g, 3.94 mmol, 90%). $^1$H NMR (400 MHz, $CDCl_3$) δ 7.65 (d, *J*=8.6 Hz, 1H),

7.54 (d, *J*=8.6 Hz, 1H), 7.50 (t, *J*=6.4 Hz, 2H), 6.61 (t, *J*=74.3 Hz, 1H). $^{19}$F NMR (377 MHz, $CDCl_3$) δ -80.02.

Step 2: the compound **38a** (1.10 g, 3.94 mmol), bis(pinacolato)diboron (1.20 g, 4.73 mmol), potassium acetate (773 mg, 7.88 mmol), and trans-dichlorobis(triphenyl-phosphine)palladium(II) (274 mg, 0.39 mmol) were added into the reaction flask, followed by adding dioxane (10 mL), under nitrogen atmosphere, the resulting mixture was stirred for 12 h at 100°C. After the reaction was completed, the reaction mixture was directly mixed with the silica gel, and purified by silica gel column chromatography (petroleum ether: ethyl acetate=20:1) to obtain a compound **38b** (760 mg, 2.33 mmol, 59%). $^1$H NMR (400 MHz, $CDCl_3$) δ 7.78 (dd, *J*=8.1, 0.5 Hz, 1H), 7.71 (d, *J*=8.1 Hz, 1H), 7.56 (d, *J*=5.3 Hz, 1H), 7.43 (d, *J=5.5* Hz, 1H), 6.68 (t, *J*=76.2 Hz, 1H), 1.38 (s, 12H). $^{19}$F NMR (377 MHz, $CDCl_3$) δ -80.86.

Step 3: the compound **4d** (100 mg, 0.39 mmol), 2-(4-(difluoromethoxy)benzo[b]-thiophen-5-yl)-4,4,5,5-tetramethyl-1,3,2-dioxaborane **38b** (153 mg, 0.47 mmol), cesium carbonate (254 mg, 0.78 mmol), and [1,1'-bis(diphenylphosphino)ferrocene]palladium dichloride (29.3 mg, 0.04 mmol) were added into dioxane (1 mL) and water (0.2 mL), under nitrogen atmosphere, the resulting mixture was stirred for 12 h at 100°C. After the reaction was completed, the reaction mixture was directly mixed with the silica gel, and purified by silica gel column chromatography (dichloromethane: methanol=10:1) to obtain a crude product, and then the crude product was subjected to reversed-phase preparative separation to obtain a compound **38** (40.0 mg, 0.095 mmol, 24%). MS/ESI[M+H]$^+$=422.2. $^1$H NMR (400 MHz, MeOD) δ 8.50 (s, 1H), 8.07 (d, *J*=8.3 Hz, 1H), 7.84 (d, *J*=5.6 Hz, 1H), 7.57 (d, *J*=5.6 Hz, 1H), 7.51 (d, *J*=8.3 Hz, 1H), 6.81 (t, *J*=73.2 Hz, 1H), 4.31-4.20 (m, 1H), 3.37 (d, *J*=9.0 Hz, 1H), 3.29 (s, 3H), 3.16-3.01 (m, 1H), 2.74 (d, *J*=8.2 Hz, 2H), 2.66 (s, 3H), 2.12-1.93 (m, 2H), 1.86-1.66 (m, 2H).

**Embodiment 39:**

(*R*)-3-(4-cyclopropyl-2-hydroxyphenyl)-4-methyl-6-((1-methylpiperidin-3-yl)amino)-1,2,4-tri azin-5(4H)-ketone

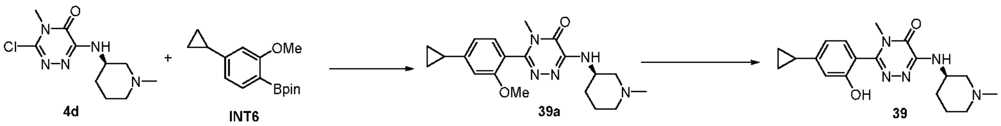

4d INT6 39a 39

(*R*)-3-(4-cyclopropyl-2-hydroxyphenyl)-4-methyl-6-((1-methylpiperidin-3-yl)amino)-1,2 ,4-triazin-5(4H)-ketone was prepared by referring to Embodiment 11. MS/ESI[M+H]$^+$=356.2. $^1$H NMR (400 MHz, $CD_3OD$) δ 7.55 (d, *J*=4.1 Hz, 1H), 7.17-7.10 (m, 2H), 4.25-4.19 (m, 1H), 3.34 (s, 3H), 2.95-2.75 (m, 3H), 2.18 (s, 3H), 1.97-1.78 (m, 3H), 1.77-1.61 (m, 2H), 1.32-1.25 (m, 5H), 1.23-1.12 (m, 1H).

**Embodiment 40:**

6-((3*R*,5*R*)-5-fluoro-1-methylpiperidine-3-yl)amino)-3-(4-hydroxybenzo[b]thiophene-5-yl)-4-methyl-1,2,4-triazine -5(4H)-ketone

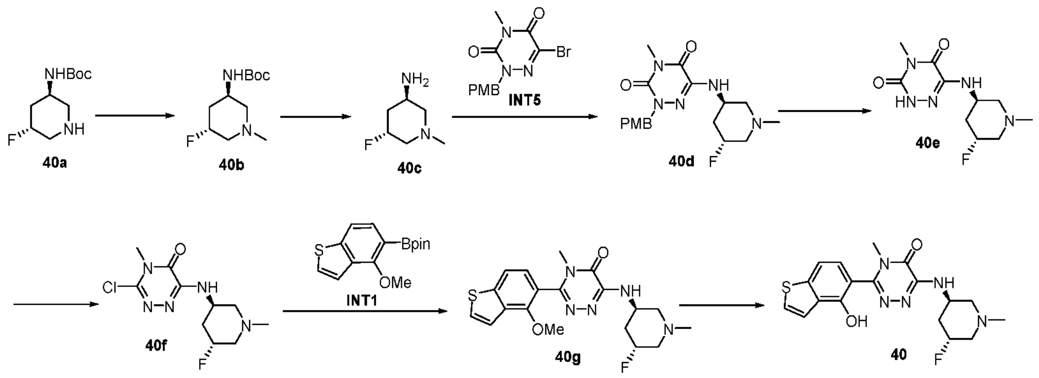

40a 40b 40c INT5 40d 40e 40f INT1 40g 40

Step 1: the compound **40a** (4.75 g, 21.8 mmol), paraformaldehyde (1.31 g, 43.6 mmol), and acetic acid (0.26 g, 4.36 mmol) were added into methanol (50 mL), followed by adding sodium cyanoborohydride (2.74 g, 43.6 mmol), and the resulting mixture was stirred for 1 h at 50°C. After the reaction was completed, the reaction mixture was mixed with silica gel

and concentrated under reduced pressure, and purified by column chromatography (DCM: MeOH=10: 1, iodine color development) to obtain a compound **40b** (4.67 g, 20.1 mmol, yield: 92%). $^1$H NMR (400 MHz, $CDCl_3$-*d*) δ 4.92-4.64 (m, 2H), 3.99 (s, 1H), 2.77-2.39 (m, 3H), 2.30 (s, 3H), 1.94-1.80 (m, 1H), 1.45 (s, 9H). MS/ESI[M+H]$^+$=233.0.

Step 2: the compound **40b** (4.67 g, 20.1 mmol) was dissolved in dioxane (25 mL), followed by adding HCl/dioxane (25 mL, 101 mmol, 4.0 M), and the resulting mixture was stirred for 1 h at 25°C, after the reaction was completed, the reaction mixture was concentrated under reduced pressure to obtain a target product **40c** (4.60 g, 22.4 mmol, yield: 111%). $^1$H NMR (400 MHz, $CD_3OD$-$d_4$) δ 5.37-5.24 (m, 1H), 4.98-4.89 (m, 1H), 3.92-3.77 (m, 3H), 3.51 (dd, *J*=39.2, 13.9 Hz, 1H), 3.06 (s, 3H), 2.63-2.55 (m, 1H), 2.15-1.98 (m, 1H). MS/ESI[M+H]$^+$=133.0.

6-(((3R, 5R)-5-fluoro-1-methylpiperidine-3-yl) amino)-3-(4-hydroxybenzo[b]thiophene-5-yl)-4-methyl-1,2,4-triazine -5(4H)-ketone was prepared by referring to Embodiment 4. MS/ESI[M+H]$^+$=390.0. $^1$H NMR (400 MHz, $CDCl_3$-*d*) δ 7.60 (d, *J*=5.4 Hz, 1H), 7.43-7.31 (m, 2H), 7.19 (d, *J*=8.5 Hz, 1H), 6.17 (d, *J*=8.1 Hz, 1H), 4.90-4.72 (m, 1H), 4.45 (s, 1H), 3.57 (s, 3H), 2.91-2.78 (m, 1H), 2.68-2.60 (m, 1H), 2.56-2.48 (m, 1H), 2.46-2.38 (m, 1H), 2.37 (s, 3H), 2.28-2.17 (m, 1H), 1.91-1.81 (m, 1H).

**Embodiment 41:**

6-(((3*R*,5*R*)-1-ethyl-5-fluoropiperidin-3-yl)amino)-3-(4-hydroxybenzo[b]thiophen-5-yl)-4-me thyl-1,2,4-triazin-5(4H)-ketone

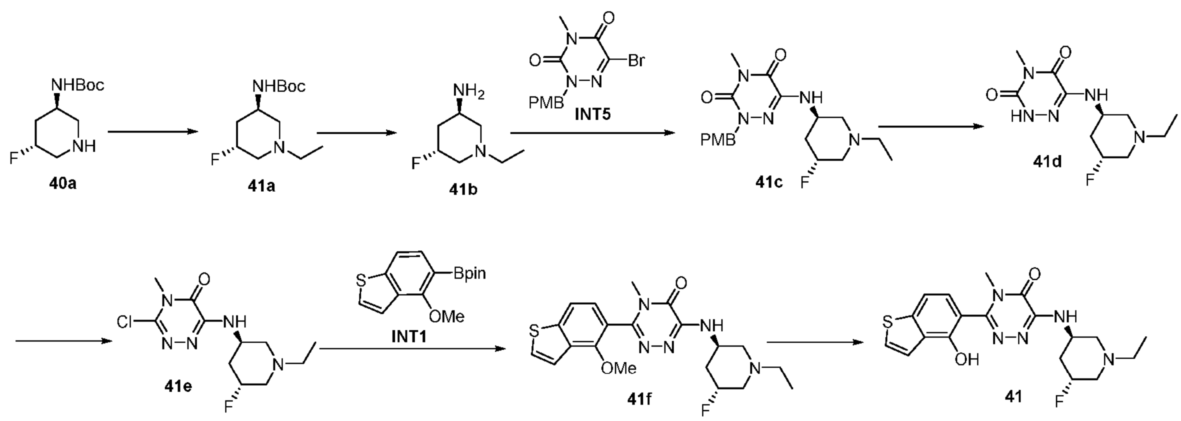
40a
41a
41b
INT5
41c
41d
41e
INT1
41f
41

6-(((3*R*,5*R*)-1-ethyl-5-fluoropiperidin-3-yl)amino)-3-(4-hydroxybenzo[b]thiophen-5-yl)-4-methyl-1,2,4-triazin-5(4H)-ketone was prepared by referring to Embodiment 40. MS/ESI[M+H]$^+$=404.0. $^1$H NMR (400 MHz, $CD_3OD$-*d*) δ 7.61 (d, *J*=5.6 Hz, 1H), 7.58-7.49 (m, 2H), 7.28 (d, *J*=8.3 Hz, 1H), 4.98-4.93 (m, 1H), 4.85-4.80 (m, 2H), 4.51-4.43 (m, 1H), 3.34 (s, 3H), 3.12-3.01 (m, 1H), 2.98-2.83 (m, 1H), 2.55 (t, *J*=7.2 Hz, 2H), 2.52-2.41 (m, 1H), 2.31-2.13 (m, 2H), 2.03-1.86 (m, 1H), 1.12 (t, *J*=7.2 Hz, 3H).

**Embodiment 42:**

4-ethyl-6-(((3*R*,5*R*)-1-ethyl-5-fluoropiperidin-3-yl)amino)-3-(4-hydroxybenzo[b]thiophen-5-y 1)-1,2,4-triazin-5(4H)-ketone

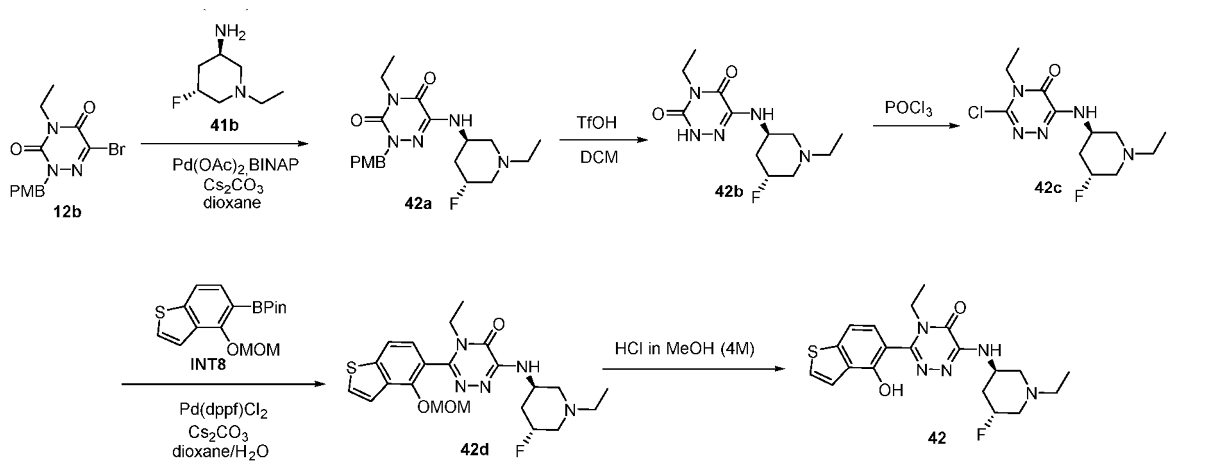


Step 1: the compound **12b** (300 mg, 0.88 mmol), **41b** (192 mg, 0.88 mmol), $Pd(OAc)_2$ (20.4 mg, 0.09 mmol), BINAP (56.04 mg, 0.09 mmol), and $Cs_2CO_3$ (1.15 g, 3.52 mmol) were added into the drying flask, with dioxane (8 mL) as a solvent, under nitrogen atmosphere, the resulting mixture was reacted overnight at 100°C , after the completion of the reaction was confirmed by TLC, the reaction mixture was filtered and concentrated under reduced pressure, the resulting residue was purified by column chromatography to obtain a compound **42a** (yellow oily liquid, 217 mg). MS/ESI $[M+H]^+$=406.2.

Step 2: the compound **42a** (217 mg, 0.54 mmol), DCM (5 mL) and TfOH (243.2 mg, 1.62 mmol) were added into the drying flask and reacted overnight at room temperature, after the completion of the reaction was confirmed by TLC, the reaction mixture was adjusted to about 8-9 with dry $NaHCO_3$ and a small amount of ammonia water, and the pH-adjusted reaction mixture was dried with anhydrous $Na_2SO_4$ and filtered, the filtrate was concentrated under reduced pressure, and the resulting residue was purified by column chromatography to obtain a compound **42b** (light yellow solid, 114 mg). MS/ESI$[M+H]^+$=286.2.

Step 3: the compound **42b** (114 mg, 0.4 mmol) and $POCl_3$ (8 mL) were added into the drying flask, and the resulting mixture was reacted overnight at 100°C, after the reaction was completed, the reaction mixture was concentrated under reduced pressure to remove excess $POCl_3$, and the resulting residue was purified by column chromatography to obtain a compound **42c** (light brown solid, 106 mg). MS/ESI$[M+H]^+$=304.1.

Step 4: the compound **42c** (106 mg, 0.35 mmol), **INT8** (112 mg, 0.35 mmol), $Pd(dppf)Cl_2$ (29.3 mg, 0.04 mmol), $Cs_2CO_3$ (456.4 mg, 1.4 mmol) and 1,4-dioxane/$H_2O$ (5 mL/1 mL) were added into the drying flask, and reacted overnight at 100°C. After the completion of the reaction was confirmed by TLC, the reaction solution was returned to room temperature and filtered, and the filtrate was concentrated under reduced pressure. The resulting residue was purified by column chromatography to obtain a compound **42d** (yellow oily liquid, 86 mg). MS/ESI$[M+H]^+$=462.2.

Step 5: the compound **42d** (86 mg, 0.19 mmol) and dioxane (2 mL) were added into the drying flask, followed by adding HCL in MeOH (4 M) (0.2 mL) dropwise, and the resulting mixture was reacted for 2 h at room temperature. The reaction mixture was concentrated under reduced pressure. The resulting residue was purified by column chromatography to obtain a compound **42** (light yellow solid, 7.8 mg). MS/ESI$[M+H]^+$=418.2. $^1H$ NMR (400 MHz, $CD_3OD$) $\delta$ 7.51 (d, J=5.5 Hz, 1H), 7.22 (d, J=5.5 Hz, 1H), 7.12-7.05 (m, 2H), 4.41-4.30 (m,1H), 3.94 -3.84 (m, 2H), 3.02-2.94 (m, 1H), 2.89-2.75 (m, 1H), 2.48-2.38 (m, 3H ), 2.19-2.02 (m, 3H), 1.99-1.76 (m, 2H), 1.02 (t, J=7.2 Hz, 3H), 0.96 (t, J=7.2 Hz, 3H).

## Embodiment 43:

4-ethyl-6-(((3*R*,5*R*)-5-fluoro-1-methylpiperidin-3-yl)amino)-3-(4-hydroxybenzo[b]thiophen-5 -yl)-1,2,4-triazin-5(4H)-ketone

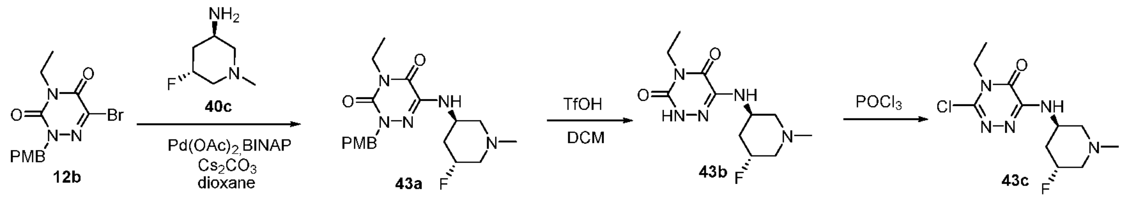

12b 40c 43a 43b 43c

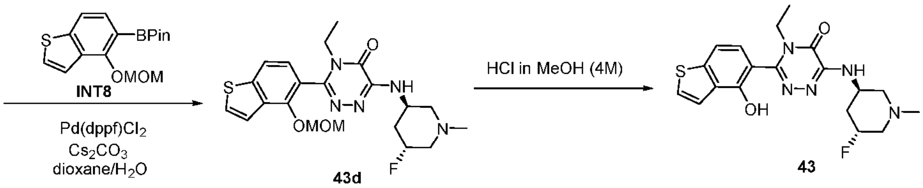

INT8 43d 43

4-ethyl-6-(((3R,5R)-5-fluoro-1-methylpiperidin-3-yl)amino)-3-(4-hydroxybenzo[b]thiop hen-5-yl)-1,2,4-triazin-5(4H)-ketone was prepared by referring to Embodiment 42. MS/ESI[M+H]$^+$=404.2. $^1$H NMR (400 MHz, $CD_3OD$) δ 7.51 (d, *J*=5.5 Hz, 1H), 7.26 (d, *J*=5.5 Hz, 1H), 7.15 (d, J=8.1 Hz, 1H), 7.08 (d, *J*=8.2 Hz, 1H), 4.43-4.31 (m, 1H), 3.89-3.85 (m, 2H), 2.99-2.93 (m, 1H), 2.83-2.75 (m, 1H), 2.41-2.28 (m, 1H), 2.24 (s, 3H), 2.16-2.03 (m, 3H), 1.96-1.78 (m, 2H), 0.97 (t, *J*=7.1 Hz, 3H).

**Embodiment 44:**

(*R*)-3-(4-(cyclopropylethynyl)-2-hydroxyphenyl)-4-methyl-6-((1-methylpiperidin-3-yl)amino) -1,2,4-triazin-5(4H)-ketone

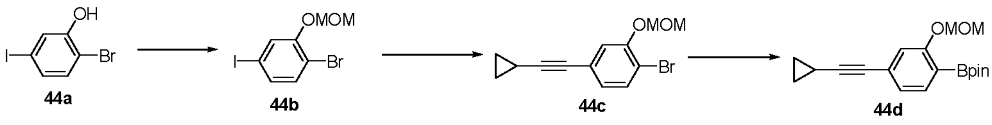
44a 44b 44c 44d

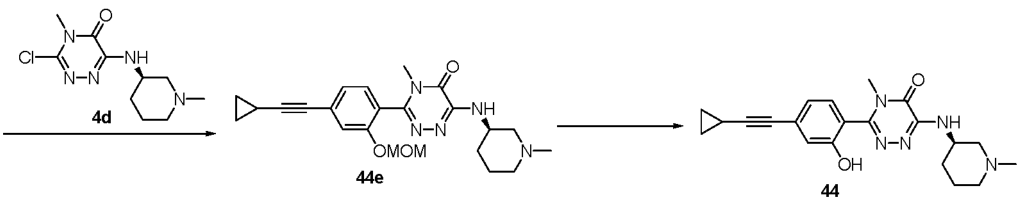
4d 44e 44

Step 1: 2-bromo-5-iodophenol **44a** (5.0 g, 16.72 mmol) and tetrahydrofuran (50 mL) were added into a reaction flask and cooled to 0°C, followed by adding NaH (1.0 g, 25.08 mmol, 60%) in batches, the resulting mixture was reacted for 0.5 h, followed by adding chloromethoxymethane (2.02 g, 25.08 mmol) and reacting for 1 h at room temperature. After the reaction was completed, a saturated aqueous ammonium chloride solution (50 mL) was added into the reaction mixture to quench the reaction, and then the quenched reaction mixture was extracted with ethyl acetate (50 mL), the aqueous phase was further extracted with ethyl acetate (20 mL), ethyl acetate layers were combined, directly mixed with silica gel, and purified by silica gel column chromatography (petroleum ether: ethyl acetate=1:0-19:1) to obtain a compound **44b** (5.7 g, 99.5%). MS/ESI [M+H]$^+$ = 342.8.

Step 2: the compound **44b** (2000 mg, 5.85 mmol), Pd($PPh_3$)$Cl_2$ (410 mg, 0.59 mmol), CuI (222 mg, 1.17 mmol), DIEA (2.3 g, 17.54 mmol), and tetrahydrofuran (50 mL) were added into the reaction flask, under nitrogen atmosphere, the resulting mixture was stirred for 0.5 h at room temperature, followed by adding ethynylcyclopropane (385 mg, 5.85 mmol) and reacting for 15 h at room temperature. After the reaction was completed, the reaction mixture was directly mixed with the silica gel, and the resulting mixture was purified by silica gel column chromatography (petroleum ether: ethyl acetate=1: 0-24: 1) to obtain a compound **44c** (1.7 g, crude product).

Step 3: the compound **44c** (1.4 g, 5.00 mmol) was added into the reaction flask, followed by adding tetrahydrofuran (50 mL) under nitrogen atmosphere. The resulting mixture was cooled to -78°C, followed by slowly adding n-butyl lithium (2.6 mL, 6.50 mmol, 0.5 M) dropwise and reacting for 1 h at a constant temperature. Upon the addition of a solution of 2-isopropoxy-4,4,5,5-tetramethyl-1,3,2-dioxaborolane (1.4 g, 7.50 mmol) in tetrahydrofuran (10 mL), the reaction mixture was reacted for 1 h at the constant temperature, followed by warming up to room temperature and reacting for 1 h.

After the reaction was completed, the saturated aqueous ammonium chloride solution (50 mL) was added into the reaction mixture to quench the reaction, the quenched reaction mixture was extracted with ethyl acetate (50 mL), and the aqueous phase was further extracted with ethyl acetate (50 mL), ethyl acetate layers were combined, directly mixed with silica gel, and purified by silica gel column chromatography (petroleum ether: ethyl acetate=1:0-10:1) to obtain a compound **44d** (1.2 g, 73.1%). $^1$H NMR (400 MHz, Chloroform-d) δ 7.58 (d, *J*=8.0 Hz, 1H), 7.26 (s, 1H), 7.02 (d, *J*=8.0 Hz, 1H), 5.17 (s, 2H), 3.50 (s, 3H), 1.47-1.41 (m, 1H), 1.34 (s, 12H), 0.90-0.79 (m, 4H).

(*R*)-3-(4-(cyclopropylethynyl)-2-hydroxyphenyl)-4-methyl-6-((1-methylpiperidin-3-yl)a mino)-1,2,4-triazin-5(4H)-ketone was prepared by referring to Embodiment 24. MS/ESI[M+H]$^+$=380.2. $^1$H NMR NMR (400 MHz, $CD_3OD$) δ 7.25 (d, *J*=7.8 Hz, 1H), 6.96 (d, *J*=7.8 Hz, 1H), 6.90 (s, 1H), 4.25 (s, 1H), 3.56-3.43 (m, 1H), 3.32 (s, 3H), 3.01-2.81 (m, 2H), 2.77 (s, 3H), 2.12-1.99 (m, 3H), 1.89-1.80 (m, 1H), 1.78-1.67 (m, 1H), 1.54-1.44 (m, 1H), 1.37-1.27 (m, 1H), 0.97-0.88 (m, 2H), 0.79-0.70 (m, 2H).

**Embodiment 45:**

(*R*)-3-(4-(cyclopropylethynyl)-2-hydroxyphenyl)-6-((1-ethylpiperidin-3-yl)amino)-4-methyl-1 ,2,4-triazin-5(4H)-ketone

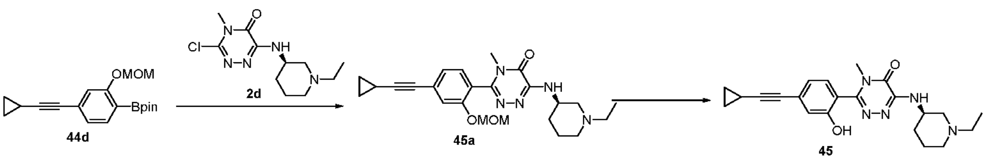


(*R*)-3-(4-(cyclopropylethynyl)-2-hydroxyphenyl)-6-((1-ethylpiperidin-3-yl)amino)-4-met hyl-1,2,4-triazin-5(4H)-ketone was prepared by referring to Embodiment 44. MS/ESI[M+H]$^+$=394.2. $^1$H NMR (400 MHz, $CD_3OD$) δ 7.25 (d, *J*=7.8 Hz, 1H), 6.95 (d, *J*=7.9 Hz, 1H), 6.90 (s, 1H), 4.25 (d, J=9.6 Hz, 1H), 3.55-3.42 (m, 1H), 3.33 (s, 3H), 3.05 (dd, *J*=14.1, 6.9 Hz, 2H), 2.93-2.76 (m, 2H), 2.10-2.01 (m, 3H), 1.93-1.70 (m, 2H), 1.53-1.43 (m, 1H), 1.32-1.26 (m, 4H), 0.95-0.86 (m, 2H), 0.80-0.69 (m, 2H).

**Embodiment 46:**

(*R*)-3-(4'-fluoro-3-hydroxy-[1,1'-biphenyl]-4-yl)-4-methyl-6-((1-methylpiperidin-3-yl)amino)-1,2,4-triazin-5(4H)-ketone

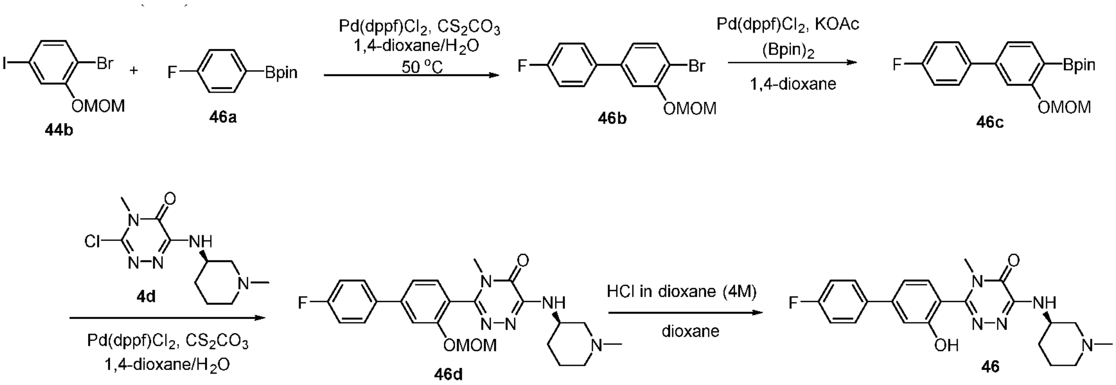


Step 1: the compound **44b** (400 mg, 1.17 mmol), **46a** (259.7 mg, 1.17 mmol), $Pd(dppf)Cl_2$ (87.8 mg, 0.12 mmol), $Cs_2CO_3$ (750.0 mg, 2.3 mmol) and 1,4-dioxane/$H_2O$ (10 mL/2 mL) were added into the drying flask, and reacted overnight at 100°C under nitrogen atmosphere. After the reaction was completed, the mixed solution was returned to room temperature and filtered, and the filtrate was concentrated under reduced pressure. The resulting residue was purified by column chromatography to obtain a compound **46b** (312 mg, 86%). $^1$H NMR (400 MHz,$CDCl_3$ ) δ 7.51 (d, *J*=8.2 Hz,2H), 7.47-7.40 (m, 1H), 7.24 (d, J=2.0 Hz, 1H), 7.09-6.96 (m, 3H), 5.23 (s, 2H), 3.48 (s, 3H).

Step 2: the compound **46b** (312 mg, 1.01 mmol), $(Bpin)_2$ (304.8 mg, 1.2 mmol), $Pd(dppf)Cl_2$ (73.2 mg, 0.1 mmol), KOAc (198.2 mg, 2.02 mmol), and 1,4-dioxane (10 mL) were added into the drying flask and stirred overnight at 100°C. After the reaction was completed, the mixed solution was returned to room temperature and extracted three times with

ethyl acetate upon the addition of water, the resulting organic phases were combined, dried with anhydrous sodium sulfate and filtrated, and the filtrate was concentrated under reduced pressure. The resulting residue was purified by column chromatography to obtain a compound **46c** (201 mg, 56%). MS/ESI[M+H]$^+$=359.2.

Step 3: the compound **46c** (201 mg, 0.56 mmol), **4d** (143.9 mg, 0.56 mmol), Pd(dppf)$Cl_2$ (43.9 mg, 0.06 mmol), $Cs_2CO_3$ (730.2 mg, 2.24 mmol) and 1,4-dioxane/$H_2O$ (6 mL/1 mL) were added into the drying flask, and reacted overnight at 100°C under nitrogen atmosphere. After the reaction was completed, the mixed solution was returned to room temperature and filtered, and the filtrate was concentrated under reduced pressure. The resulting residue was purified by column chromatography to obtain a compound **46b** (154 mg, 60%). MS/ESI[M+H]$^+$=454.2.

Step 4: the compound **46d** (154 mg, 0.34 mmol) and 1,4-dioxane (3 mL), and HCl (4 M, in dioxane) (0.4 mL) were added into the drying flask, and reacted for 3 h at room temperature, the reaction mixture was concentrated under reduced pressure to remove the excess solvent to obtain a compound **46** (16.5 mg, 12%). MS/ESI[M+H]$^+$=410.2. $^1$H NMR (400 MHz, $CD_3OD$) δ 7.65 (dd, *J*=8.8, 5.3 Hz, 2H), 7.40 (d, *J*=7.9 Hz, 1H), 7.25-7.12 (m, 4H), 4.18-4.10 (m, 1H), 3.37 (s, 3H), 3.05-2.89 (m, 1H), 2.70-2.54 (m, 1H), 2.31 (s, 3H), 2.30-2.14 (m, 2H), 1.98-1.87 (m, 1H), 1.85-1.76 (m, 1H), 1.74-1.62 (m, 1H), 1.61-1.50 (m, 1H), 1.36-1.28 (m,1H).

**Embodiment 47:**

(*R*)-3-(2-hydroxy-4-(pyrimidin-2-yl)phenyl)-4-methyl-6-((1-methylpiperidin-3-yl)amino)-1,2, 4-triazin-5(4H)-ketone

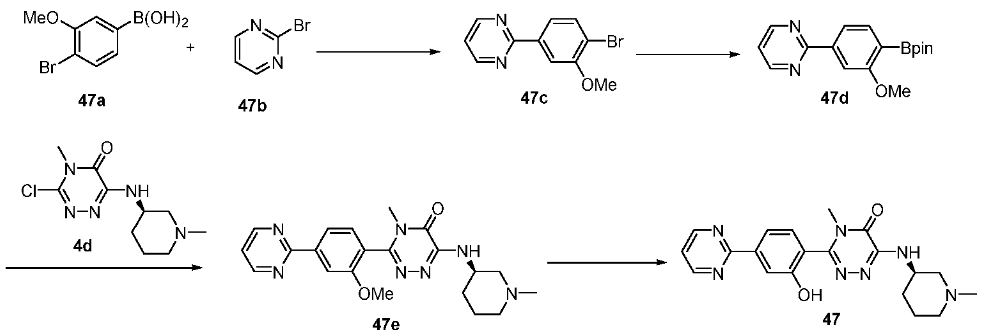

Step 1: the compound **47a** (460 mg, 2 mmol), **47b** (480 mg, 3 mmol), Pd($PPh_3$)$_4$ (230 mg, 0.2 mmol), sodium carbonate (640 mg, 6 mmol), dioxane (10 mL), and water (2 mL) were added into the reaction flask. Under nitrogen atmosphere as the replacement reaction system, the resulting mixture was reacted overnight at 100°C. The reaction mixture was filtered, and the filtrate was concentrated under reduced pressure to obtain a compound **47c** (310 mg), MS/ESI[M+H]$^+$=266.1.

Step 2: the compound **47c** (160 mg, 0.6 mmol), Pd(dppf)$Cl_2$ (44 mg, 0.06 mmol), potassium acetate (176 mg, 1.08 mmol), $B_2pin_2$ (305 mg, 1.2 mmol), and dioxane (6 mL) were added into the reaction flask. Under nitrogen atmosphere as the replacement reaction system, the resulting mixture was reacted overnight at 100°C. The reaction mixture was filtered, and the filtrate was concentrated under reduced pressure, mixed with silica gel, and purified by column chromatography to obtain a target compound **47d** (220 mg), MS/ESI [M+H]$^+$=313.1.

(*R*)-3-(2-hydroxy-4-(pyrimidin-2-yl)phenyl)-4-methyl-6-((1-methylpiperidin-3-yl)amino )-1,2,4-triazin-5(4H)-ketone was prepared by referring to Embodiment 4. MS/ESI[M+H]$^+$=394.4. $^1$H NMR (400 MHz, Chloroform-d) δ 8.82 (d, *J*=4.8 Hz, 2H), 8.13 (d, *J*=1.7 Hz, 1H), 8.01 (dd, *J*=8.2, 1.7 Hz, 1H), 7.42 (d, *J*=8.3 Hz, 1H), 7.23 (t, *J*=4.8 Hz, 1H), 6.42 (s, 1H), 4.34-4.12 (m, 1H), 3.58 (s, 3H), 2.59 (s, 2H), 2.50 (s, 2H), 2.28 (s, 3H), 1.76 (d, *J*=21.9 Hz, 4H), 1.67-1.55 (m, 1H).

**Embodiment 48:**

3-(4-hydroxybenzo[b]thiophen-5-yl)-4-methyl-6-(7-methyloctahydro-1H-pyrrolo[2,3-b]pyridi n-1-yl)-1,2,4-triazin-5(4H)-ketone

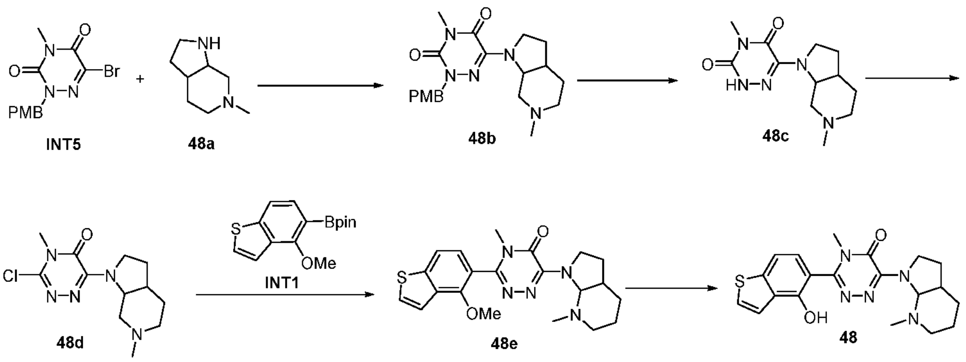


3-(4-hydroxybenzo[b]thiophen-5-yl)-4-methyl-6-(7-methyloctahydro-1H-pyrrolo[2,3-b] pyridin-1-yl)-1,2,4-triazin-5(4H)-ketone was prepared by referring to Embodiment 4. MS/ESI[M+H]$^+$=398.4. $^1$H NMR (400 MHz, $CD_3OD$) δ 7.53 (d, *J*=5.7 Hz, 1H), 7.43-7.37 (m, 2H), 7.21 (d, *J*=8.3 Hz,1 H), 4.21-4.13 (m, 1H), 3.31 (s, 3H), 2.95-2.87 (m, 2H), 2.67-2.42 (m, 2H), 2.55-2.39 (m, 3H), 2.18 (s, 3H), 1.97-1.78 (m, 3H), 1.53-1.34 (m, 2H).

**Embodiment 49:**

6-(((2R,3R)-1,2-dimethylpiperidin-3-yl)amino)-3-(4-hydroxybenzo[b]thiophen-5-yl)-4-methy 1-1,2,4-triazin-5(4H)-ketone

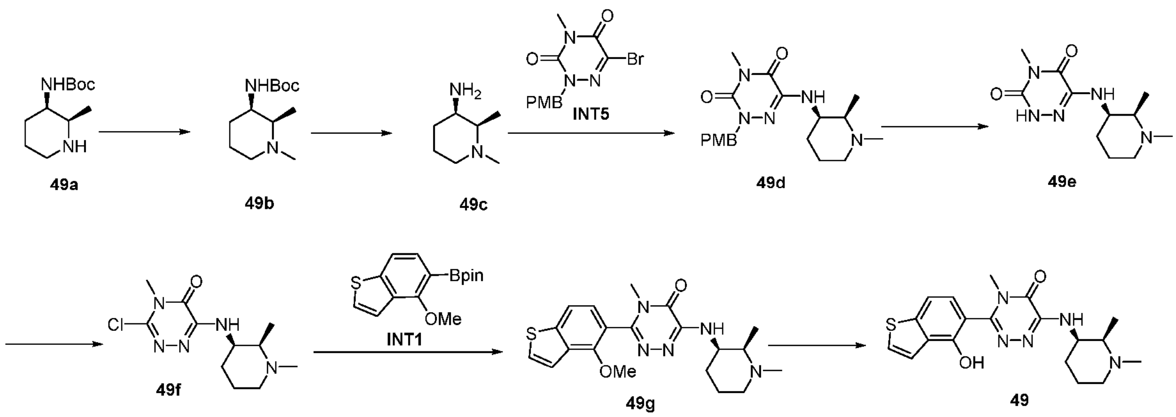


6-(((2R,3R)-1,2-dimethylpiperidin-3-yl)amino)-3-(4-hydroxybenzo[b]thiophen-5-yl)-4-methyl-1,2,4-triazin-5 (4H)-ketone was prepared by referring to Embodiment 40 to obtain a compound 49 (115 mg, 33.8%). $^1$H NMR (400 MHz, DMSO-$d_6$) δ 10.22 (br s, 1H), 7.72 (d, *J*=5.6 Hz, 1H), 7.69 (d, *J*=5.6 Hz, 1H), 7.59 (d, *J*=8.0 Hz, 1H), 7.29 (d, *J*=8.0 Hz, 1H), 6.39-6.31 (m, 1H), 4.07-3.97 (m, 1H), 3.20 (s, 3H), 2.87-2.74 (m, 1H), 2.44-2.31 (m, 1H), 2.21 (s, 3H), 2.16-2.02 (m, 1H), 1.99-1.90 (m, 1H), 1.74-1.58 (m, 1H), 1.55-1.39 (m, 2H), 1.04 (d, *J*=6.4 Hz, 3H). MS/ESI[M+H]$^+$=386.0.

**Embodiment 50:**

6-(((3R,6S)-1,6-dimethylpiperidin-3-yl)amino)-3-(4-hydroxybenzo[b]thiophen-5-yl)-4-methyl -1,2,4-triazin-5 (4H)-ketone

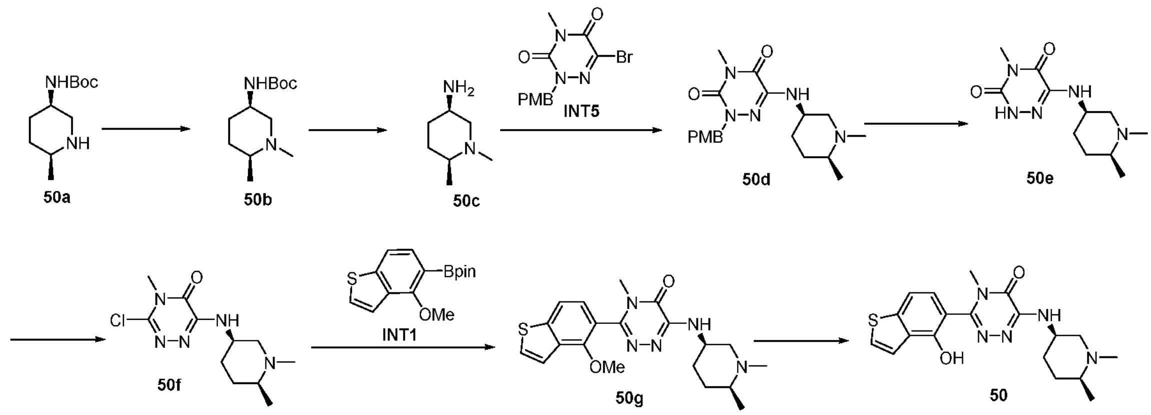
50a 50b 50c INT5 50d 50e 50f INT1 50g 50

6-(((3R,6S)-1,6-dimethylpiperidin-3-yl)amino)-3-(4-hydroxybenzo[b]thiophen-5-yl)-4-m ethyl-1,2,4-triazin-5(4H)-ketone was prepared by referring to Embodiment 40. MS/ESI[M+H]$^+$=386.2. $^1$H NMR (400 MHz, $CD_3OD$) δ 7.53 (d, *J*=5.7 Hz, 1H), 7.43-7.37 (m, 2H), 7.21 (d, *J*=8.3 Hz,1 H), 4.03 (m, 1H), 3.27 (s, 3H), 2.86-2.74 (m, 2H), 2.46-2.41 (m, 2H), 2.18 (s, 3H), 1.84-1.79 (m, 2H), 1.77-1.61 (m, 2H), 1.16 (s, 3H).

**Embodiment 51:**

3-(4-hydroxybenzo[b]thiophen-5-yl)-6-{[(3*R*)-1-methylpiperidin-3-yl]amino}-4-(2-methoxyet hyl)-4*H*,5*H*-1,2,4-triazin-5-ketone

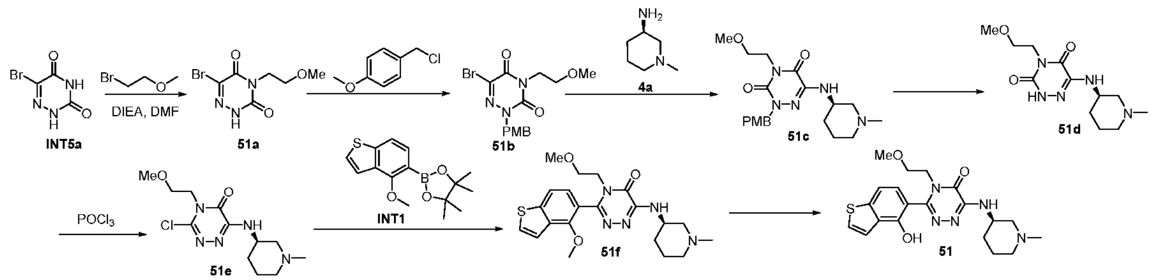

INT5a 51a 51b 4a 51c 51d 51e INT1 51f 51

3-(4-hydroxybenzo[b]thiophen-5-yl)-6-{[(3*R*)-1-methylpiperidin-3-yl]amino}-4-(2-meth oxyethyl)-4*H*,5*H*-1,2,4-triazin-5-ketone was prepared by referring to Embodiment 12 to obtain a compound **51** (24 mg, 44.4%). $^1$H NMR (400 MHz, Methanol-$d_4$) δ 7.61 (d, *J*=5.6 Hz, 1H), 7.56-7.51 (m, 2H), 7.29 (d, *J*=8.4 Hz, 1H), 4.23-4.14 (m, 1H), 4.08 (t, *J*=5.6 Hz, 2H), 3.44 (t, *J*=5.6 Hz, 2H), 3.11-3.01 (m, 4H), 2.76-2.66 (m, 1H), 2.45-2.34 (m, 5H), 2.02-1.81 (m, 2H), 1.77-1.56 (m, 2H). LC-MS:ESI[M+H]$^+$=416.0.

**Embodiment 52:**

3-(2-fluoro-4-hydroxybenzo[b]thiophen-5-yl)-6-{[(3R,5R)-5-fluoro-1-methylpiperidin-3-yl]a mino}-4-methyl-4H,5H-1,2,4-triazin-5-ketone

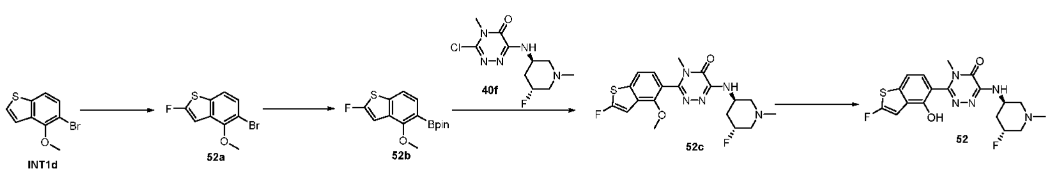
INT1d 52a 52b 40f 52c 52

Step 1: the compound **INT1d** (5.00 g, 20.6 mmol) was dissolved in tetrahydrofuran (25 mL), under nitrogen atmosphere, the resulting solution was cooled to -70°C, followed by slowing adding LDA (12.4 mL, 2.0 M, 24.7 mmol)

dropwise and then stirring for 1 h at a constant temperature. Then, a solution of N-fluorodibenzenesulfonamide (7.16 g, 22.7 mmol) in tetrahydrofuran (25 mL) was added into the resulting mixture dropwise,after the dropwise addition, the resulting mixture was reacted for 1 h at the constant temperature, and then the reaction mixture was warmed to room temperature and stirred overnight. After the reaction was completed, a saturated aqueous ammonium chloride solution (100 mL) was added into the reaction mixture to quench the reaction, the quenched mixture was extracted with ethyl acetate (30 mL × 3), dried with anhydrous sodium sulfate, and filtered, the filtrate was mixed with silica gel, concentrated under reduced pressure, and then purified by column chromatography (PE: EtOAc=1: 0) to obtain a compound **52a** (2.80 g, 10.7 mmol, yield: 52%). $^1H$ NMR (400 MHz, $CDCl_3$-*d*) δ 7.41 (d, *J*=8.4 Hz, 1H), 7.23 (d, *J*=6.4 Hz, 1H), 6.80 (d, *J*=2.4 Hz, 1H), 3.91 (s, 3H).

Step 2: the compound **52a** (2.80 g, 10.7 mmol), bis(pinacolato)diboron (3.25 g, 12.8 mmol), KOAc (2.10 g, 21.4 mmol), and Pd(dppf)$Cl_2$ (751 mg, 1.07 mmol) were added into anhydrous dioxane (30 mL), and the resulting mixture was reacted for 12 h at 100°C under nitrogen atmosphere. After the reaction was completed, the reaction mixture was filtered, the filtrate was concentrated under reduced pressure, and purified by column chromatography (PE: EtOAc=30: 1) to obtain a target compound **52b** (2.10 g, 6.81 mmol, colorless solid, yield: 64%), $^1H$ NMR (400 MHz, $CDCl_3$) δ 7.63 (d, *J*=8.0 Hz, 1H), 7.40 (d, *J*=8.4 Hz, 1H), 6.84 (d, *J*=2.4 Hz, 1H), 3.94 (s, 3H), 1.38 (s, 12H).

3-(2-fluoro-4-hydroxybenzo[b]thiophen-5-yl)-6-{[(3R,5R)-5-fluoro-1-methylpiperidin-3 -yl]amino}-4-methyl-4H,5H-1,2,4-triazin-5-ketone was prepared by referring to Embodiment 4. $^1H$ NMR (400 MHz, $CDCl_3$-*d*) δ 7.20-7.13 (m, 2H), 6.94 (d, *J*=2.4 Hz, 1H), 6.17 (d, *J*=8.0 Hz, 1H), 4.90-4.71 (m, 1H), 4.42 (s, 1H), 3.54 (s, 3H), 2.86-2.77 (m, 1H), 2.67-2.61 (m, 1H), 2.55-2.48 (m, 1H), 2.45-2.39 (m, 1H), 2.37 (s, 3H), 2.25-2.17 (m, 1H), 1.89-1.81 (m, 1H). LC-MS: ESI[M+H]$^+$=408.0.

**Embodiment 53:**

3-(4-cyclopropyl-2-hydroxyphenyl)-6-(((3R,5R)-1-ethyl-5-fluoropiperidin-3-yl)amino)-4-met hyl-1,2,4-triazin-5 (4H)-ketone

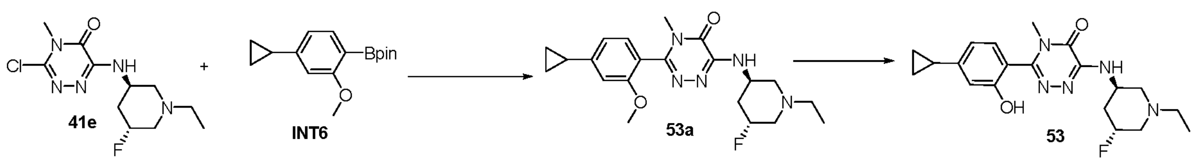


3-(4-cyclopropyl-2-hydroxyphenyl)-6-(((3R,5R)-1-ethyl-5-fluoropiperidin-3-yl)amino)-4 -methyl-1,2,4-triazin-5 (4H)-ketone was prepared by referring to Embodiment 11 to obtain a compound 53 (light yellow solid, 42.3 mg). $^1H$ NMR (400 MHz, $CD_3OD$) δ 7.41 (d, J=8.0 Hz, 1H), 6.85 (d, J=8.1 Hz, 1H), 6.79 (s, 1H), 4.65 (t, J=12.0 Hz, 1H), 3.91-3.72 (m, 3H), 3.49 (s, 3H), 3.37 (m, 4H), 3.19-3.03 (m, 1H), 2.57 (m, 1H), 1.99 (m, 1H), 1.41 (m, 4H), 1.11 (m, 2H), 0.80 (m, 2H).

**Embodiment 54:**

(*R*)-3-(4-hydroxybenzo[b]thiophen-5-yl)-4-isopropyl-6-((1-methylpiperidin-3-yl)amino)-1,2,4 -triazin-5(4H)-ketone

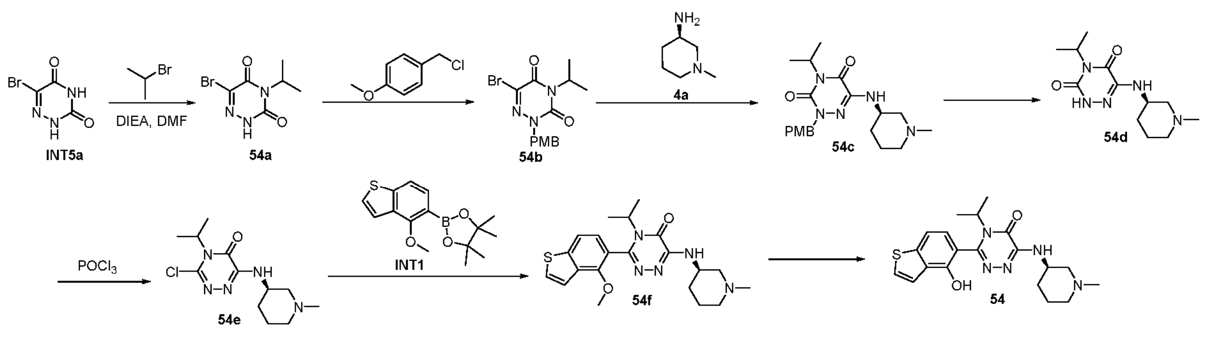


(*R*)-3-(4-hydroxybenzo[b]thiophen-5-yl)-4-isopropyl-6-((1-methylpiperidin-3-yl)amino)-1,2,4-triazin-5(4H)-ketone was prepared by referring to Embodiment 12 to obtain a compound 54 (11 mg, yield: 19.30%). LC-MS: ESI $[M+H]^+$=400.0. NMR (400 MHz, MeOD)δ 7.53 (d, *J*=5.6 Hz), 7.47 (dd, *J*=8.2, 7.2 Hz), 7.14 (d, *J*=8.3 Hz), 4.32 (dt, *J*=12.8, 6.4 Hz), 3.96-3.86 (m), 2.25 (s), 2.14-2.05 (m), 1.91 (s), 1.76 (dd, *J*=9.2, 4.4 Hz), 1.64-1.55 (m), 1.54-1.40 (m), 1.29 (d, *J*=6.4 Hz).

**Embodiment 55:**

(*R*)-6-((1-ethylpiperidin-3-yl)amino)-3-(4-hydroxybenzo[b]thiophen-5-yl)-4-(methyl-d3)-1,2, 4-triazin-5(4H)-ketone

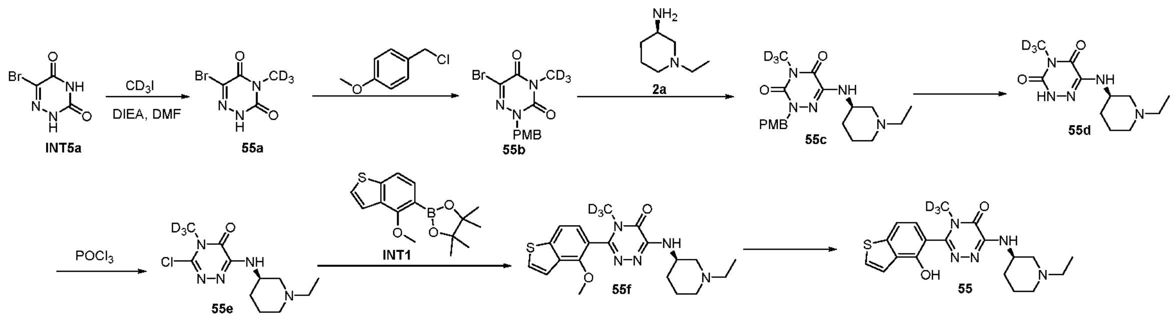


(*R*)-6-((1-ethylpiperidin-3-yl)amino)-3-(4-hydroxybenzo[b]thiophen-5-yl)-4-(methyl-d3) -1,2,4-triazin-5(4H)-ketone was prepared by referring to Embodiment 12 to obtain a target compound 55 (yellow solid, 37mg, yield: 14.34%). LC-MS: ESI[M+H]=389.0. $^1$H NMR (400 MHz, DMSO) δ 7.76 (q, *J*=5.6 Hz, 2H), 7.65 (d, *J*=8.3 Hz, 1H), 7.30 (d, *J*=8.3 Hz, 1H), 3.54 (dd, *J*=25.8, 10.7 Hz, 2H), 3.20 (d, *J*=7.0 Hz, 2H), 2.91-2.75 (m, 2H), 1.99 (d, *J*=10.6 Hz, 2H), 1.84-1.67 (m, 2H), 1.24 (t, *J*=7.2 Hz, 3H).

**Biological activity tests**

**1. Determination of inhibitory activity of NLRP3 inflammasome in human monocytes**

Reagents: THP-1 cells: Wuhan Pricella Biotechnology Co., Ltd., PMA: Sigma-Aldrich, RPMI culture medium: Hyclone, LPS: Sigma-Aldrich, Opti-MEM culture medium: Gibco, Nigericin: Invivogen, Human IL-1β ELISA assay kit: 4A Biotech, reference compound MCC950: MedChemExpress (MCE).

Experimental method: THP-1 cells were cultured in RPMI culture medium containing PMA (10 μM), and then inoculated in 48-well plates at a density of $2\times10^5$ cells/mL, the plates were incubated overnight in a 5% $CO_2$ incubator at 37°C. The next day, the above culture medium was replaced with Opti-MEM culture medium containing 1 μg/mL LPS. After 3 hours, the grown cells were simulated for 40 min with a drug, and then simulated for 40 min with Nigericin (10 μM), and a cell supernatant was harvested for ELISA analysis. A compound MCC950 was purchased from MCE, Ref-1 was synthesized according to a synthesis method of Embodiment 63 in Patent WO2021193897, Ref-2 was synthesized according to a synthesis method of Embodiment 1 in Patent WO2022230912, Ref-3 was synthesized according to a synthesis method of Embodiment 26 in Patent WO2022238347.

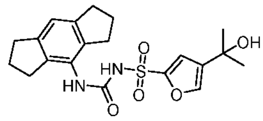
MCC950
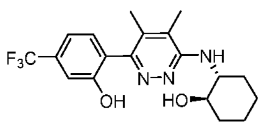
Ref-1
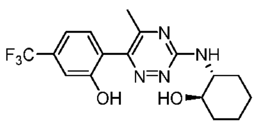
Ref-2
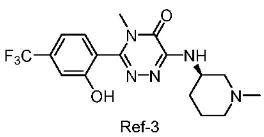
Ref-3

The experimental results are shown in Table 2.

Table 2 Inhibitory activity of NLRP3 inflammasome

| Compound number | $IC_{50}$(nM) | Compound number | $IC_{50}$(nM) |
|---|---|---|---|
| **1** | 4.24 | **2** | 4.86 |
| **3** | 2.11 | **4** | 4.21 |

(continued)

| Compound number | $IC_{50}$(nM) | Compound number | $IC_{50}$(nM) |
|---|---|---|---|
| **5** | 3.56 | **6** | 3.01 |
| **7** | 22.10 | **8** | 1.92 |
| **9** | 3.95 | **10** | 3.90 |
| **11** | 6.50 | **12** | 7.20 |
| **13** | 1.36 | **14** | 32.03 |
| **15** | 1.45 | **16** | 1.62 |
| **17** | 3.19 | **18** | 25.04 |
| **19** | 19.05 | **20** | 35.06 |
| **21** | 3.51 | **22** | 2.57 |
| **23** | 8.95 | **24** | 12.05 |
| **25** | 6.70 | **26** | 25.08 |
| **27** | 14.34 | **28** | 29.60 |
| **29** | 46.51 | **30** | 1.97 |
| **31** | 3.64 | **32** | 8.53 |
| **33** | 12.50 | **34** | 75.01 |
| **36** | 1.55 | **37** | 15.11 |
| **38** | 257.01 | **39** | 30.01 |
| **40** | 2.97 | **41** | 2.02 |
| **42** | 4.76 | **43** | 5.66 |
| **44** | 80.01 | **45** | 345.05 |
| **46** | 25.51 | **47** | 450.08 |
| **48** | 18.53 | **49** | 8.12 |
| **50** | 350.45 | **51** | 180.05 |
| **52** | 5.06 | **53** | 18.70 |
| **55** | 3.50 | MCC950 | 120 |
| Ref-1 | 12.22 | Ref-2 | 14.72 |
| Ref-3 | 6.49 | | |
| Conclusion: the compounds of the present invention have good inhibitory activity against NLRP3 inflammasome, and preferred compounds have better inhibitory effects on NLRP3 inflammasome than MCC950, Ref-1, Ref-2, and Ref-3. | | | |

**2. Determination of inhibitory activity of compounds on IL-1β in human PBMC cells**

Human peripheral blood mononuclear cells (PBMCs) were taken and cultured overnight in RPMI 1640 culture medium containing 10% FBS and antibiotics before stimulation. On the second day, the culture medium was replaced with a serum-reduced culture medium, the grown cells were stimulated for 3 h with LPS at a working concentration of 1 μg/mL, stimulated for 40 min with a drug, and then stimulated for 40 min with Nigericin at a concentration of 10 μM, the cell supernatant was harvested, and the yield of IL-1β was detected by ELISA.
The experimental results are shown in Table 3:

Table 3 Inhibitory effect of compounds on IL-1β in PBMC cells

| Compound number | $IC_{50}$(nM) | Compound number | $IC_{50}$(nM) |
|---|---|---|---|
| **3** | 1.23 | **4** | 1.42 |

(continued)

| Compound number | $IC_{50}$(nM) | Compound number | $IC_{50}$(nM) |
|---|---|---|---|
| MCC950 | 7.81 | | |
| Conclusion: the compounds of the present invention have good inhibitory activity against IL-1β in human PBMC cells, and preferred compounds **3, 4** and others have better inhibitory activity $IC_{50}$ on IL-1β in human PBMC cells than MCC950. | | | |

### 3. Inhibition experiment of compounds on hERG potassium ion channel

Cell culture and treatment: CHO cells stably expressing hERG were cultured in cell culture flasks and incubated in a 5% $CO_2$ incubator at 37°C. When a cell density reached 60%-80%, the cell culture medium was aspirated, washed once with PBS, and then digested with Detachin. After digestion, the grown cells were neutralized in the cell culture medium, and then centrifuged, the supernatant was aspirated, the cell culture medium was added again to resuspend the grown cells, and the cell density was adjusted to 2-5×$10^6$ cells/mL for later use.

Compound preparation: the compound stock solution was diluted in 100% DMSO, that is, 10 μL compound stock solution was serially diluted in 20 μL DMSO by 3 folds to obtain six concentrations. Six concentrations of the compound (4 μL for each concentration) were diluted in 396 μL extracellular fluid by 100 folds to obtain six intermediate concentrations. Six intermediate concentrations of the compound (80 μL for each concentration) were diluted in 320 μL extracellular fluid by 5 folds to obtain final concentrations to be tested. A highest test concentration is 40 μM, with six subsequent concentrations of 40 μM, 13.33 μM, 4.44 μM, 1.48 μM, 0.49 μM, and 0.16 μM in total. DMSO in a final test concentration was not more than 0.2%, and DMSO at this concentration had no effect on the hERG potassium channel. The entire dilution process for compound preparation was performed using Bravo instrument.

Electrophysiological recording process: the processes of single-celled high-impedance sealing and whole-cell mode formation were all automatically completed by Qpatch instrument, under the whole-cell recording mode, the cells were clamped at -80 mV, before 5-second + 40 mV depolarization stimulus, the cells were given 50-millisecond - 50 mV pre-voltage, followed by repolarization to -50 mV for 5 seconds, and then returning to -80 mV. The voltage stimulus was given every 15 seconds, after 2 minutes of recording, extracellular fluid was recorded for 5 min, and then a drug was administrated, started from a lowest test concentration, each test concentration of the compound was given for 2.5 minutes, after all concentrations were given consecutively, a positive control compound Cisapride (3 μM) was given. At least 3 cells were tested for each concentration (n≥3).

Data processing: data analysis and processing were performed using GraphPad Prism 5.0 and Excel software. $IC_{50}$ values of the compounds were calculated by fitting with the following equation using GraphPad Prism 5 software:

$$Y=Bottom + (Top-Bottom)/(1+10^{\wedge}((LogIC50-X)*HillSlope))$$ ;

where X is the Log value of the test sample concentration, Y is an inhibition percentage at a corresponding concentration, and Bottom and Top are the minimum and maximum inhibition percentages, respectively.

The experimental results are shown in Table 4:

Table 4 Results of inhibition of compounds on hERG potassium ion channel

| Compound | Structure | 20 uM Inhibition ratio | Compound | Structure | 20 uM Inhibition ratio |
|---|---|---|---|---|---|
| Ref- 1 | 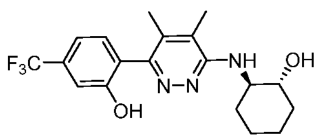 | 52.40% | **3** | 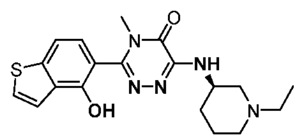 | 38.23% |
| **4** | 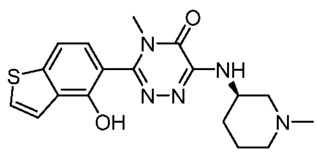 | 14.87% | **5** | 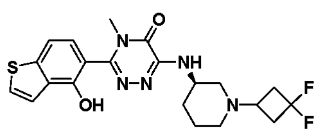 | 19.97% |

(continued)

| Compound | Structure | 20 uM Inhibition ratio | Compound | Structure | 20 uM Inhibition ratio |
|---|---|---|---|---|---|
| **8** | 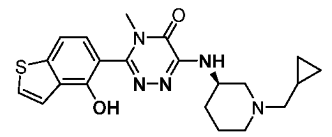 | 69.48% | **9** | 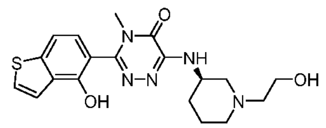 | 8.83% |
| **12** | 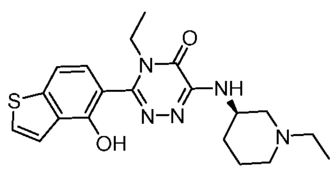 | 52.99% | **16** | 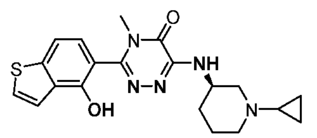 | 35.63% |
| **17** | 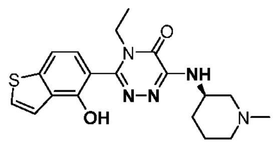 | 22.83% | **24** | 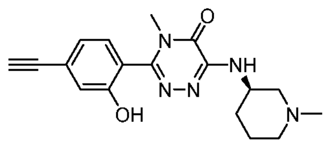 | 6.83% |
| **30** | 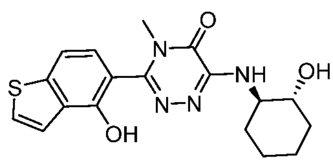 | 1.70% | **40** | 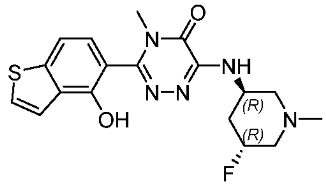 | 3.48% |
| **41** | 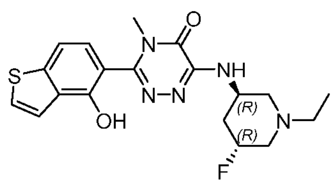 | 13.31% | **42** | 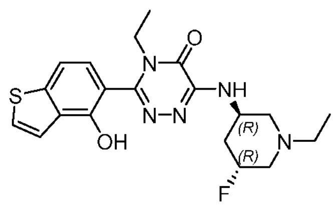 | 14.98% |
| **43** | 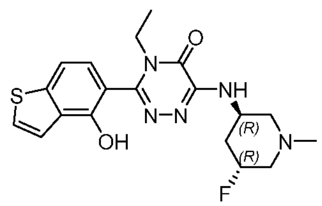 | 14.23% | **54** | 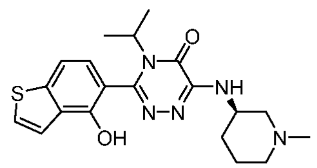 | 7.77% |
| Conclusion: compounds **3, 4, 5, 9, 16, 17, 24, 30, 40, 41, 42, 43, 54** and others have weak inhibitory effects on the hERG potassium ion channel, having weaker inhibitory effect than the reference compound Ref-1. | | | | | |

**4. Pharmacokinetic evaluation of compounds in Balb/c mice**

Experimental purpose: to understand pharmacokinetic properties of the compounds.

Experimental basis: Technical Guidelines for Nonclinical Pharmacokinetic Studies of Chemical Drugs, 2014.

Experimental protocol: the pharmacokinetic properties of the compounds were investigated via intravenous administration and intragastric administration in Balb/c mice.

Sample preparation: the compound was weighed and dissolved in DMSO, followed by adding a sodium chloride solution for injection, to prepare a compound solution for administration.

Sampling: 6 Balb/c male mice (Chengdu Dossy Experimental Animals Co., Ltd., license number: SCXK (Chuan) 2020-030) were selected, where three mice were subjected to intravenous administration (IV) and other three mice were subjected for intragastric administration (PO), approximately 0.05 mL blood was respectively harvested at 5min, 15 min, 30 min, 1 h, 2 h, 4 h, 6 h, 8 h, 10 h, 24 h and 48 h after administration, the harvested blood was centrifuged at 3500 rpm for 15

min, and the supernatant plasma was harvested and stored at -40°C until determination. The blood drug concentration was quantitatively analyzed by LC-MS/MS, and pharmacokinetic parameters were calculated, such as peak concentration (Cmax), area under the concentration-time curve ($AUC_{(0-t)}$), half-life ($T_{1/2}$), clearance (CL), volume of distribution at steady state (Vdss), and bioavailability (F).

The results of pharmacokinetic evaluation are shown in Table 5 below:

Table 5 Pharmacokinetic test results of compounds in Balb/c mice

| Compound | Dose, administration route | $C_{max}$ ug/L | $AUC_{(0-t)}$ ug/L*h | $T_{1/2}$ h | CL L/h/kg | Vdss L/kg | F % |
|---|---|---|---|---|---|---|---|
| Ref- 2 | 5 mg/kg, PO | 246 | 270 | 2.6 | 18.59 | 67.02 | - |
| Ref- 3 | 5 mg/kg, PO | 1136 | 1822 | 0.87 | 2.01 | 1.50 | - |
| **2** | 5 mg/kg, PO | 2694 | 15214 | 2.82 | 0.33 | 1.34 | - |
| **3** | 5 mg/kg, IV | 1989 | 8305 | 5.4 | 0.58 | 4.51 | 81.05 |
| | 5 mg/kg, PO | 1008 | 6730 | 4.9 | 0.72 | 4.34 | |
| **4** | 5 mg/kg, PO | 4613 | 16881 | 3.00 | 0.30 | 1.28 | - |
| **6** | 5 mg/kg, PO | 1335 | 8298 | 3.18 | 0.61 | 2.75 | - |
| **9** | 5 mg/kg, PO | 2277 | 4400 | 0.85 | 1.14 | 1.40 | - |
| **12** | 5 mg/kg, PO | 2735 | 12832 | 3.08 | 0.40 | 1.80 | |
| **13** | 5 mg/kg, PO | 11356 | 98703 | 5.47 | 0.05 | 0.39 | - |
| **17** | 5 mg/kg, PO | 1847 | 17765 | 4.20 | 0.28 | 1.70 | - |
| **30** | 5 mg/kg, PO | 9807 | 4383 | 0.19 | 1.44 | 0.39 | - |
| **36** | 5 mg/kg, PO | 1510 | 3318 | 1.24 | 1.47 | 2.59 | - |
| **40** | 5 mg/kg, PO | 15039 | 40421 | 2.86 | 0.12 | 0.42 | - |
| **42** | 5 mg/kg, PO | 2705 | 2496 | 1.61 | 1.99 | 4.57 | - |
| **43** | 5 mg/kg, PO | 5068 | 15692 | 2.25 | 0.32 | 1.03 | - |
| **51** | 5 mg/kg, PO | 2940 | 9784 | 2.39 | 0.51 | 1.78 | - |
| **52** | 5 mg/kg, PO | 8574 | 21440 | 3.16 | 0.23 | 1.04 | - |
| **53** | 5 mg/kg, PO | 1421 | 1405 | 3.85 | 3.53 | 19.69 | - |
| **54** | 5 mg/kg, PO | 1553 | 7468 | 4.25 | 0.47 | 2.84 | - |
| **55** | 5 mg/kg, PO | 1706 | 7454 | 2.91 | 0.67 | 2.82 | - |
| Conclusion: the compounds of the present invention have good pharmacokinetic properties in Balb/c mice, including good oral bioavailability, exposure, half-life, and clearance, after oral administration, compounds **2, 3, 4, 6, 9, 12, 13, 17, 30, 36, 40, 41, 43, 51, 52, 53, 54, 55** and others have superior PK parameters such as Cmax, $AUC_{(0-t)}$, and $T_{1/2}$ to the reference compounds Ref-2 and Ref-3. | | | | | | | |

### 5. Evaluation of LPS-induced mouse model

Experimental method: Balb/c mice at 7-8 weeks were orally administered with 25 mg/kg or 50 mg/kg of the compound or a vehicle control (sterile 0.9% NaCl solution), after 1 hour, the mice were intraperitoneally injected with 10 mg/kg LPS (Sigma, L2880). The survival status of mice was observed every 12 h for 72 h, and the 72-hour survival rate of mice was obtained.

The experimental results are shown in Table 6:

Table 6 Survival rate of LPS-induced mice with compounds

| Group description | 72h Survival rate | Cage separation | 0h | 12h | 24h | 36h | 48h | 72h |
|---|---|---|---|---|---|---|---|---|
| LPS | **20%** | First cage (5 mice) | 5 | 5 | 5 | 4 | 2 | 2 |
| | | Second cage (5 mice) | 5 | 5 | 4 | 2 | 1 | 0 |

(continued)

| Group description | 72h Survival rate | Cage separation | 0h | 12h | 24h | 36h | 48h | 72h |
|---|---|---|---|---|---|---|---|---|
| MCC950 25 mg/kg | **60%** | First cage (5 mice) | 5 | 5 | 5 | 4 | 4 | 4 |
| | | Second cage (5 mice) | 5 | 5 | 4 | 4 | 2 | 2 |
| Ref-2 25 mg/kg | **50%** | First cage (5 mice) | 5 | 5 | 5 | 4 | 2 | 2 |
| | | Second cage (5 mice) | 5 | 5 | 4 | 4 | 3 | 3 |
| Compound **3** 25 mg/kg | **90%** | First cage (5 mice) | 5 | 5 | 5 | 5 | 4 | 4 |
| | | Second cage (5 mice) | 5 | 5 | 5 | 5 | 5 | 5 |
| Compound **3** 50 mg/kg | **92%** | First cage (6 mice) | 6 | 6 | 6 | 6 | 6 | 6 |
| | | Second cage (6 mice) | 6 | 6 | 6 | 5 | 5 | 5 |
| Compound **4** 25 mg/kg | **80%** | First cage (5 mice) | 5 | 5 | 5 | 5 | 5 | 5 |
| | | Second cage (5 mice) | 5 | 5 | 5 | 4 | 4 | 3 |
| Compound **4** 50 mg/kg | **100%** | First cage (5 mice) | 5 | 5 | 5 | 5 | 5 | 5 |
| | | Second cage (5 mice) | 5 | 5 | 5 | 5 | 5 | 5 |
| Compound **9** 25 mg/kg | **70%** | First cage (5 mice) | 5 | 5 | 5 | 5 | 5 | 3 |
| | | Second cage (5 mice) | 5 | 5 | 5 | 5 | 5 | 4 |
| Compound **40** 25 mg/kg | **90%** | First cage (5 mice) | 5 | 5 | 5 | 5 | 5 | 5 |
| | | Second cage (5 mice) | 5 | 5 | 5 | 5 | 4 | 4 |
| Compound **41** 25 mg/kg | **100%** | First cage (5 mice) | 5 | 5 | 5 | 5 | 5 | 5 |
| | | Second cage (5 mice) | 5 | 5 | 5 | 5 | 5 | 5 |
| Conclusion: the compounds of the present invention can increase the survival rate of LPS-induced mice, especially at a same dose, compounds **3, 4, 9, 40, 41** and others have higher survival rates of LPS-induced mice than the reference compounds MCC950 and Ref-2. | | | | | | | | |

**6. Evaluation of in vivo brain-to-plasma ratio of compound**

Experimental purpose: to obtain the brain-to-plasma ratio of the compound

Experimental protocol: the brain-to-plasma ratio of the compound was investigated by monitoring the content of the compound in mouse brain and plasma.

Experimental procedure: the compound was weighed, followed by adding a small amount of DMSO and adding a sodium chloride solution for injection, to prepare a 10 mg · $mL^{-1}$ compound solution for administration. Male mice were orally administered at 10 mg · $kg^{-1}$, and whole plasma and whole brain tissue were sampled at 1 h and 6 h after administration (n=3). Whole plasma was centrifuged at 3500 rpm for 15 min, and the supernatant plasma was harvested. A centrifuge tube was weighed as M1, the centrifuge tube containing the whole brain tissue was weighed as M2, the centrifuge tube containing water for homogenization was weighed as M3, and the centrifuge tube after the removal of 30 μL homogenate was weighed as M4. 30 μL plasma and 30 μL brain homogenate were sampled and added into the centrifuge tube, followed by adding 120 μL acetonitrile containing 20 ng · $mL^{-1}$ internal standard SAHA to each sample for precipitate and vortex for 30 seconds, and the resulting mixture was centrifuged at 13,000 rpm for 15 min, and the supernatant was taken and added into a sample vial for determination.

Standard curve range: 10-10,000 ng·$mL^{-1}$.

$$\text{Content of drug in brain} = \text{measured value} \times 0.03 \times (M3\text{-}M1) / [(M2\text{-}M1) \times (M3\text{-}M4)].$$

Brain-to-plasma ratios of mice after compound administration are shown in Table 7 below:

Table 7 Test results of brain-to-plasma ratios in mice after compound administration

| Compound | 1 h | | | 6 h | | |
|---|---|---|---|---|---|---|
| | Brain (mg/g) | Plasma (ng/mL) | Brain-to-plasma ratio | Brain (mg/g) | Plasma (ng/mL) | Brain-to-plasma ratio |
| **3** | 761 | 2082 | 0.36 | 104 | 312 | 0.33 |
| **4** | 1779 | 3344 | 0.53 | 335 | 1369 | 0.25 |
| **9** | 66 | 1830 | 0.04 | 20 | 46 | 0.46 |
| **12** | 498 | 3840 | 0.13 | 215 | 1699 | 0.13 |
| **17** | 745 | 5243 | 0.14 | 290 | 1872 | 0.16 |
| **40** | 1346 | 7238 | 0.19 | 199 | 1464 | 0.14 |
| Ref- 1 | 56 | 2329 | 0.02 | 24 | 523 | 0.06 |
| Ref- 2 | 168 | 264 | 0.63 | 1.53 | 8.27 | 0.17 |
| Conclusion: some compounds of the present invention have good brain penetration potential, and particularly, compounds **3, 4, 12, 17, 40** and others have significantly superior brain-to-plasma ratios to the reference compounds Ref-1 and Ref-2. | | | | | | |

**7. Evaluation of in vivo distribution of cerebrospinal fluid and plasma after compound administration**

Experimental procedure: the compound was weighed, followed by adding a small amount of DMSO and then adding a sodium chloride solution for injection, to prepare a 5 mg·mL$^{-1}$ compound solution for administration. Large male mice were intravenously administered at 5 mg · kg$^{-1}$, and cerebrospinal fluid and whole plasma were respectively sampled at 0.25 h and 2 h after administration (n=1). Whole plasma was centrifuged at 3500 rpm for 15 min, and the supernatant plasma was harvested. 10 $\mu$L plasma and 10 $\mu$L cerebrospinal fluid were sampled and added into the centrifuge tube, followed by adding 40 $\mu$L acetonitrile containing 20 ng · mL$^{-1}$ internal standard SAHA to each sample for precipitate and vortex for 30 seconds, and the resulting mixture was centrifuged at 13,000 rpm for 15 min, and the supernatant was taken and added into a sample vial for determination. Standard curve range: 1-1000 ng·mL$^{-1}$.

Table 8 Test results of distribution of cerebrospinal fluid and plasma in mice after compound administration

| Compound | 0.25h | | 2h | |
|---|---|---|---|---|
| | Cerebrospinal fluid (ng/g) | Plasma (ng/mL) | Cerebrospinal fluid (ng/g) | Plasma (ng/mL) |
| **3** | 79 | 781 | 15 | 185 |
| **17** | 47 | 713 | 25 | 590 |
| **40** | 191 | 5028 | 0.51 | 826 |
| **41** | 67 | 668 | 1 | 38 |
| **42** | 35 | 965 | 1 | 46 |
| **43** | 151 | 6797 | 40 | 1911 |
| Conclusion: some compounds of the present invention have good brain penetration potential, and particularly, compounds **40, 43** and others have high distribution concentrations in cerebrospinal fluid. | | | | |

**8. Evaluation of affinity of compound for human NLRP3 protein**

Experimental purpose: protein was immobilized on a CM5 chip as a solid phase, and small molecules were diluted in a concentration gradient and then injected for interaction with the protein, so as to obtain fitted affinity values.

Instrument models and specific consumables: Biacore T200 Cytiva (Sweden), consumables: Series S Sensor Chip CM5 Cytiva (Sweden) BR100012 LOT 10344853, Amine Coupling Kit Cytiva (Sweden) BR100050 LOT 35063.

Reagents: Buffer: PBS 10X (1.37 M NaCl, 26.8 mM KCl, 81 mM $Na_2HPO_4$, 17.6 mM $KH_2PO_4$, pH 7.2-7.4) (Sangon Biotech, Lot #J806FC0551), Tween20 (Sigma) (Cat. No. P9416), DMSO (Sigma) (Cat. No. 67-68-5, Lot #WXBF-1310V).

Experimental procedure:

Protein immobilization: Chip selection: CM5 experimental procedure: Run → Manual Run: Manual Run was selected for controlled immobilization. The protein was immobilized under a specific acidic environment (tests were conducted at pH values of 4.0, 4.5, and 5.0), and a ligand protein was diluted to 10 ug/mL with 10 mM sodium acetate having different pH values, in a manual mode, samples were sequentially injected in the pH environment having strongest signal response. Protein immobilization parameter settings: concentration 30-50 ug/mL, pH environment 4.0, flow rate 10 μL/min, temperature 25°C. Solutions required for immobilization: 100 μL NHS, 100 μL EDC, 150 μL Ethanolamine (NHS and EDC are chip activation reagents, to activate dextran groups on the chip surface, and Ethanolamine is a blocking reagent used to block groups that are not bound to the protein) instruments automatically mixed EDC and NHS to activate the chip surface. After protein immobilization, the reference channel was blocked (with EDC, NHS, and Ethanolamine).

Injection of small molecules: setting of experimental procedure: ethod-LMW Kinetics, injection time setting: 120s contact, 300s dissociation time, all samples and buffers were filtered through a 0.22μm filter membrane before the experiment.

NLRP3 protein sequence SEQ ID NO:1:

MKMASTRCKLARYLEDLEDVDLKKFKMHLEDYPPQKGCIPLPRGQTEKADHVD
LATLMIDFNGEEKAWAMAVWIFAAINRRDLYEKAKRDEPKWGSDNARVSNPTVICQE
DSIEEEWMGLLEYLSRISICKMKKDYRKKYRKYVRSRFQCIEDRNARLGESVSLNKR
YTRLRLIKEHRSQQEREQELLAIGKTKTCESPVSPIKMELLFDPDDEHSEPVHTVVFQG
AAGIGKTILARKMMLDWASGTLYQDRFDYLFYIHCREVSLVTQRSLGDLIMSCCPDP
NPPIHKIVRKPSRILFLMDGFDELQGAFDEHIGPLCTDWQKAERGDILLSSLIRKKLLPE

ASLLITTRPVALEKLQHLLDHPRHVEILGFSEAKRKEYFFKYFSDEAQARAAFSLIQEN
EVLFTMCFIPLVCWIVCTGLKQQMESGKSLAQTSKTTTAVYVFFLSSLLQPRGGSQEH
GLCAHLWGLCSLAADGIWNQKILFEESDLRNHGLQKADVSAFLRMNLFQKEVDCEK
FYSFIHMTFQEFFAAMYYLLEEEKEGRTNVPGSRLKLPSRDVTVLLENYGKFEKGYLI
FVVRFLFGLVNQERTSYLEKKLSCKISQQIRLELLKWIEVKAKAKKLQIQPSQLELFYC
LYEMQEEDFVQRAMDYFPKIEINLSTRMDHMVSSFCIENCHRVESLSLGFLHNMPKEE
EEEKEGRHLDMVQCVLPSSSHAACSHGLVNSHLTSSFCRGLFSVLSTSQSLTELDLSD
NSLGDPGMRVLCETLQHPGCNIRRLWLGRCGLSHECCFDISLVLSSNQKLVELDLSDN
ALGDFGIRLLCVGLKHLLCNLKKLWLVSCCLTSACCQDLASVLSTSHSLTRLYVGENA
LGDSGVAILCEKAKNPQCNLQKLGLVNSGLTSVCCSALSSVLSTNQNLTHLYLRGNTL
GDKGIKLLCEGLLHPDCKLQVLELDNCNLTSHCCWDLSTLLTSSQSLRKLSLGNNDL
GDLGVMMFCEVLKQQSCLLQNLGLSEMYFNYETKSALETLQEEKPELTVVFEPSW.

Experimental result shows that the affinity Kd value of compound **40** for human NLRP3 protein is 62.13 nM.

## Claims

1. A compound of formula I or a pharmaceutically acceptable form thereof, wherein a structure of the formula I is as follows:

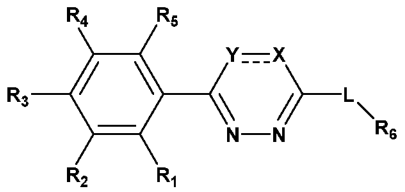

式 I

where:

Y---X represents a single bond, Y is selected from $NR_{7a}$, and X is selected from C(=O);

$R_1$ is selected from hydrogen, deuterium, halogen, -OH, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, and 3- to 6-membered cycloalkyl; and in $R_1$, the substituents are selected from deuterium, halogen, -OH, $-NH_2$, or -CN;

$R_2$ and $R_4$ are each independently selected from hydrogen, deuterium, halogen, -OH, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, and 3- to 6-membered cycloalkyl; and in $R_2$ and $R_4$, the substituents are selected from deuterium, halogen, -OH, $-NH_2$, or -CN;

$R_3$ is selected from hydrogen, deuterium, halogen, -OH, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, 3- to 6-membered cycloalkyl, 5- and 6-membered heterocycloalkyl, phenyl, and 5- and 6-membered heteroaryl; in $R_3$, the substituents are selected from deuterium, halogen, -OH, $-NH_2$, -CN, or 3- to 6-membered cycloalkyl; and in $R_3$, 5- and 6-membered heterocycloalkyl and 5- and 6-membered heteroaryl contain 1-3 heteroatoms selected from at least one of N, S, and O;

$R_5$ is selected from hydrogen, deuterium, halogen, $-NH_2$, -CN, or the following groups optionally substituted with 0-6 substituents: $C_{1-6}$ alkyl, -O-$C_{1-6}$ alkyl, -S-$C_{1-6}$ alkyl, -NHC(=O)-$C_{1-6}$ alkyl, -(C=O)NH-$C_{1-6}$ alkyl, $C_{2-6}$ alkenyl, $C_{2-6}$ alkynyl, and 3- to 6-membered cycloalkyl; and in $R_5$, the substituents are selected from deuterium, halogen, -OH, $-NH_2$ or -CN;

or, $R_2$ and $R_3$, $R_3$ and $R_4$, or $R_4$ and $R_5$, together with atoms to which these groups are bound, form a 5- and 6-membered alkane ring, a benzene ring, a 5- and 6-membered alkane heterocyclic ring, or a 5- and 6-membered heteroaromatic ring that is substituted with 0-6 substituents, wherein the substituents are selected from deuterium, halogen, -OH, $-NH_2$, -CN, an oxo group, $C_{1-6}$ alkyl, $C_{1-6}$ fluoroalkyl, $C_{1-6}$ deuterated alkyl, -O-$C_{1-6}$ alkyl, -O-$C_{1-6}$ fluoroalkyl, -O-$C_{1-6}$ deuterated alkyl, $C_{3-6}$ cycloalkyl, $C_{3-6}$ fluorocycloalkyl, or 3- to 6-membered cycloalkyl formed by two of the substituents bound to a same carbon atom; when $R_2$ and $R_3$, $R_3$ and $R_4$, or $R_4$ and $R_5$, together with the atoms to which these groups are bound, form a ring, the 5- and 6-membered alkane heterocyclic ring and the 5- and 6-membered heteroaromatic ring contain 1-3 heteroatoms selected from at least one of N, S, and O;

when $R_1$ is selected from -OH, $R_2$ and $R_3$, together with the atoms to which these groups are bound, form the benzene ring, a 5-7 membered alkane heterocyclic ring, or the 5- and 6-membered heteroaromatic ring that is substituted with 0-6 substituents, wherein the substituents are selected from deuterium, halogen, -OH, $-NH_2$, -CN, $C_{1-6}$ alkyl, $C_{1-6}$ fluoroalkyl, $C_{1-6}$ deuterated alkyl, -O-$C_{1-6}$ alkyl, -O-$C_{1-6}$ fluoroalkyl, -O-$C_{1-6}$ deuterated alkyl, $C_{3-6}$ cycloalkyl, $C_{3-6}$ fluorocycloalkyl, or 3- to 6-membered cycloalkyl formed by two of the substituents bound to the same carbon atom; and when $R_2$ and $R_3$, together with the atoms to which these groups are bound, form a ring, the 5-7 membered alkane heterocyclic ring and the 5- and 6-membered heteroaromatic ring contain 1-3 heteroatoms selected from at least one of N and S, or 2 atoms of O;

L is selected from $-(CH_2)_{n1}-$, O, $-(CH_2)_{n1}$-NH-, -NH-$(CH_2)_{n1}$-, and -NH-CH$(CH_2)_{n1}(CH_3)$-, where n1 is an integer selected from 0 to 3;

$R_6$ is selected from 6- to 10-membered aryl, 5- to 10-membered heteroaryl, 3- to 8-membered heterocycloalkyl, 3- to 8-membered cycloalkyl, 6- to 10-membered spirocycloalkyl, 6- to 10-membered heterospirocycloalkyl, 6- to 10-membered bridged cycloalkyl, 6- to 10-membered heterobridged cycloalkyl, and $C_{1-6}$ alkyl that are substituted with 0-6 substituents; in $R_6$, the substituents are selected from $R_{8a}$, halogen, the oxo group, $-OR_{8a}$, $-SR_{8a}$, $-C(=O)R_{8a}$, $-OC(=O)R_{8a}$, $-C(=O)OR_{8a}$, $-C(=O)NR_{8a}R_{8b}$, $-NR_{8a}C(=O)R_{8b}$, $-NR_{8a}R_{8b}$, $-SO_2R_{8a}$, $-SO_2NR_{8a}R_{8b}$, $-NR_{8a}$-$SO_2R_{8b}$, and -CN; and in $R_6$, 5- to 10-membered heteroaryl, 3- to 8-membered heterocycloalkyl, 6- to 10-membered heterospirocycloalkyl, and 6- to 10-membered bridged cycloalkyl contain 1-3 heteroatoms selected from at least one of N, S and O;

$R_{8a}$ and $R_{8b}$ are each independently selected from hydrogen, deuterium, or the following groups substituted with

0-6 substituents: $C_{1-4}$ alkyl, 3- to 6-membered cycloalkyl, 4- to 6-membered heterocycloalkyl, phenyl, 5- and 6-membered heteroaryl, (3- to 6-membered cycloalkyl)-methylene, and (4- to 6-membered heterocycloalkyl)-methylene; in $R_{8a}$ and $R_{8b}$, the substituents are selected from deuterium, halogen, $-N(R_{10a}R_{10b})$, -OH, -CN, $C_{1-4}$ alkyl, $C_{1-4}$ alkoxy, $C_{1-4}$ deuterated alkyl, 3- to 6-membered cycloalkyl, 4- to 6-membered heterocycloalkyl, (3- to 6-membered cycloalkyl)-methylene, or (4- to 6-membered heterocycloalkyl)-methylene; and in $R_{8a}$ and $R_{8b}$, 4- to 6-membered heterocycloalkyl, 5- and 6-membered heteroaryl, and (4- to 6-membered heterocycloalkyl)-methylene contain 1-3 heteroatoms selected from at least one of N, S and O; and 4- to 6-membered heterocycloalkyl and (4- to 6-membered heterocycloalkyl)-methylene in the substituents contain 1-3 heteroatoms selected from at least one of N, S and O;

or, $R_{8a}$ and $R_{8b}$, together with atoms to which these groups are bound, form a 3- to 6-membered alkylheterocyclic ring substituted with 0-6 substituents; when $R_{8a}$ and $R_{8b}$, together with the atoms to which these groups are bound, form a ring, the substituents are selected from deuterium, halogen, $-N(R_{11a}R_{11b})$, -OH, -CN, $C_{1-4}$ alkyl, 3- to 6-membered cycloalkyl, and 4- to 6-membered heterocycloalkyl; and when $R_{8a}$ and $R_{8b}$, together with the atoms to which these groups are bound, form the ring, 3- to 6-membered heterocycloalkyl contains 1-3 heteroatoms selected from at least one of N, S and O, and 4- to 6-membered heterocycloalkyl in the substituents contains 1-3 heteroatoms selected from at least one of N, S and O;

$R_{7a}$ is selected from hydrogen, or the following groups substituted with 0-6 substituents: $C_{1-6}$ alkyl, $C_{2-6}$ alkenyl, $C_{2-6}$ alkynyl, 3- to 6-membered cycloalkyl, 4- to 6-membered heterocycloalkyl, phenyl, and 5- and 6-membered heteroaryl; in $R_{7a}$, the substituents are selected from hydrogen, deuterium, halogen, -OH, $-NH_2$, or -CN; and in $R_{7a}$, 4- to 6-membered heterocycloalkyl and 5- and 6-membered heteroaryl contain 1-3 heteroatoms selected from at least one of N, S and O;

$R_{10a}$, $R_{10b}$, $R_{11a}$, and $R_{11b}$ are each independently selected from hydrogen or $C_{1-4}$ alkyl;

the pharmaceutically acceptable form is selected from pharmaceutically acceptable salts, esters, stereoisomers, polymorphs, solvates, nitrogen oxides, isotope markers, metabolites, or prodrugs.

**2.** The compound according to claim 1, wherein

$R_1$ is selected from hydrogen, deuterium, halogen, -OH, $-NH_2$, -CN, or the following groups optionally substituted with 0-6 substituents: $C_{1-4}$ alkyl, $-O-C_{1-4}$ alkyl, $-S-C_{1-4}$ alkyl, $C_{2-4}$ alkenyl, $C_{2-4}$ alkynyl, and 3- to 6-membered cycloalkyl, wherein the substituents are selected from deuterium, halogen, -OH, $-NH_2$, or -CN;

preferably, $R_1$ is selected from hydrogen, deuterium, halogen, F, Cl, -OH, $-NH_2$, -CN, or the following groups optionally substituted with 0-3 substituents: $C_{1-4}$ alkyl, $-O-C_{1-4}$ alkyl, $-S-C_{1-4}$ alkyl, and 3- to 6-membered cycloalkyl, wherein the substituents are selected from deuterium, F, Cl, -OH, $-NH_2$, or -CN;

more preferably, $R_1$ is selected from hydrogen, deuterium, F, Cl, -OH, $-CH_3$, fluoromethyl, deuterated methyl, methoxy, fluoromethoxy, deuterated methoxy, cyclopropyl, and fluorocyclopropyl.

**3.** The compound according to claim 1 or 2, wherein

$R_2$ and $R_4$ are each independently selected from hydrogen, deuterium, halogen, -OH, $-NH_2$, -CN, or the following groups optionally substituted with 0-6 substituents: $C_{1-4}$ alkyl, $-O-C_{1-4}$ alkyl, $-S-C_{1-4}$ alkyl, $C_{2-4}$ alkenyl, $C_{2-4}$ alkynyl, and 3- to 6-membered cycloalkyl, wherein the substituents are selected from deuterium, halogen, -OH, $-NH_2$, or -CN;

preferably, $R_2$ and $R_4$ are each independently selected from hydrogen, deuterium, F, Cl, -OH, $-NH_2$, -CN, or the following groups optionally substituted with 0-3 substituents: $C_{1-4}$ alkyl, $-O-C_{1-4}$ alkyl, $-S-C_{1-4}$ alkyl, and 3- to 6-membered cycloalkyl, wherein the substituents are selected from deuterium, F, Cl, -OH, $-NH_2$, or -CN;

more preferably, $R_2$ and $R_4$ are each independently selected from hydrogen, deuterium, F, Cl, -OH, $-CH_3$, fluoromethyl, deuterated methyl, methoxy, fluoromethoxy, deuterated methoxy, cyclopropyl, and fluorocyclopropyl.

**4.** The compound according to any of claims 1-3, wherein

$R_3$ is selected from hydrogen, deuterium, halogen, -OH, $-NH_2$, -CN, or the following groups optionally substituted with 0-6 substituents: $C_{1-4}$ alkyl, $-O-C_{1-4}$ alkyl, $-S-C_{1-4}$ alkyl, $C_{2-4}$ alkenyl, $C_{2-4}$ alkynyl, 3- to 6-membered cycloalkyl, 5- and 6-membered heterocycloalkyl, phenyl, and 5- and 6-membered heteroaryl; in $R_3$, the substituents are selected from deuterium, halogen, -OH, $-NH_2$, -CN, $-CF_3$, or cyclopropyl; and in $R_3$, 5- and 6-membered heterocycloalkyl and 5- and 6-membered heteroaryl contain 1-3 heteroatoms selected from at least one of N, S, and O;

preferably, $R_3$ is selected from hydrogen, deuterium, F, Cl, -CN, or the following groups optionally substituted with

0-3 substituents: $C_{1-4}$ alkyl, -O-$C_{1-4}$ alkyl, -S-$C_{1-4}$ alkyl, $C_{2-4}$ alkenyl, $C_{2-4}$ alkynyl, 3- to 6-membered cycloalkyl, 5- and 6-membered heterocycloalkyl, phenyl, and 5- and 6-membered heteroaryl; in $R_3$, the substituents are selected from deuterium, F, Cl, -OH, -$NH_2$, -$CF_3$, -CN, or cyclopropyl; and in $R_3$, 5- and 6-membered heterocycloalkyl and 5- and 6-membered heteroaryl contain 1-2 heteroatoms selected from at least one of N, S, and O; more preferably, $R_3$ is selected from hydrogen, deuterium, F, Cl, methyl, fluoromethyl, deuterated methyl, methylthio, fluoromethylthio, deuterated methylthio, methoxy, fluoromethoxy, deuterated methoxy, cyclopropyl, fluorocyclopropyl, vinyl, ethynyl, phenyl, fluorophenyl, and deuterated phenyl.

**5.** The compound according to any of claims 1-4, wherein

$R_5$ is selected from hydrogen, deuterium, halogen, -$NH_2$, -CN, or the following groups optionally substituted with 0-6 substituents: $C_{1-4}$ alkyl, -O-$C_{1-4}$ alkyl, -S-$C_{1-4}$ alkyl, $C_{2-4}$ alkenyl, $C_{2-4}$ alkynyl, and 3- to 6-membered cycloalkyl, wherein the substituents are selected from deuterium, halogen, -OH, -$NH_2$, or -CN;
preferably, $R_5$ is selected from hydrogen, deuterium, F, Cl, -$NH_2$, -CN, or the following groups optionally substituted with 0-3 substituents: $C_{1-4}$ alkyl, -O-$C_{1-4}$ alkyl, -S-$C_{1-4}$ alkyl, and 3- to 6-membered cycloalkyl, wherein the substituents are selected from deuterium, F, Cl, -OH, -$NH_2$, or -CN;
more preferably, $R_5$ is selected from hydrogen, deuterium, F, Cl, -CH, -$CH_3$, fluoromethyl, deuterated methyl, methoxy, fluoromethoxy, deuterated methoxy, cyclopropyl, and fluorocyclopropyl.

**6.** The compound according to any of claims 1-5, wherein

$R_2$ and $R_3$, $R_3$ and $R_4$, or $R_4$ and $R_5$, together with the atoms to which these groups are bound, form the 5- and 6-membered alkane ring, the benzene ring, the 5- and 6-membered alkane heterocyclic ring, or the 5- and 6-membered heteroaromatic ring that is substituted with 0-6 substituents, wherein the substituents are selected from deuterium, halogen, -OH, -$NH_2$, -CN, the oxo group, $C_{1-4}$ alkyl, $C_{1-4}$ fluoroalkyl, $C_{1-4}$ deuterated alkyl, -O-$C_{1-4}$ alkyl, -O-$C_{1-4}$ fluoroalkyl, -O-$C_{1-4}$ deuterated alkyl, 3- to 6-membered cycloalkyl, 3- to 6-membered fluorocycloalkyl, or 3- and 4-membered cycloalkyl formed by two of the substituents bound to a same carbon atom; when $R_2$ and $R_3$, $R_3$ and $R_4$, or $R_4$ and $R_5$, together with the atoms to which these groups are bound, forms the ring and the 5- and 6-membered alkane heterocyclic ring, the 5- and 6-membered heteroaromatic ring contain 1-2 heteroatoms selected from at least one of N, S, and O;
preferably, $R_2$ and $R_3$, or $R_3$ and $R_4$, together with atoms to which these groups are bound, forms

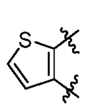, 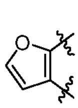, 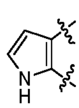, 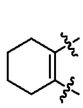, 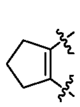, 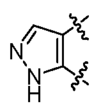, 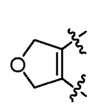, 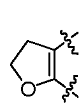, 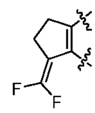, 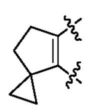,

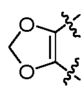, 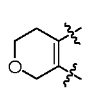, 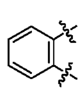, 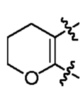, 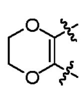, 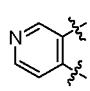 or 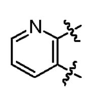

that is substituted with 0-3 substituents, wherein the substituents are selected from deuterium, F, Br, Cl, -OH, -$NH_2$, -CN, the oxo group, methyl, fluoromethyl, deuterated methyl, methoxy, fluoromethoxy, deuterated methoxy, cyclopropyl, fluorocyclopropyl, or3- and 4-membered cycloalkyl formed by two of the substituents bound to the same carbon atom.

**7.** The compound according to any of claims 1-5, wherein

when $R_1$ is selected from -OH, $R_2$ and $R_3$, together with the atoms to which these groups are bound, form the benzene ring, the 5- and 6-membered alkane heterocyclic ring, or the 5- and 6-membered heteroaromatic ring that is substituted with 0-6 substituents, wherein the substituents are selected from deuterium, halogen, -OH, -$NH_2$, -CN, $C_{1-4}$ alkyl, $C_{1-4}$ fluoroalkyl, $C_{1-4}$ deuterated alkyl, -O-$C_{1-4}$ alkyl, -O-$C_{1-4}$ fluoroalkyl, -O-$C_{1-4}$ deuterated alkyl, 3- to 6-membered cycloalkyl, 3- to 6-membered fluorocycloalkyl, or 3- and 4-membered cycloalkyl formed by two of the substituents bound to the same carbon atom; and when $R_2$ and $R_3$, together with the atoms to which these groups are bound, form the ring, the 5- and 6-membered alkane heterocyclic ring contains two atoms of O, and the 5- and 6-membered heteroaromatic ring contains 1-2 heteroatoms selected from at least one of N, S, and O;
preferably, when $R_1$ is selected from -OH, $R_2$ and $R_3$, together with the atoms to which these groups are bound,

form

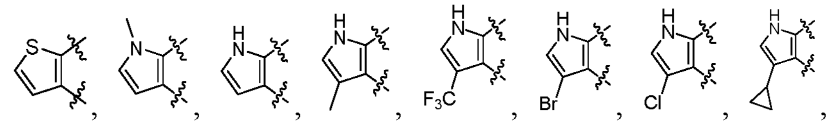
, , , , , , , ,

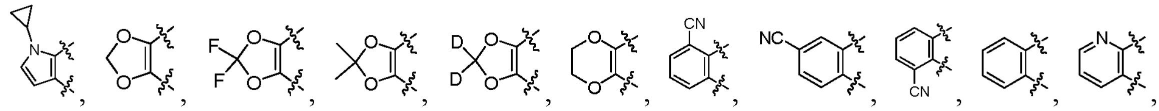
, , , , , , , , , , ,

and

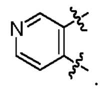
.

**8.** The compound according to any of claims 1-7, wherein a structural unit

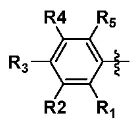

is selected from:

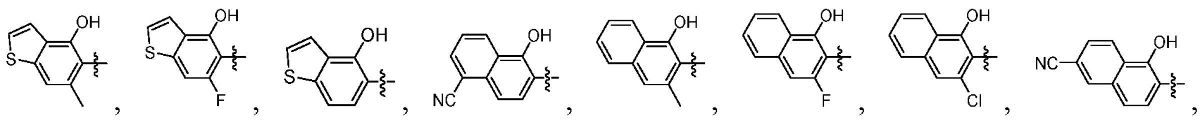
, , , , , , , ,

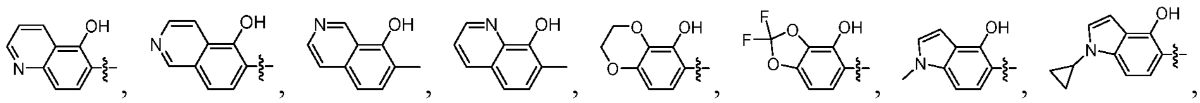
, , , , , , , ,

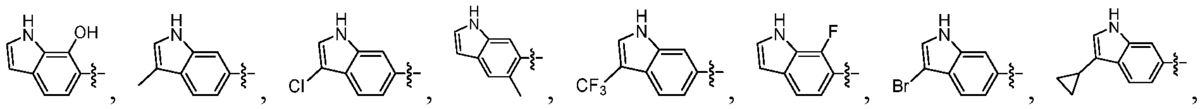
, , , , , , , ,

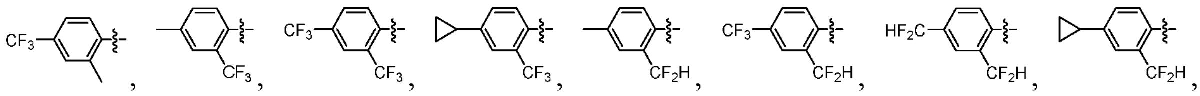
, , , , , , , ,

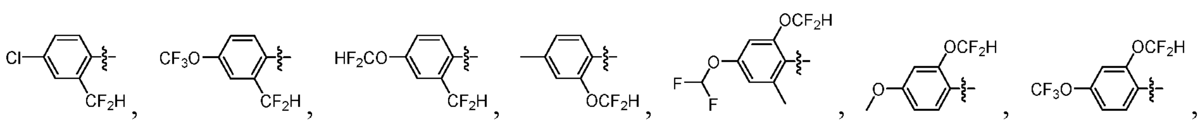
, , , , , , ,

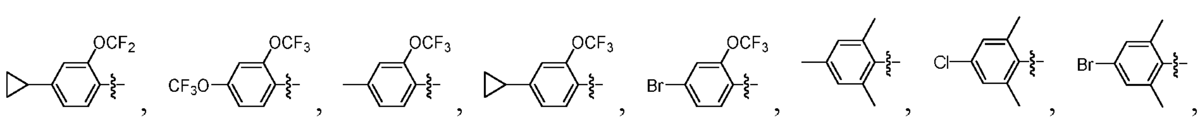
, , , , , , , ,

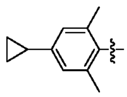, 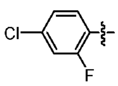, 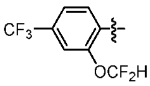, 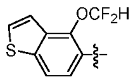, 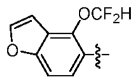, 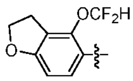, 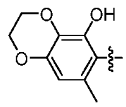, 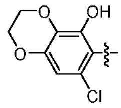,

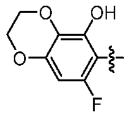, 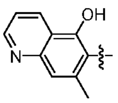, 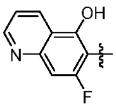, 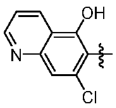, 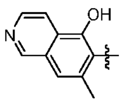, 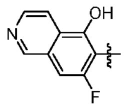, 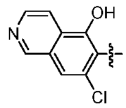, 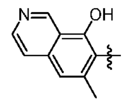,

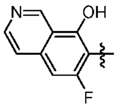, 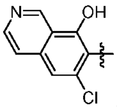, 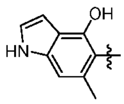, 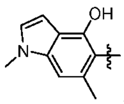, 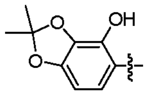, 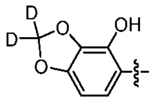, and 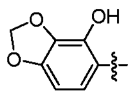.

**9.** The compound according to any of claims 1-8, wherein

$R_{7a}$ is selected from hydrogen, or the following groups optionally substituted with 0-6 substituents: $C_{1-4}$ alkyl, $C_{2-6}$ alkenyl, $C_{2-6}$ alkynyl, 3- to 6-membered cycloalkyl, 4- to 6-membered heterocycloalkyl, phenyl, and 5- and 6-membered heteroaryl; in $R_{7a}$, the substituents are selected from hydrogen, deuterium, halogen, -OH, $-NH_2$, or -CN;

preferably, $R_{7a}$ is selected from hydrogen, $C_{1-4}$ alkyl, $C_{1-4}$ fluoroalkyl, $C_{1-4}$ deuterated alkyl, $C_{1-4}$ alkoxy, $C_{1-4}$ fluoroalkoxy, $C_{1-4}$ deuterated alkoxy, carboxyl, $C_{1-4}$ alkoxycarbonyl, $C_{1-4}$ fluoroalkoxycarbonyl, $C_{1-4}$deuterated alkoxycarbonyl, 3- to 6-membered cycloalkyl, 3- to 6-membered fluorocycloalkyl, phenyl, pyridinyl, or

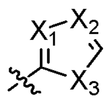;

$X_1$ and $X_2$ are each independently selected from CH and N; and $X_3$ is selected from N-$C_{1-4}$ alkyl, NH, S, and O;

more preferably, $R_{7a}$ is selected from hydrogen, methyl, deuterated methyl, fluoromethyl, ethyl, fluoroethyl, isopropyl, fluoroisopropyl, cyclopropyl, fluorocyclopropyl, cyclohexyl, fluorocyclohexyl, phenyl, 2-pyridinyl, and

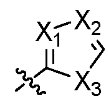;

$X_1$ is selected from CH and N; and $X_3$ is selected from N-methyl, S and O.

**10.** The compound according to any of claims 1-9, wherein a structural unit

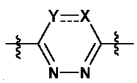

is selected from:

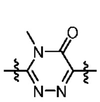, 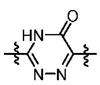, 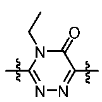, 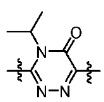, 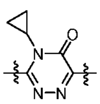, 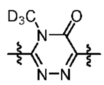, 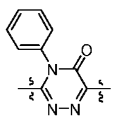, 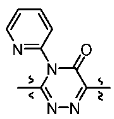, 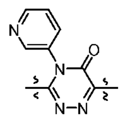,

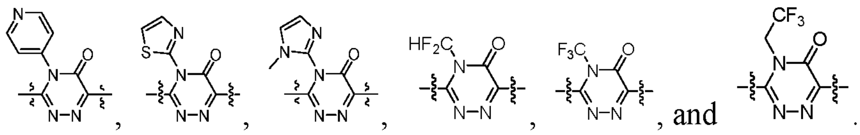

, , , , , and .

11. The compound according to any of claims 1-10, wherein L is selected from O, -NH-, $-NH-CH_2-$, and $-NH-CH(CH_3)-$.

12. The compound according to any of claims 1-11, wherein in $R_6$, the substituents are selected from fluorine, chlorine, a hydroxy group, a cyanogroup, the oxo group, $C_{1-4}$ alkyl, $C_{1-4}$ fluoroalkyl, $C_{1-4}$ deuterated alkyl, $C_{1-4}$ alkoxy, $C_{1-4}$ fluoroalkoxy, $C_{1-4}$ deuterated alkoxy, 3- to 6-membered cycloalkyl, 3- to 6-membered fluorocycloalkyl, an amino group, a dimethylamino group,

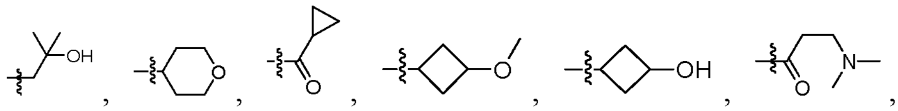

, , , , , , ,

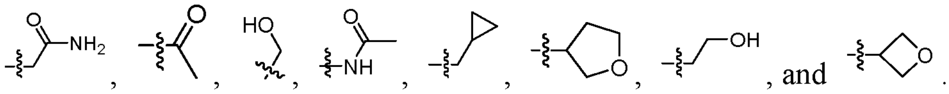

, , , , , , , and .

13. The compound according to any of claims 1-12, wherein $R_6$ is selected from the following structures:

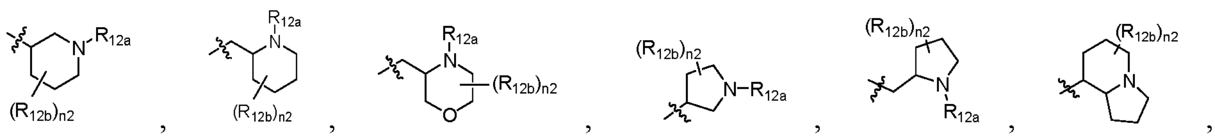

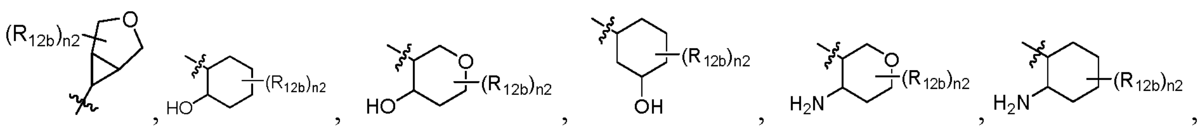

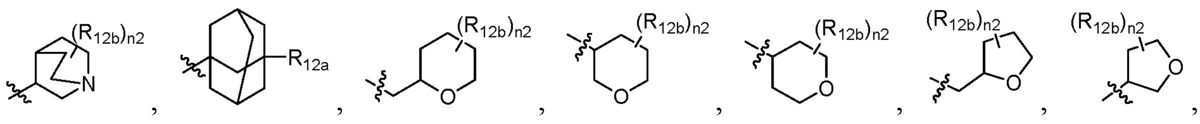

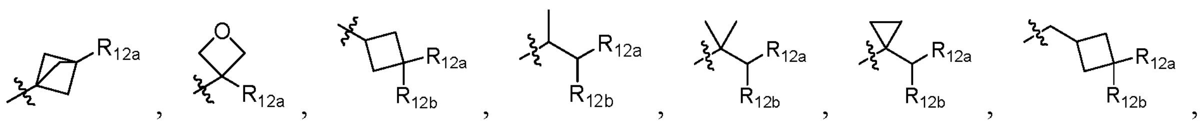

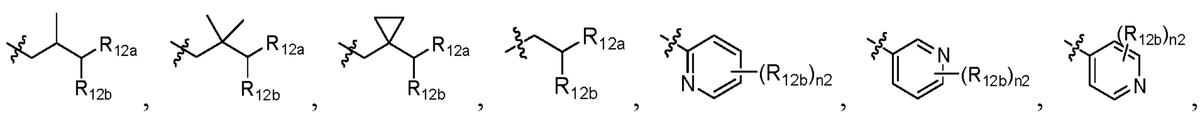

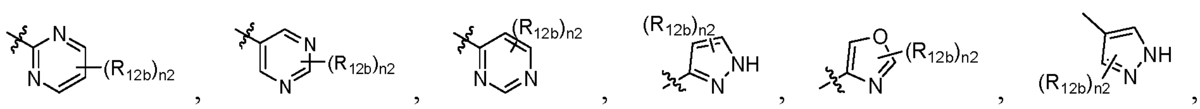

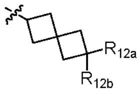, 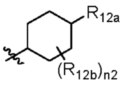, 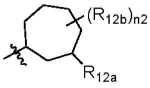, 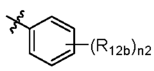, and 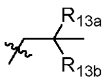,

wherein

$R_{12a}$ and $R_{12b}$ are each independently selected from $R_{8a}$, halogen, the oxo group, $-OR_{8a}$, $-SR_{8a}$, $-C(=O)R_{8a}$, $-OC(=O)R_{8a}$, $-C(=O)OR_{8a}$, $-C(=O)NR_{8a}R_{8b}$, $-NR_{8a}C(=O)R_{8b}$, $-NR_{8a}R_{8b}$, $-SO_2R_{8a}$, $-SO_2NR_{8a}R_{8b}$, $-NR_{8a}SO_2R_{8b}$, and -CN, and n2 is an integer selected from 0 to 6;
preferably, $R_{12a}$ and $R_{12b}$ are each independently selected from $R_{8a}$, halogen, the oxo group, $-OR_{8a}$, $-SR_{8a}$, $-C(=O)R_{8a}$, $-OC(=O)R_{8a}$, $-C(=O)OR_{8a}$, $-C(=O)NR_{8a}R_{8b}$, $-NR_{8a}C(=O)R_{8b}$, $-NR_{8a}R_{8b}$, $-SO_2R_{8a}$, $-SO_2NR_{8a}R_{8b}$, $-NR_{8a}SO_2R_{8b}$, and -CN, and n2 is an integer selected from 0 to 3;
more preferably, $R_{12a}$ and $R_{12b}$ are each independently selected from fluorine, chlorine, the hydroxy group, the cyanogroup, the oxo group, $C_{1-4}$ alkyl, $C_{1-4}$ fluoroalkyl, $C_{1-4}$ deuterated alkyl, $C_{1-4}$ alkoxy, $C_{1-4}$ fluoroalkoxy, $C_{1-4}$ deuterated alkoxy, 3- to 6-membered cycloalkyl, 3-to 6-membered fluorocycloalkyl, the amino group, the dimethylamino group,

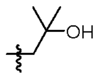,

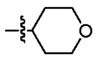, 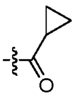, 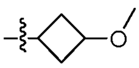, 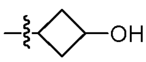, 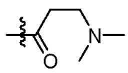, 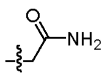, 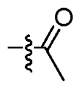, 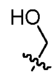, 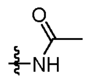,

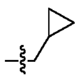, 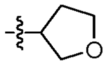, 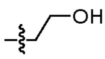, and 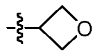.

**14.** The compound according to any of claims 1-13, wherein $R_6$ is selected from the following structures:

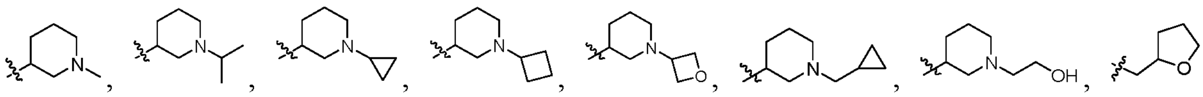, , , , , , , ,

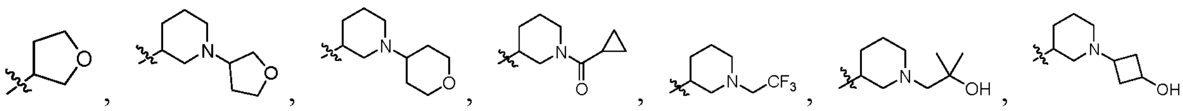, , , , , , ,

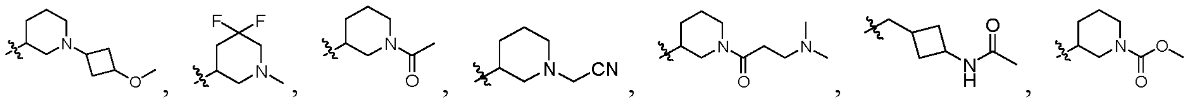, , , , , , ,

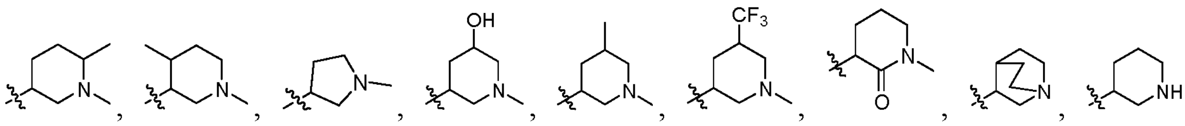, , , , , , , , ,

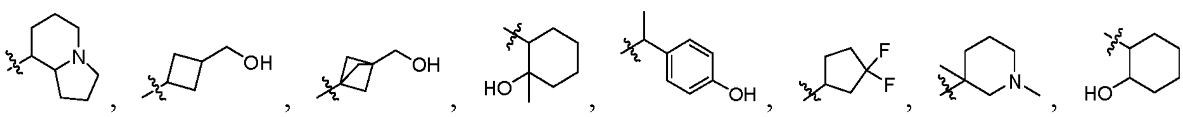, , , , , , , ,

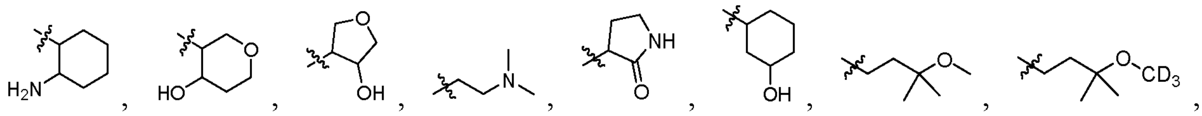,

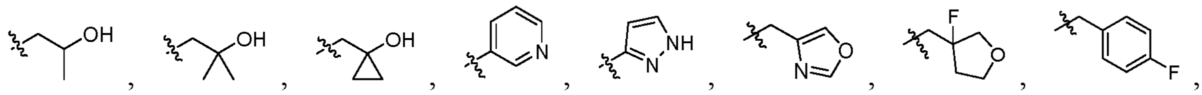,

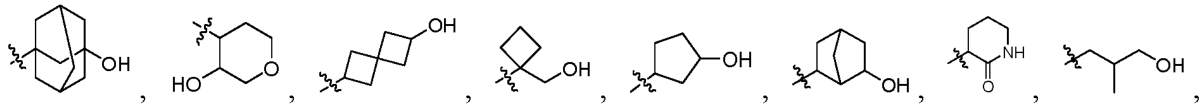,

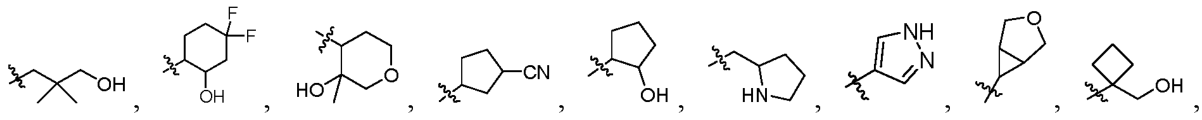,

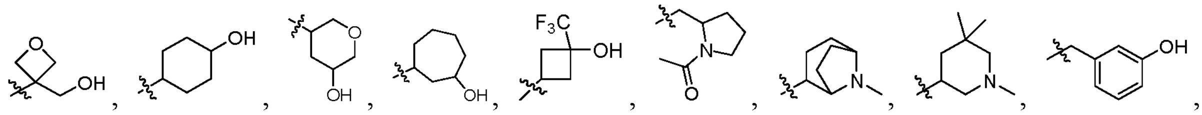,

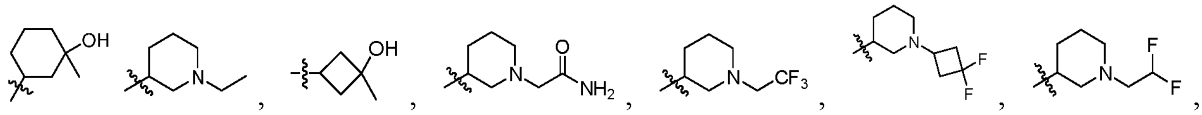,

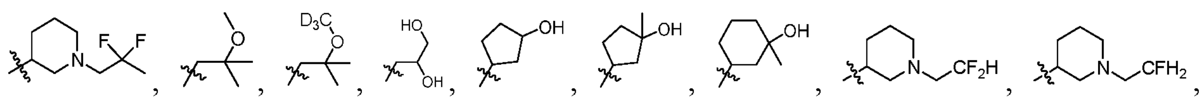,

and

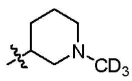.

**15.** The compound according to any of claims 1-14, wherein the compound is selected from:

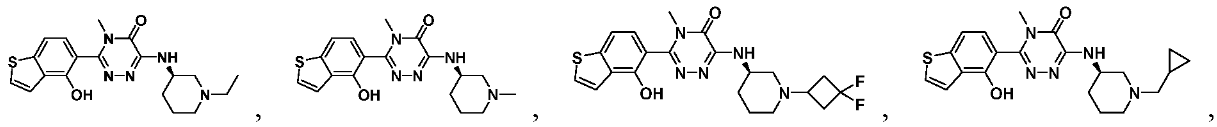,

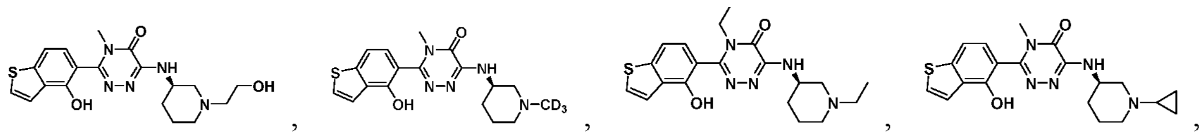,

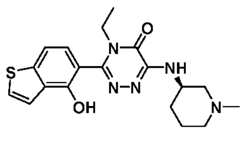, 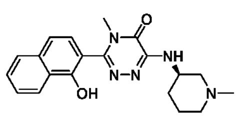, 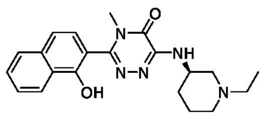, 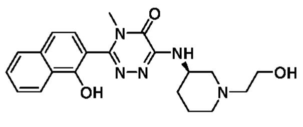,

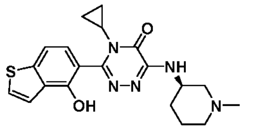, 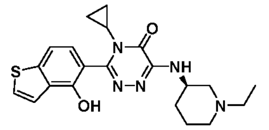, 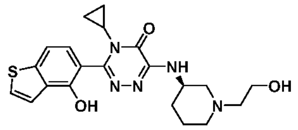, 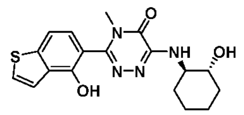,

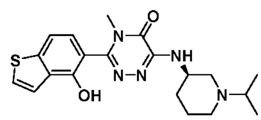, 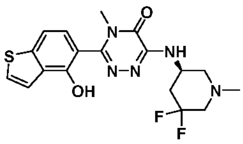, 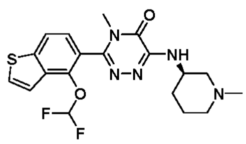, or 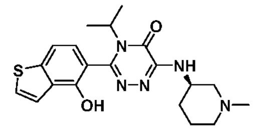.

**16.** The compound according to claim 1, wherein a structural unit

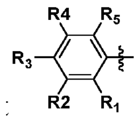

is selected from:

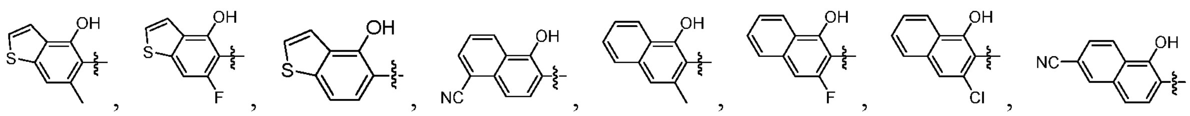
, , , , , , , ,

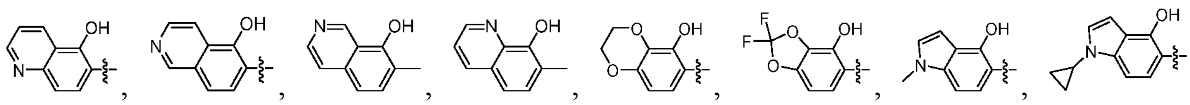
, , , , , , , ,

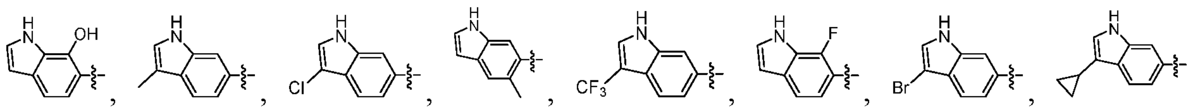
, , , , , , , ,

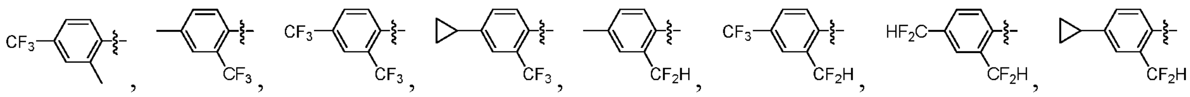
, , , , , , , ,

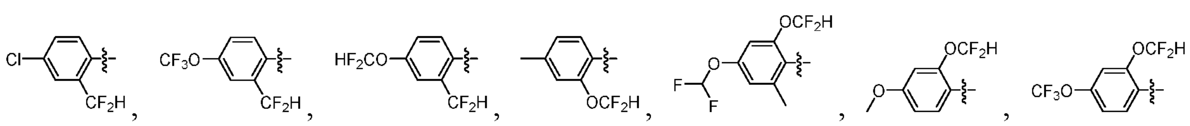
, , , , , , ,

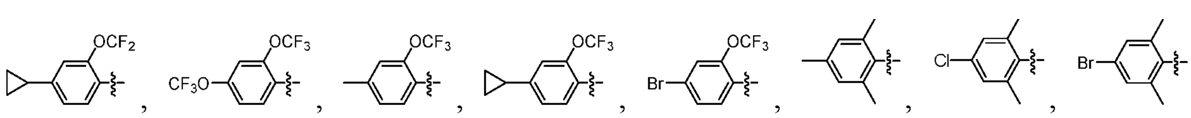
, , , , , , , ,

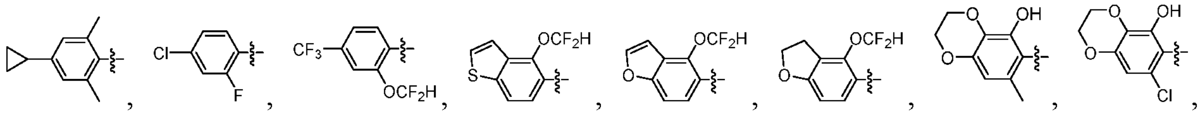

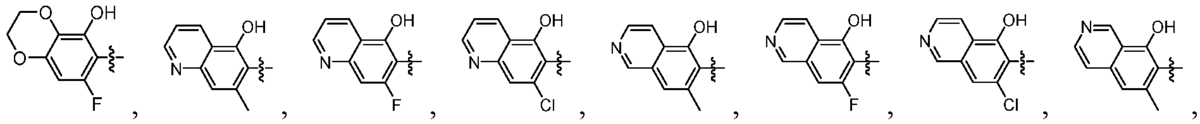

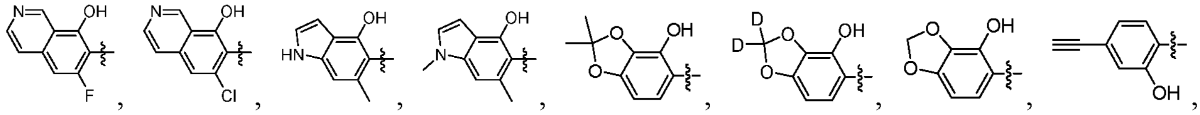

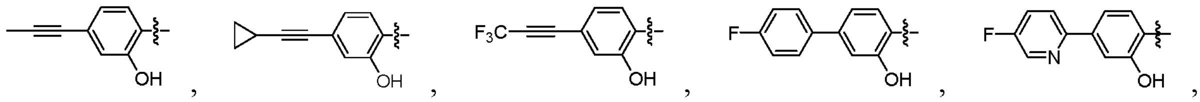

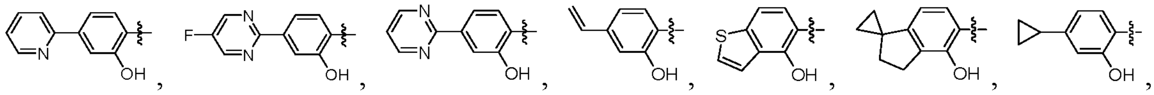

and

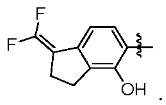

**17.** The compound according to claim 16, wherein a structural unit

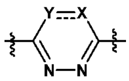

is selected from:

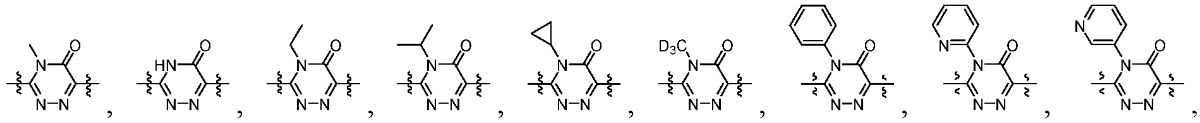

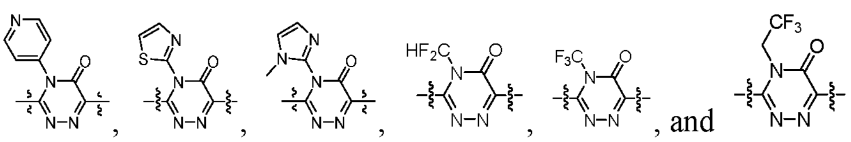

, and

**18.** The compound according to claim 16 or 17, wherein L is selected from O, -NH-, $-NH-CH_2-$, and$-NH-CH(CH_3)-$.

**19.** The compound according to any of claims 1-18, wherein $R_6$ is selected from the following structures:

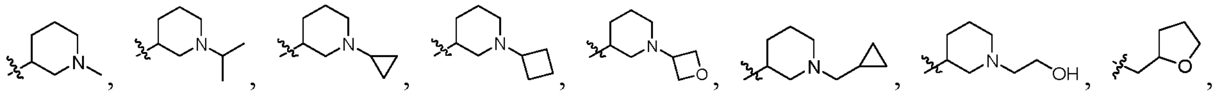

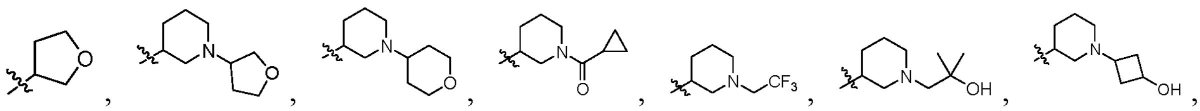
, , , , , , ,

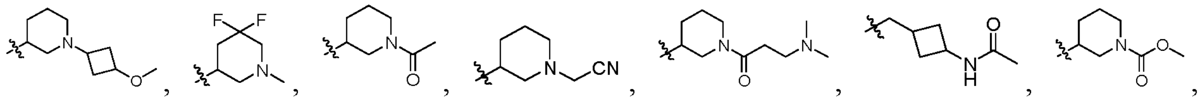
, , , , , , ,

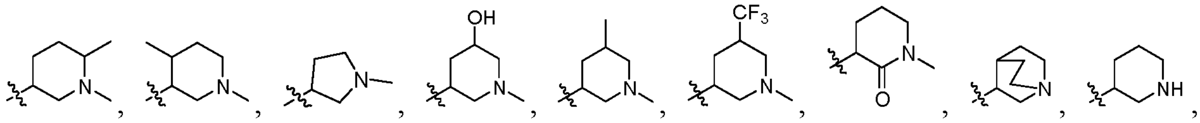
, , , , , , , , ,

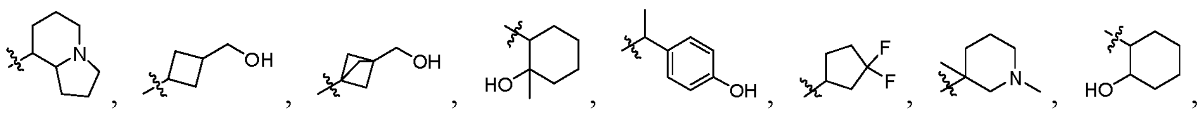
, , , , , , , ,

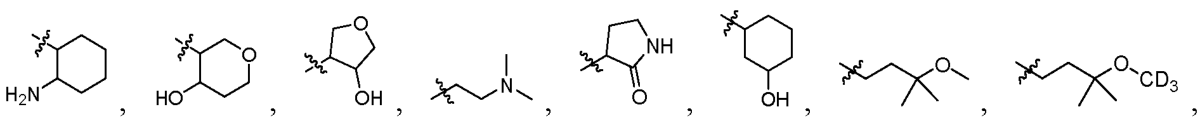
, , , , , , , ,

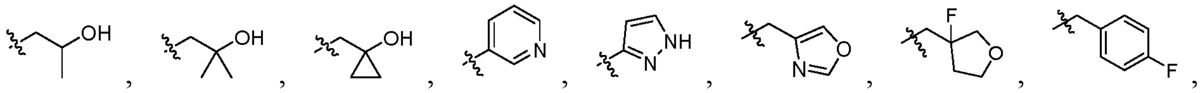
, , , , , , , ,

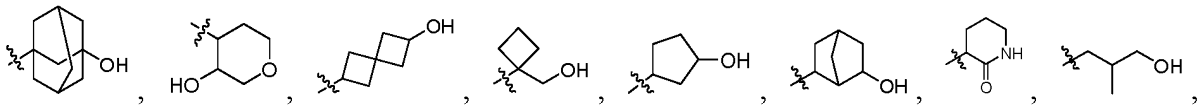
, , , , , , , , ,

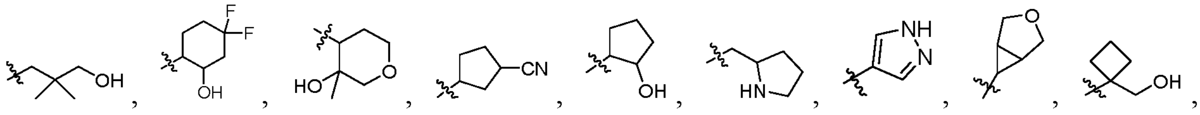
, , , , , , , , ,

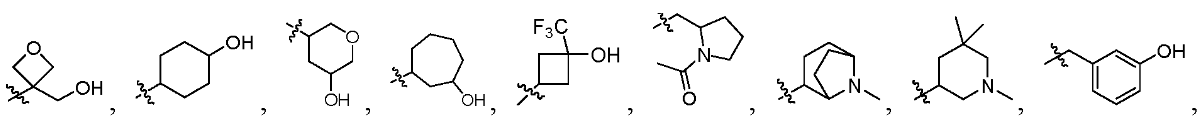
, , , , , , , , ,

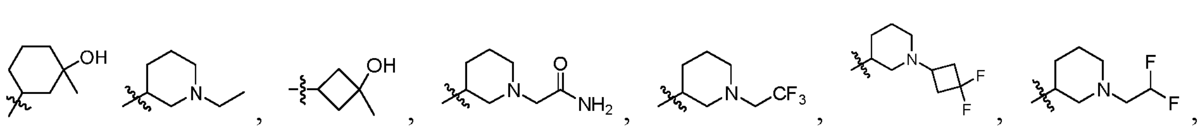
, , , , , ,

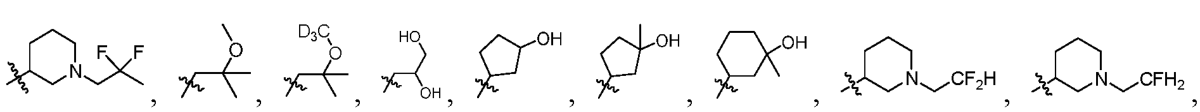
, , , , , , , , ,

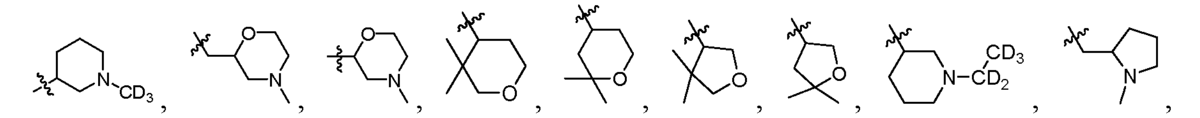
, , , , , , , , ,

and

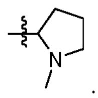.

**20.** The compound according to any of claims 16-19, wherein the compound is selected from:

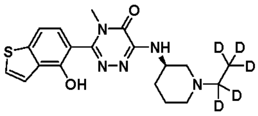, 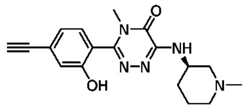, 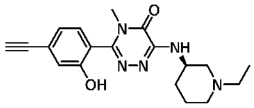, 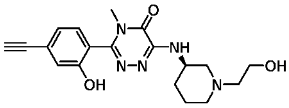,

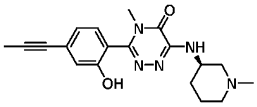, 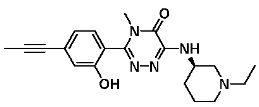, 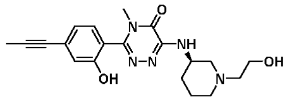, 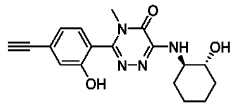,

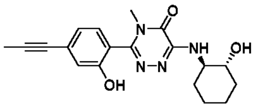, 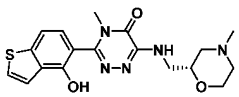, 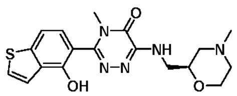, 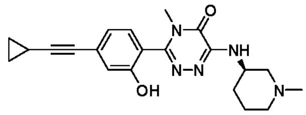,

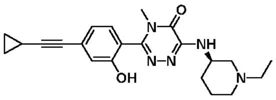, 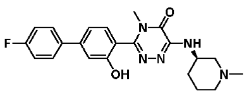, 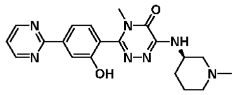, 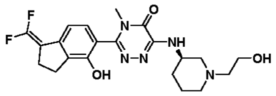,

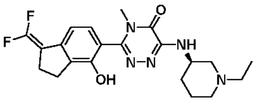, 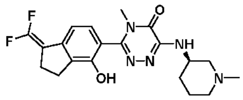, 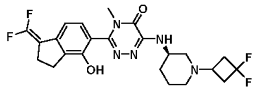, 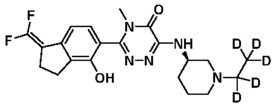,

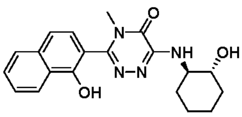, 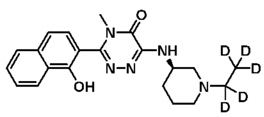, or 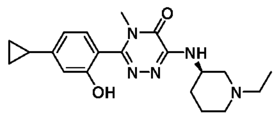.

**21.** The compound according to claim 1, wherein a structural unit

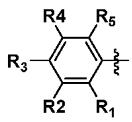

is selected from:

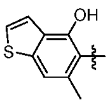, 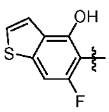, 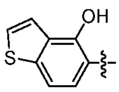, 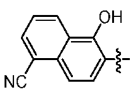, 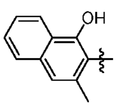, 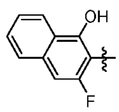, 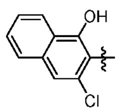, 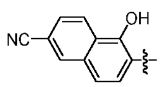,

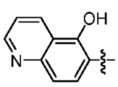, 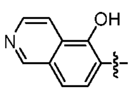, 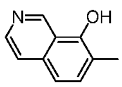, 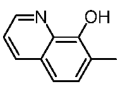, 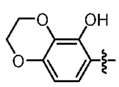, 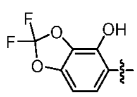, 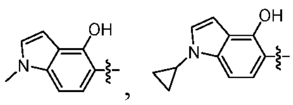, ,

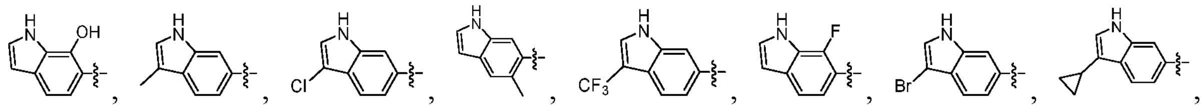
, , , , , , , ,

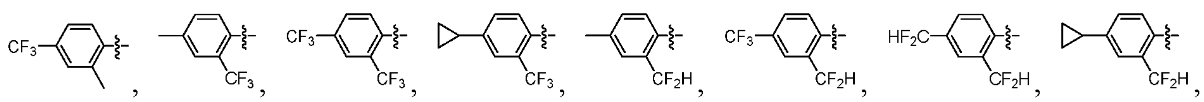
, , , , , , , ,

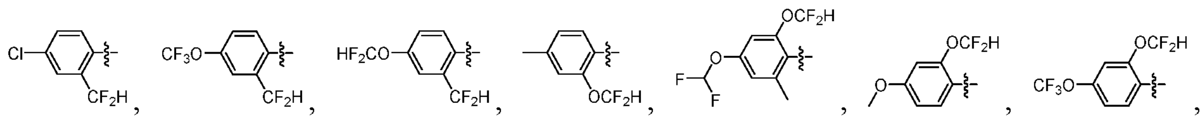
, , , , , , ,

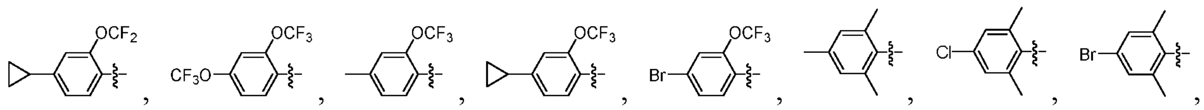
, , , , , , , ,

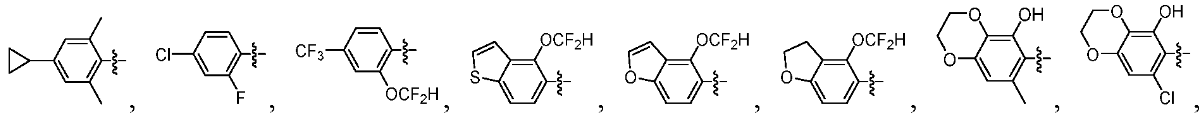
, , , , , , , ,

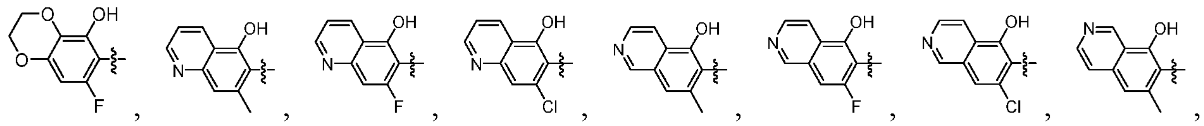
, , , , , , , ,

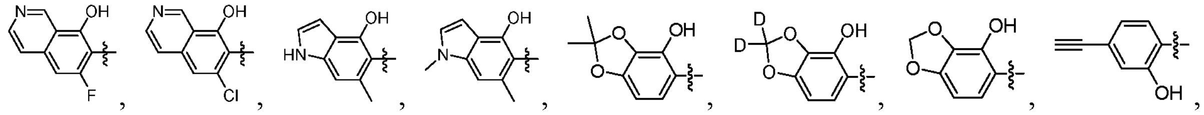
, , , , , , , ,

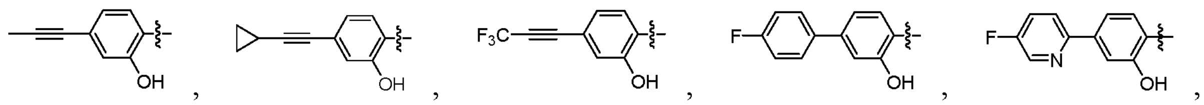
, , , , ,

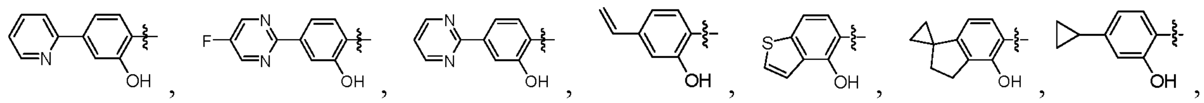
, , , , , , ,

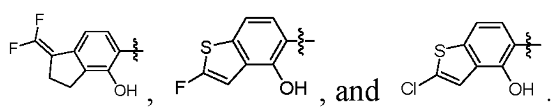
, , and .

**22.** The compound according to claim 21, wherein a structural unit

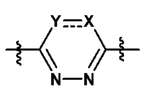

is selected from:

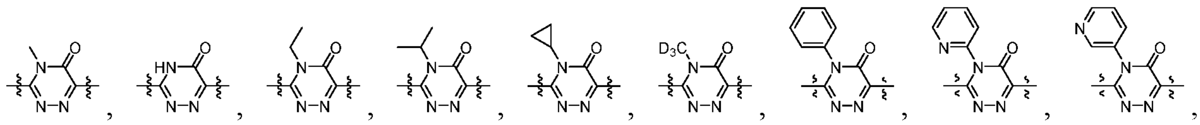,

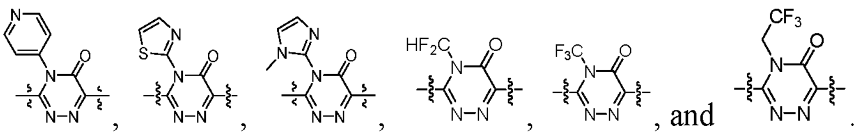, and .

**23.** The compound according to claim 21 or 22, wherein L is selected from O, -NH-, $-NH-CH_2-$, and $-NH-CH(CH_3)-$.

**24.** The compound according to any of claims 21-23, wherein $R_6$ is selected from the following structures:

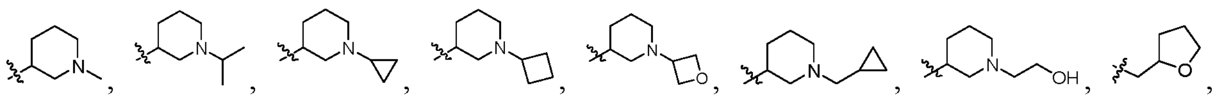,

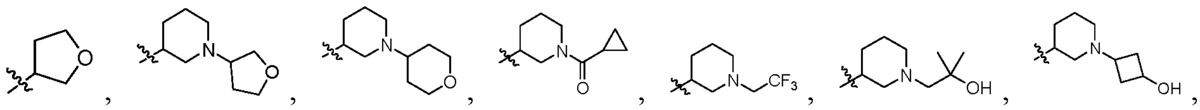,

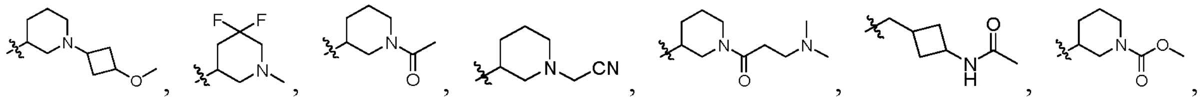,

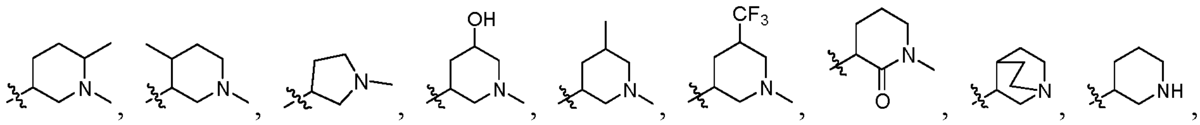,

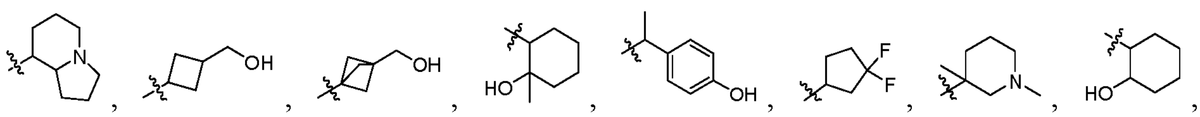,

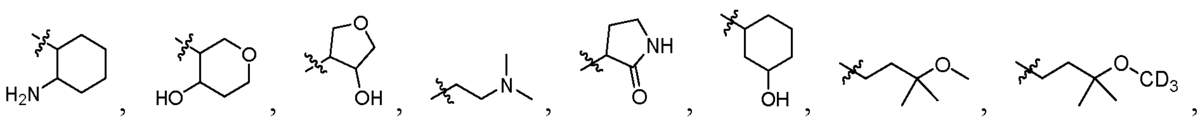,

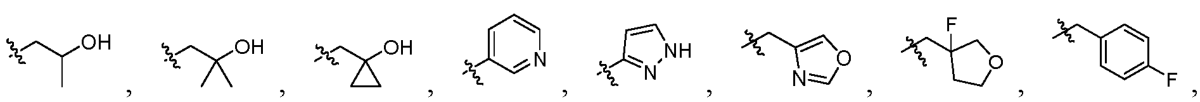,

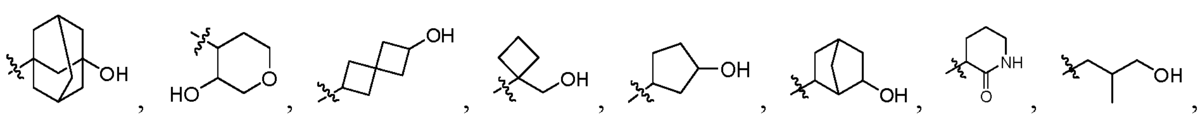,

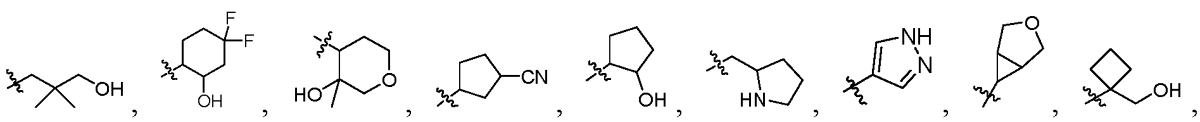,

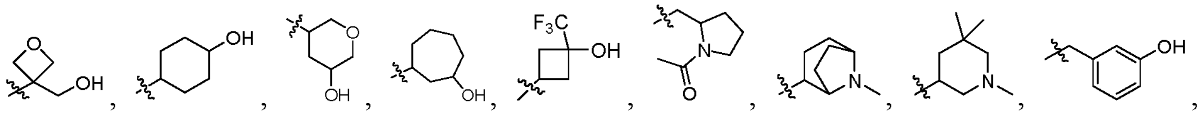
, , , , , , , , , ,

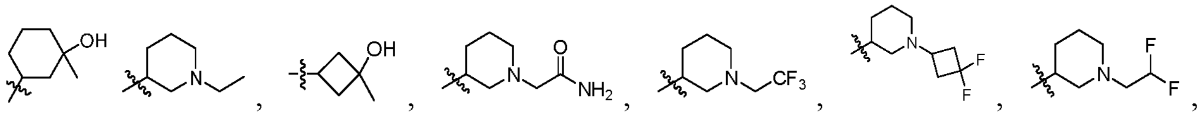
, , , , , ,

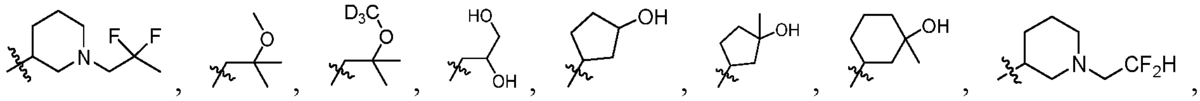
, , , , , , , ,

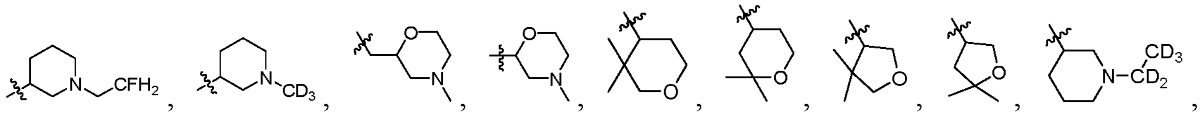
, , , , , , , , ,

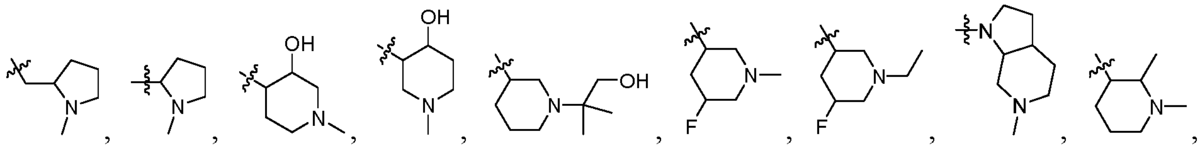
, , , , , , , , ,

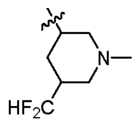
, and
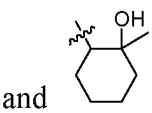
.

**25.** The compound according to any of claims 21-24, wherein the compound is selected from:

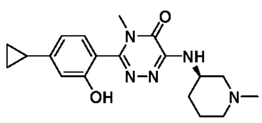
,
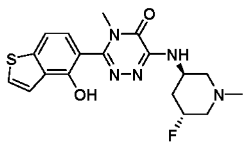
,
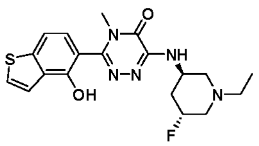
,
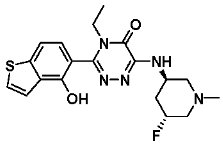
,
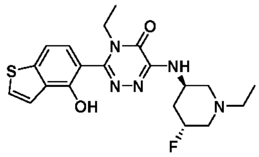
,
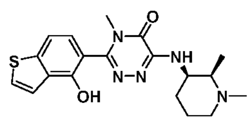
,
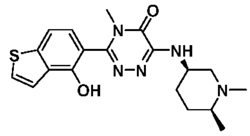
,
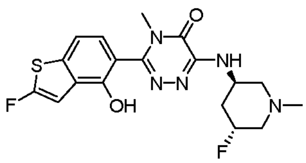
,

or

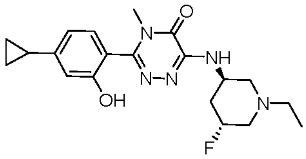
.

**26.** The compound according to any of claims 1-14, wherein a structural unit

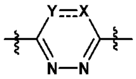

is selected from:

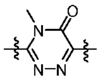, 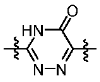, 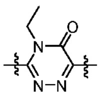, 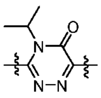, 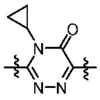, 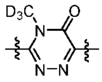, 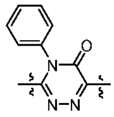, 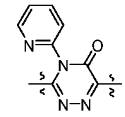, 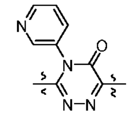,

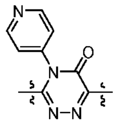, 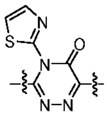, 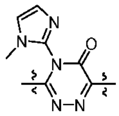, 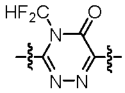, 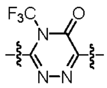, 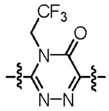, and 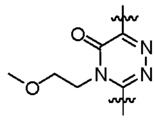.

**27.** The compound according to claim 26, wherein $R_6$ is selected from the following structures:

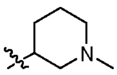, 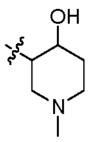, 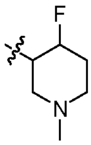, 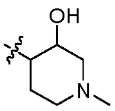, 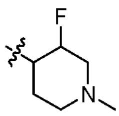, 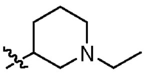, and 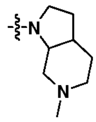.

**28.** The compound according to claim 26 or 27, wherein the compound is selected from:

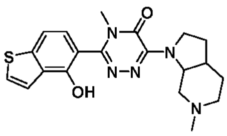, 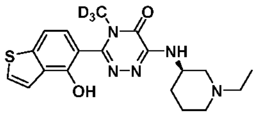, or 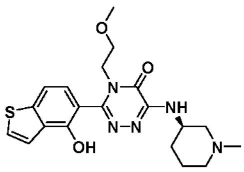.

**29.** A pharmaceutical composition, wherein the pharmaceutical composition takes the compound according to any one of claims 1-28, or pharmaceutically acceptable salts, esters, stereoisomers, tautomers, polymorphs, solvates, nitrogen oxides, isotope markers, metabolites, or prodrugs thereof as active ingredients, together with a pharmaceutically acceptable carrier.

**30.** A use of the compound according to any one of claims 1-28or pharmaceutically acceptable salts, esters, stereoisomers, tautomers, polymorphs, solvates, nitrogen oxides, isotope markers, metabolites, or prodrugs, and the pharmaceutical composition according to claim 29 in preparation of drugs for prevention and/or treatment of NLRP3-related diseases.

**31.** The use according to claim 30, wherein the NLRP3-related diseases comprise inflammatory diseases, autoimmune diseases, cardiovascular diseases, cancers, renal system diseases, gastrointestinal diseases, respiratory diseases, endocrine system diseases, or central nervous system diseases.

**32.** The use according to claim 31, wherein the NLRP3-related diseases comprise cryopyrin-associated periodic syndrome (CAPS), Muckle-Wells syndrome, familial cold autoinflammatory syndrome, neonatal-onset multisystem inflammatory disease (NOMID), familial Mediterranean fever, non-alcoholic steatohepatitis, alcoholic liver disease, graft-versus-host disease, multiple sclerosis, rheumatoid arthritis, type I/II diabetes mellitus and related complications, psoriasis, Alzheimer's disease, atherosclerosis, gout, chronic kidney disease, sepsis, liver fibrosis, idiopathic pulmonary fibrosis, epilepsy, neuropathic pain, depressive disorders, Parkinson's disease, asthma, acute myocardial infarction, systemic lupus erythematosus, rheumatoid arthritis, Crohn's disease, ulcerative colitis, inflammatory bowel disease, rheumatoid arthritis, ankylosing spondylitis, bronchial asthma, acute respiratory distress syndrome, chronic obstructive pulmonary disease, or ischemic stroke.

**INTERNATIONAL SEARCH REPORT**

International application No.
**PCT/CN2024/083349**

**A. CLASSIFICATION OF SUBJECT MATTER**

C07D409/14(2006.01)i; C07D401/12(2006.01)i; A61P35/00(2006.01)i; A61P29/00(2006.01)i; A61P37/06(2006.01)i; A61K31/53(2006.01)i

According to International Patent Classification (IPC) or to both national classification and IPC

**B. FIELDS SEARCHED**

Minimum documentation searched (classification system followed by classification symbols)

IPC: C07D A61P A61K

Documentation searched other than minimum documentation to the extent that such documents are included in the fields searched

Electronic data base consulted during the international search (name of data base and, where practicable, search terms used)

CNTXT, ENTXT, DWPI, STN(REGISTRY, CAPLUS), CNKI, Baidu, Google学术, Google Scholar: 成都賾灵生物医药, 陈俐娟, 王太津, 贾涛, 李刚, 苯并噻唑, 三嗪, 酮, 哌啶, NLRP3, benzothiaz?, triazi?, piperi?d, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| E | CN 117986258 A (TRANSTHERA SCIENCES (NANJING), INC.) 07 May 2024 (2024-05-07)<br>see intermediates of embodiments 1-2 and 32 | 1, 3-5, 9-11, 13, 26 |
| E | WO 2024099996 A1 (F. HOFFMANN-LA ROCHE AG et al.) 16 May 2024 (2024-05-16)<br>see compounds of embodiments 1-3, embodiments A-B, and claims 7-17 | 1-2, 5-6, 9-14, 19, 26-27, 29-32 |
| E | WO 2024099992 A1 (F. HOFFMANN-LA ROCHE AG et al.) 16 May 2024 (2024-05-16)<br>embodiments 2, 5, and 6, preparation of intermediates of embodiments 1, 3-4, and 7, and claims 18-26 | 1-7, 9-12, 26-27, 29-32 |
| X | ACS. "RNs: 1808699-98-0, 1022043-72-6, and 896061-81-7"<br>*STN,* 29 September 2015 (2015-09-29),<br>see pp. 1-10 | 1-5, 9-10, 13, 26 |
| X | CN 1349514 A (SYNGENTA PARTICIPATIONS AG) 15 May 2002 (2002-05-15)<br>see abstract, and table 1 | 1-5, 7, 10, 13, 26 |
| X | JP 2020079325 A (SUMITOMO CHEMICAL CO., LTD.) 28 May 2020 (2020-05-28)<br>see description, paragraphs 0273 and 0358, and table 4 | 1-6, 9-10, 12-13, 26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 June 2024** | **17 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/083349

C. DOCUMENTS CONSIDERED TO BE RELEVANT

| Category* | Citation of document, with indication, where appropriate, of the relevant passages | Relevant to claim No. |
|---|---|---|
| X | WO 2022238347 A1 (F. HOFFMANN-LA ROCHE AG; HOFFMANN-LA ROCHE INC.;) 17 November 2022 (2022-11-17) see examples 1-35, and claims 1-34 | 1-7, 9-14, 19, 26-27, 29-32 |
| A | WO 2022166890 A1 (MEDSHINE DISCOVERY INC.) 11 August 2022 (2022-08-11) see claims 1-25 | 1-32 |

Form PCT/ISA/210 (second sheet) (July 2022)

**INTERNATIONAL SEARCH REPORT**
**Information on patent family members**

International application No.
**PCT/CN2024/083349**

| Patent document cited in search report | | | Publication date (day/month/year) | Patent family member(s) | | | Publication date (day/month/year) |
|---|---|---|---|---|---|---|---|
| CN | 117986258 | A | 07 May 2024 | WO | 2024094185 | A1 | 10 May 2024 |
| WO | 2024099996 | A1 | 16 May 2024 | None | | | |
| WO | 2024099992 | A1 | 16 May 2024 | None | | | |
| CN | 1349514 | A | 15 May 2002 | DK | 1175410 | T3 | 18 April 2006 |
| | | | | CO | 5210943 | A1 | 30 October 2002 |
| | | | | JP | 2002543191 | A | 17 December 2002 |
| | | | | JP | 4782289 | B2 | 28 September 2011 |
| | | | | AU | 4298600 | A | 17 November 2000 |
| | | | | AU | 762755 | B2 | 03 July 2003 |
| | | | | WO | 0066568 | A1 | 09 November 2000 |
| | | | | IL | 145543 | A | 08 March 2007 |
| | | | | CA | 2368582 | A1 | 09 November 2000 |
| | | | | CA | 2368582 | C | 13 July 2010 |
| | | | | ZA | 200108943 | B | 25 June 2002 |
| | | | | US | 2003036544 | A1 | 20 February 2003 |
| | | | | US | 6723720 | B2 | 20 April 2004 |
| | | | | ATE | 312085 | T1 | 15 December 2005 |
| | | | | ID | 30390 | A | 29 November 2001 |
| | | | | CZ | 20013961 | A3 | 13 February 2002 |
| | | | | RU | 2252217 | C2 | 20 May 2005 |
| | | | | DE | 60024610 | D1 | 12 January 2006 |
| | | | | DE | 60024610 | T2 | 03 August 2006 |
| | | | | IL | 145543 | A0 | 30 June 2002 |
| | | | | ES | 2254165 | T3 | 16 June 2006 |
| | | | | TWI | 250153 | B | 01 March 2006 |
| | | | | MXPA | 01011054 | A | 04 June 2002 |
| | | | | EP | 1175410 | A1 | 30 January 2002 |
| | | | | EP | 1175410 | B1 | 07 December 2005 |
| | | | | PL | 351434 | A1 | 22 April 2003 |
| | | | | KR | 20020011406 | A | 08 February 2002 |
| | | | | KR | 100746498 | B1 | 07 August 2007 |
| | | | | HUP | 0600529 | A2 | 29 January 2007 |
| | | | | BR | 0010294 | A | 13 February 2002 |
| JP | 2020079325 | A | 28 May 2020 | None | | | |
| WO | 2022238347 | A1 | 17 November 2022 | KR | 20240007152 | A | 16 January 2024 |
| | | | | AU | 2022275300 | A1 | 21 September 2023 |
| | | | | CO | 2023016204 | A2 | 11 December 2023 |
| | | | | CA | 3214626 | A1 | 17 November 2022 |
| | | | | PE | 20240126 | A1 | 22 January 2024 |
| | | | | BR | 112023023527 | A2 | 30 January 2024 |
| | | | | CR | 20230529 | A | 04 December 2023 |
| | | | | IL | 307188 | A | 01 November 2023 |
| | | | | JP | 2024517917 | A | 23 April 2024 |
| | | | | AR | 125818 | A1 | 16 August 2023 |
| | | | | TW | 202311245 | A | 16 March 2023 |
| | | | | EP | 4337652 | A1 | 20 March 2024 |
| WO | 2022166890 | A1 | 11 August 2022 | JP | 2024505735 | A | 07 February 2024 |
| | | | | EP | 4289823 | A1 | 13 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021193897 A
- WO 2022230912 A
- WO 2022238347 A


### Non-patent literature cited in the description


- Remington's Pharmaceutical Sciences. Mack Publishing Company, 2005
- **STAHL** ; **WERMUTH**. Handbook of Pharmaceutical Salts: Properties, Selection, and Use. Wiley-VCH, 2002
- **T.L. GILCHRIST**. Comprehensive Organic Synthesis,. Academic Press, vol. 7, 748-750
- **G.W.H. CHEESEMAN** ; **E.S.G WERSTIUK**. Advances in Heterocyclic Chemistry. Academic Press, vol. 22, 390-392
- **T. HIGUCHI** ; **V. STELLA**. Pro-drugs as Novel Delivery Systems. *ACS Symposium Series*, vol. 14
- **H. BUNDGAARD**. Design of Prodrugs. Elsevier, 1985
- *Technical Guidelines for Nonclinical Pharmacokinetic Studies of Chemical Drugs*, 2014